# EUROPÄISCHE PATENTSCHRIFT

(11) **EP 1 429 607 B1**
(45) Veröffentlichungstag und Bekanntmachung des Hinweises auf die Patenterteilung: **04.04.2007**
(21) Anmeldenummer: 02798644.7
(22) Anmeldetag: 04.09.2002
(51) Int. Cl.: A01N 43/36, C07D 207/20, C07D 413/10, C07D 409/12, C07D 405/12

(54) **DELTA 1-PYRROLINE ALS SCHÄDLINGSBEKÄMPFUNGSMITTEL**
DELTA 1-PYRROLINES USED AS PESTICIDES
DELTA 1-PYRROLINES UTILISEES COMME PESTICIDES

(30) Priorität: 17.09.2001 DE 10145772
(43) Veröffentlichungstag der Anmeldung: 23.06.2004
(73) Patentinhaber: Bayer CropScience AG, 40789 Monheim (DE)
(72) Erfinder: SEITZ, Thomas, 40764 Langenfeld (DE); FÜSSLEIN, Martin, 40215 Düsseldorf (DE); JANSEN, Johannes, Rudolf, 40789 Monheim (DE); KRAATZ, Udo, 51375 Leverkusen (DE); ERDELEN, Christoph, verstorben (DE); TURBERG, Andreas, 42781 Haan (DE); HANSEN, Olaf, 42799 Leichlingen (DE); Lubos-Erdellen, Angelika., 42799 Leichlingen (DE)
(86) Internationale Anmeldenummer: PCT/EP2002/009866
(87) Internationale Veröffentlichungsnummer: WO 2003/024220

(56) Entgegenhaltungen:
- WO-A-02/46151
- WO-A-98/22438
- WO-A-02/064588
- CH-A- 490 012

## Beschreibung

Die vorliegende Erfindung betrifft neue Δ¹-Pyrroline, mehrere Verfahren zu ihrer Herstellung und ihre Verwendung als Schädlingsbekämpfungsmittel.

Es ist bereits bekannt, dass zahlreiche Δ¹-Pyrroline insektizide Eigenschaften besitzen (vgl. WO 00/21958, WO 99/59968, WO 99/59967 und WO 98/22438). Die Wirksamkeit dieser Stoffe ist gut, lässt aber in manchen Fällen zu wünschen übrig.

Es wurden nun neue Δ¹-Pyrroline der Formel (I) 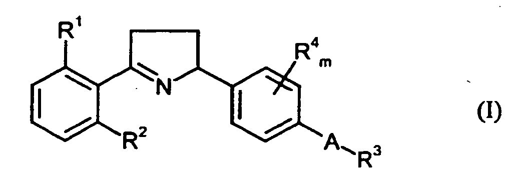 gefunden, in welcher
- R¹: für Halogen oder Methyl steht,
- R²: für Wasserstoff oder Halogen steht,
- R³: für -N(R⁶)-C(=Y)-X-R⁷ steht,
und
a)
   A für jeweils gegebenenfalls einfach bis vierfach, gleich oder verschieden durch R⁵ substituiertes Arylen oder 5-gliedriges Heteroarylen mit 1 bis 3 Heteroatomen, welches 0 bis 3 Stickstoffatome, 0 bis 1 Sauerstoffatom und/oder 0 bis 1 Schwefelatom enthält, oder 6-gliedriges Heteroarylen mit 3 Stickstoffatomen oder 6-gliedriges Heteroarylen mit 1 Stickstoffatom und 1 bis 2 weiteren Heteroatomen, wovon 0 bis 2 Sauerstoffatome und/oder 0 bis 2 Schwefelatome sein können, steht, und
   Y für O (Sauerstoff) oder S (Schwefel) steht, und
   X für O (Sauerstoff), S (Schwefel) oder NR⁸ steht, oder
b)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für O (Sauerstoff) oder S (Schwefel) steht, und
   X für S (Schwefel) oder NR⁸ steht, oder
c)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für S (Schwefel) steht, und
   X für O (Sauerstoff) steht, oder
d)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für O (Sauerstoff) steht, und
   X für O (Sauerstoff) steht, und
- R⁴ und R⁵: unabhängig voneinander für Halogen, Alkyl, Alkoxy, Alkylthio, Halogenalkyl, Halogenalkoxy oder Halogenalkylthio stehen,
- m: für 0, 1, 2, 3 oder 4 steht,
- R⁶: für Wasserstoff oder Alkyl steht,
- R⁷ und R⁸: unabhängig voneinander für Wasserstoff, für jeweils gegebenenfalls einfach oder mehrfach, gleich oder verschieden durch Halogen, Alkylcarbonyl, Alkylcarbonyloxy, Alkylamino, Dialkylamino, Alkoxy, Alkylthio, Alkoxyalkoxy, Halogenalkoxy, Halogenalkylthio und/oder Halogenalkoxyalkoxy substituiertes Alkyl oder Alkenyl;
für jeweils gegebenenfalls einfach oder mehrfach, gleich oder verschieden durch Halogen, Alkyl, Halogenalkyl, Alkoxy, Halogenalkoxy, Alkylthio, Halogenalkylthio, Alkylcarbonyl und/oder Alkoxycarbonyl substituiertes Cycloalkyl, Cycloalkylalkyl, Aryl, Arylalkyl, gesättigtes oder ungesättigtes, 5- bis 10-gliedriges Heterocyclyl oder Heterocyclylalkyl stehen,
- R⁶ und R⁷: außerdem gemeinsam für gegebenenfalls einfach oder mehrfach durch Alkyl substituiertes Alkylen stehen, oder
- R⁷ und R⁸: außerdem gemeinsam mit dem Stickstoffatom, an das sie gebunden sind, für einen gesättigten oder ungesättigten 5- bis 10-gliedrigen Heterocyclus stehen, der gegebenenfalls eine weitere Heteroatomgruppierung aus der Reihe -O-, -S- oder -NR⁹- enthalten kann und der gegebenenfalls einfach oder mehrfach, gleich oder verschieden durch Halogen, Alkyl, Alkoxy, Alkylthio, Halogenalkyl, Halogenalkoxy und/oder Halogenalkylthio substituiert sein kann,
und
- R⁹: für Wasserstoff, Alkyl oder Alkenyl steht.

Die Verbindungen der Formel (I) können gegebenenfalls in Abhängigkeit von der Art und Anzahl der Substituenten als geometrische und/oder optische Isomere, Regioisomere bzw. Konfigurationsisomere oder deren Isomerengemische in unterschiedlicher Zusammensetzung vorliegen. Sowohl die reinen Isomere als auch die Isomerengemische werden erfindungsgemäß beansprucht.

Weiterhin wurde gefunden, dass sich Δ¹-Pyrroline der Formel (I) herstellen lassen, indem man
A) Δ¹-Pyrroline der Formel (II) 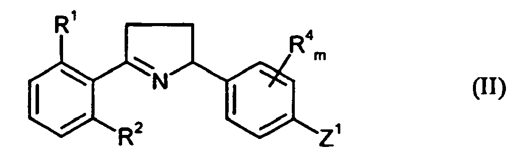 in welcher
   R¹, R², R⁴ und m die oben angegebenen Bedeutungen haben, und
   - Z¹: für Chlor, Brom, Iod, -OSO₂CF₃ oder -OSO₂(CF₂)₃CF₃ steht,
   mit (Hetero)cyclen der Formel (III) 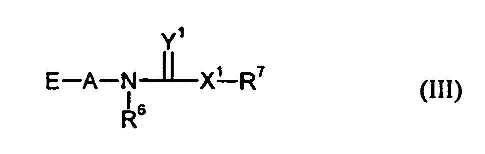 in welcher
   A, R⁶ und R⁷ die oben angegebenen Bedeutungen haben,
   - Y¹: für O (Sauerstoff) steht,
   - X¹: für O (Sauerstoff) oder NR⁸ steht,
   - E: für Chlor, Brom, Iod, -OSO₂CF₃ oder -OSO₂(CF₂)₃CF₃ steht,
   in Gegenwart eines Katalysators, in Gegenwart eines Diboronsäureesters und gegebenenfalls in Gegenwart eines Säurebindemittels und gegebenenfalls in Gegenwart eines Verdünnungsmittels in einer Tandem-Reaktion umsetzt,
   oder
B) Δ¹-Pyrroline der Formel (IV) 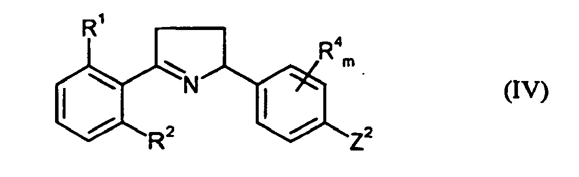 in welcher
   R¹, R², R⁴ und m die oben angegebenen Bedeutungen haben,
   - Z²: für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl steht,
   mit (Hetero)cyclen der Formel (III) 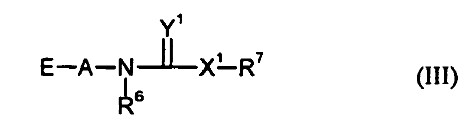 in welcher
   E, A, Y¹, X¹, R⁶ und R⁷ die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt,
   oder
C) Δ¹-Pyrroline der Formel (II) 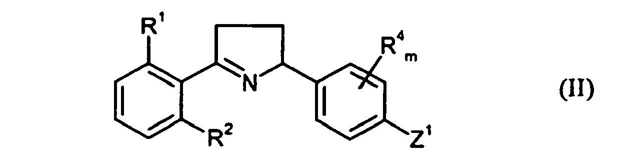 in welcher
   R¹, R², R⁴, m und Z¹ die oben angegebenen Bedeutungen haben,
   mit Boronsäure-Derivaten der Formel (V) 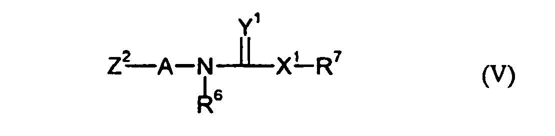 in welcher
   Z², A, Y¹, X¹, R⁶ und R⁷ die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt,
   oder
D) Δ¹-Pyrroline der Formel (II-a) 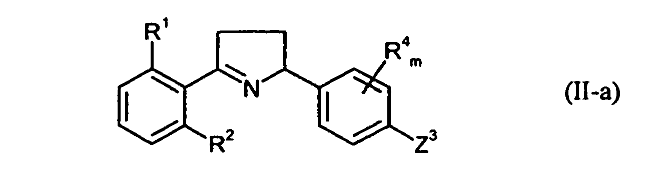 in welcher
   R¹, R², R⁴ und m die oben angegebenen Bedeutungen haben,
   - Z³: für Brom oder Iod steht,
   mit metallorganischen Verbindungen der Formel (VI) 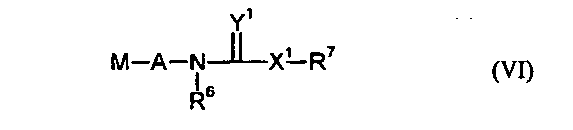 in welcher
   A, Y¹, X¹, R⁶ und R⁷ die oben angegebenen Bedeutungen haben,
   - M: für ZnCl, Sn(Me)₃ oder Sn(n-Bu)₃ steht,
   in Gegenwart eines Katalysators, gegebenenfalls in Gegenwart eines Säurebindemittels und gegebenenfalls in Gegenwart eines Verdünnungsmittels umsetzt,
   oder
E) Δ¹-Pyrroline der Formel (VII) 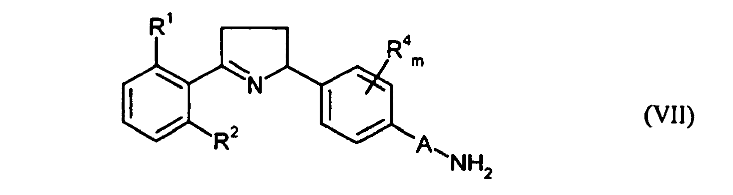
in welcher
R¹, R², A, R⁴ und m die oben angegebenen Bedeutungen haben,
entweder mit einem Iso(thio)cyanat der Formel (VIII)

R⁷- N=C=Y (VIII)

in welcher
Y und R⁷ die oben angegebenen Bedeutungen haben
oder mit einem (Thio)carbonat der Formel (IX) 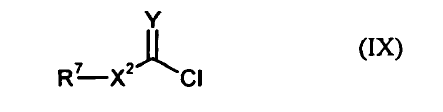 in welcher
Y und R⁷ die oben angegebenen Bedeutungen haben,
X² für O (Sauerstoff) oder S (Schwefel) steht,
jeweils gegebenenfalls in Gegenwart eines Verdünnungsmittels und gegebenenfalls in Gegenwart eines Säurebindemittels umsetzt.

Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemäßen Δ¹-Pyrroline sind durch die Formel (I) allgemein definiert.

Bevorzugt sind Verbindungen der Formel (I), in welcher
- R¹: für Fluor, Chlor, Brom oder Methyl steht,
- R²: für Wasserstoff, Fluor, Chlor oder Brom steht,
- R³: für -N(R⁶)-C(=Y)-X-R⁷ steht,
und
a)
   A für jeweils gegebenenfalls einfach bis dreifach, gleich oder verschieden durch R⁵ substituiertes Arylen (insbesondere Phenylen) oder 5-gliedriges Heteroarylen mit 1 bis 3 Heteroatomen, welches 0 bis 3 Stickstoffatome, 0 bis 1 Sauerstoffatom und/oder 0 bis 1 Schwefelatom enthält (insbesondere aus der Reihe Pyrrolylen, Furylen, Thienylen, Pyrazylen, Imidazylen, Triazylen, Thiazylen oder Oxazylen), oder 6-gliedriges Heteroarylen mit 3 Stickstoffatomen (insbesondere Triazinylen) oder 6-gliedriges Heteroarylen mit 1 Stickstoffatom und 1 bis 2 weiteren Heteroatomen, wovon 0 bis 2 Sauerstoffatome und/oder 0 bis 2 Schwefelatome sein können (insbesondere aus der Reihe Oxazinylen oder Thiazinylen), steht, und
   Y für O (Sauerstoff) oder S (Schwefel) steht, und
   X für O (Sauerstoff), S (Schwefel) oder NR⁸ steht, oder
b)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für O (Sauerstoff) oder S (Schwefel) steht, und
   X für S (Schwefel) oder NR⁸ steht, oder
c)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für S (Schwefel) steht, und
   X für O (Sauerstoff) steht, oder
d)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für O (Sauerstoff) steht, und
   X für O (Sauerstoff) steht,
und
- R⁴ und R⁵: unabhängig voneinander für Fluor, Chlor, Brom, C₁-C₆-Alkyl, C₁-C₆-Alkoxy, C₁-C₆-Alkylthio, C₁-C₆-Halogenalkyl, C₁-C₆-Halogenalkoxy oder C₁-C₆-Halogenalkylthio stehen,
- m: für 0, 1, 2 oder 3 steht,
- R⁶: für Wasserstoff oder C₁ -C₆-Alkyl steht,
- R⁷ und R⁸: unabhängig voneinander für Wasserstoff, für jeweils gegebenenfalls einfach oder mehrfach, gleich oder verschieden durch Fluor, Chlor, Brom, C₁-C₆-Alkylcarbonyl, C₁-C₆-Alkylcarbonyloxy, C₁-C₆-Alkylamino, Di-(C₁-C₆-alkyl)amino, C₁-C₁₀-Alkoxy, C₁-C₁₀-Alkylthio, C₁-C₁₀-Alkoxy-C₁-C₁₀-alkoxy, C₁-C₁₀-Halogenalkoxy, C₁-C₁₀-Halogenalkylthio und/oder C₁-C₁₀-Halogenalkoxy-C₁-C₁₀-alkoxy substituiertes C₁-C₂₀-Alkyl oder C₂-C₂₀-Alkenyl;
für jeweils gegebenenfalls einfach bis vierfach, gleich oder verschieden durch Fluor, Chlor, Brom, C₁-C₆-Alkyl, C₁-C₆-Alkoxy, C₁-C₆-Alkylthio, C₁-C₆-Halogenalkyl, C₁-C₆-Halogenalkoxy, C₁-C₆-Halogenalkylthio, C₁-C₆-Alkylcarbonyl und/oder C₁-C₆-Alkoxycarbonyl substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) stehen,
- R⁶ und R⁷: außerdem gemeinsam für gegebenenfalls einfach bis vierfach durch C₁-C₄-Alkyl substituiertes C₂-C₄-Alkylen stehen, oder
- R⁷ und R⁸: außerdem gemeinsam mit dem Stickstoffatom, an das sie gebunden sind, für einen gesättigten oder ungesättigten 5- bis 10-gliedrigen Heterocyclus stehen, der gegebenenfalls eine weitere Heteroatomgruppierung aus der Reihe -O-, -S- oder -NR⁹- enthalten kann und der gegebenenfalls einfach bis vierfach, gleich oder verschieden durch Fluor, Chlor, Brom, C₁-C₆-Alkyl, C₁-C₆-Alkoxy, C₁-C₆-Alkylthio, C₁-C₆-Halogenalkyl, C₁-C₆-Halogenalkoxy und/oder C₁-C₆-Halogenalkylthio substituiert sein kann, und
- R⁹: für Wasserstoff, C₁-C₆-Alkyl oder C₂-C₆-Alkenyl steht.

Besonders bevorzugt sind Verbindungen der Formel (I), in welcher
- R¹: für Fluor, Chlor oder Methyl steht,
- R²: für Wasserstoff, Fluor oder Chlor steht,
- R³: für -N(R⁶)-C(=Y)-X-R⁷ steht,
und
a)
   A für jeweils gegebenenfalls einfach oder zweifach, gleich oder verschieden durch R⁵ substituiertes Phenylen, Pyrrolylen, Furylen, Thienylen, Pyrazylen, Imidazylen, Triazylen, Thiazylen oder Oxazylen steht, und
   Y für O (Sauerstoff) oder S (Schwefel) steht, und
   X für O (Sauerstoff), S (Schwefel) oder NR⁸ steht, oder
b)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für O (Sauerstoff) oder S (Schwefel) steht, und
   X für S (Schwefel) oder NR⁸ steht, oder
c)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für S (Schwefel) steht, und
   X für O (Sauerstoff) steht, oder
d)
   A für jeweils gegebenenfalls einfach oder zweifach, gleich oder verschieden durch R⁵ substituiertes Pyridinylen, Pyrimidinylen, Pyrazinylen oder Pyridazinylen steht, und
   Y für O (Sauerstoff) steht, und
   X für O (Sauerstoff) steht,
und
- R⁴ und R⁵: unabhängig voneinander für Fluor, Chlor, C₁-C₄-Alkyl, C₁-C₄-Alkoxy, C₁-C₄-Alkylthio; C₁-C₄-Halogenalkyl, C₁-C₄-Halogenalkoxy oder C₁-C₄-Halogenalkylthio mit jeweils 1 bis 9 Fluor-, Chlor- und/oder Bromatomen stehen,
- m: für 0, 1 oder 2 steht,
- R⁶: für Wasserstoff oder C₁-C₄-Alkyl steht,
- R⁷ und R⁸: unabhängig voneinander für Wasserstoff, für jeweils gegebenenfalls einfach oder mehrfach, gleich oder verschieden durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₁₀-Alkoxy, C₁-C₁₀-Alkylthio, C₁-C₁₀-Alkoxy-C₁-C₆-alkoxy, C₁-C₁₀-Halogenalkoxy, C₁-C₁₀-Halogenalkylthio und/oder C₁-C₁₀-Halogenalkoxy-C₁-C₆-alkoxy mit jeweils 1 bis 21 Fluor-, Chlor- und/oder Bromatomen substituiertes C₁-C₁₆-Alkyl oder C₂-C₁₆-Alkenyl;
für jeweils gegebenenfalls einfach bis dreifach, gleich oder verschieden durch Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio mit jeweils 1 bis 9 Fluor-, Chlor- und/oder Bromatomen, C₁-C₄-Alkylcarbonyl und/oder C₁-C₄-Alkoxycarbonyl substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
- R⁶ und R⁷: außerdem gemeinsam für gegebenenfalls einfach bis dreifach durch C₁-C₄-Alkyl substituiertes C₂-C₃-Alkylen stehen, oder
- R⁷ und R⁸: außerdem gemeinsam mit dem Stickstoffatom, an das sie gebunden sind, für einen gesättigten oder ungesättigten 5- bis 7-gliedrigen Heterocyclus stehen, der gegebenenfalls eine weitere Heteroatomgruppierung aus der Reihe -O-, -S- oder -NR⁹- enthalten kann (insbesondere aus der Reihe Piperidino, Morpholino, Thiomorpholino, Piperazino, Pyrrolidino, Oxazolidino, Thiazolidino, 4H-1-Oxazinyl, 4H-1-Thiazinyl) und der gegebenenfalls einfach bis vierfach, gleich oder verschieden durch Fluor, Chlor, C₁-C₄-Alkyl, C₁-C₄-Alkoxy, C₁-C₄-Alkylthio, C₁-C₄-Halogenalkyl, C₁-C₄-Halogenalkoxy und/oder C₁-C₄-Halogenalkylthio mit jeweils 1 bis 9 Fluor-, Chlor- und/oder Bromatomen substituiert sein kann, und
- R⁹: für Wasserstoff, C₁-C₄-Alkyl oder C₂-C₄-Alkenyl steht.

Ganz besonders bevorzugt sind Verbindungen der Formel (I), in welcher
- R¹: für Fluor oder Chlor steht,
- R²: für Wasserstoff oder Fluor steht,
- R³: für -N(R⁶)-C(=Y)-X-R⁷ steht,
und
a)
   A für jeweils gegebenenfalls einfach durch R⁵ substituiertes 1,2-Phenylen, 1,4-Phenylen, 2,5-Pyrrolylen, 2,5-Furylen, 2,4-Furylen, 2,5-Thienylen, 2,4-Thienylen, 2,5-Thiazylen, 2,4-Thiazylen, 2,5-Oxazylen oder 2,4-Oxazylen steht, und
   Y für O (Sauerstoff) oder S (Schwefel) steht, und
   X für O (Sauerstoff), S (Schwefel) oder NR⁸ steht, oder
b)
   A für jeweils gegebenenfalls einfach durch R⁵ substituiertes 2,5-Pyridinylen, 2,5-Pyrimidinylen, 2,5-Pyrazinylen oder 3,6-Pyridazinylen steht, und
   Y für O (Sauerstoff) oder S (Schwefel) steht, und
   X für S (Schwefel) oder NR⁸ steht, oder
c)
   A für jeweils gegebenenfalls einfach durch R⁵ substituiertes 2,5-Pyridinylen, 2,5-Pyrimidinylen, 2,5-Pyrazinylen oder 3,6-Pyridazinylen steht, und
   Y für S (Schwefel) steht, und
   X für O (Sauerstoff) steht, oder
d)
   A für jeweils gegebenenfalls einfach durch R⁵ substituiertes 2,5-Pyridinylen, 2,5-Pyrimidinylen, 2,5-Pyrazinylen oder 3,6-Pyridazinylen steht, und
   Y für O (Sauerstoff) steht, und
   X für O (Sauerstoff) steht,
und
- R⁴ und R⁵: unabhängig voneinander für Fluor, Chlor, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy, Methylthio, Ethylthio, n-Propylthio, i-Propylthio, n-Butylthio, i-Butylthio, s-Butylthio, t-Butylthio, Trifluormethyl, Trifluorethyl, Trifluormethoxy, Trifluorethoxy, Trifluormethylthio oder Trifluorethylthio stehen,
- m: für 0 oder 1 steht,
- R⁶: für Wasserstoff, Methyl, Ethyl, n-Propyl, i-Propyl, n-Butyl, i-Butyl, s-Butyl oder t-Butyl steht,
- R⁷ und R⁸: unabhängig voneinander für Wasserstoff, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₁₀-Alkoxy, C₁-C₈-Alkoxy-C₁-C₆-alkoxy, C₁-C₁₀-Alkylthio, C₁-C₁₀-Halogenalkoxy, C₁-C₁₀-Halogenalkylthio mit jeweils 1 bis 21 Fluor-, Chlor- und/oder Bromatomen, C₁-C₈-Halogenalkoxy-C₁-C₆-alkoxy mit 1 bis 17 Fluor-, Chlor- und/oder Bromatomen substituiertes C₁-C₁₀-Alkyl oder C₂-C₁₀-Alkenyl (insbesondere Methyl, Ethyl, n-Propyl, i-Propyl, n-Butyl, i-Butyl, s-Butyl, t-Butyl, die isomeren Pentyle, die isomeren Hexyle);
für jeweils gegebenenfalls einfach bis dreifach, gleich oder verschieden durch Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio mit jeweils 1 bis 9 Fluor-, Chlor- und/oder Bromatomen, C₁-C₄-Alkylearbonyl und/oder C₁-C₄-Alkoxycarbonyl substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
- R⁶ und R⁷: außerdem gemeinsam für jeweils gegebenenfalls einfach oder zweifach, gleich oder verschieden durch Methyl, Ethyl, n-Propyl oder i-Propyl substituiertes Methylen oder Ethylen stehen, oder
- R⁷ und R⁸: außerdem gemeinsam mit dem Stickstoffatom, an das sie gebunden sind, für einen 5- bis 6-gliedrigen Heterocyclus aus der Reihe Piperidino, Morpholino, Thiomorpholino, Piperazino, Pyrrolidino, Oxazolidino, Thiazolidino, 4H-1-Oxazinyl, 4H-1-Thiazinyl stehen, der gegebenenfalls einfach bis vierfach, gleich oder verschieden durch Fluor, Chlor, Brom, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy, Methylthio, Ethylthio, n-Propylthio, i-Propylthio, n-Butylthio, i-Butylthio, s-Butylthio, t-Butylthio, C₁-C₄-Halogenalkyl, C₁-C₄-Halogenalkoxy, C₁-C₄-Halogen-alkylthio mit jeweils 1 bis 9 Fluor-, Chlor- und/oder Bromatomen substituiert sein kann, wobei der Rest Piperazino am zweiten Stickstoffatom durch R⁹ substituiert ist, und
- R⁹: für Wasserstoff, Methyl, Ethyl, n-Propyl, i-Propyl, n-Butyl, i-Butyl, s-Butyl, t-Butyl, Vinyl oder Allyl steht.

Weiterhin ganz besonders bevorzugt sind Verbindungen der Formeln (I-1) und (1-2) 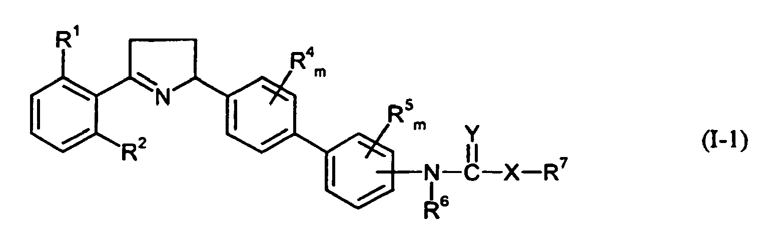 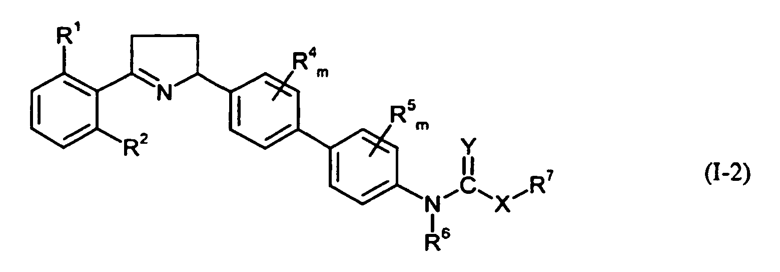 in welchen jeweils
a)
   - Y: für O (Sauerstoff) oder S (Schwefel) steht, und
   - X: für O (Sauerstoff), S (Schwefel) oder NR⁸ steht,
   und R¹, R², R⁴, R⁵, m, R⁶, R⁷, R⁸ die oben angegebenen Bedeutungen haben.

In den Formeln (I-1) und (1-2) stehen die Reste R¹, R², R⁴, R⁵, m, R⁶, R⁷, R⁸ jeweils bevorzugt, besonders bevorzugt bzw. ganz besonders bevorzugt für diejenigen Bedeutungen, die bei der Beschreibung der erfindungsgemäßen Stoffe der Formel (I) für diese Reste als bevorzugt, besonders bevorzugt etc. genannt wurden. Y und X stehen jeweils bevorzugt, besonders bevorzugt bzw. ganz besonders bevorzugt für diejenigen Bedeutungen die jeweils unter dem entsprechenden Abschnitt "a)" beschrieben wurden.

Weiterhin ganz besonders bevorzugt sind Verbindungen der Formeln (I-3) bis (I-8) 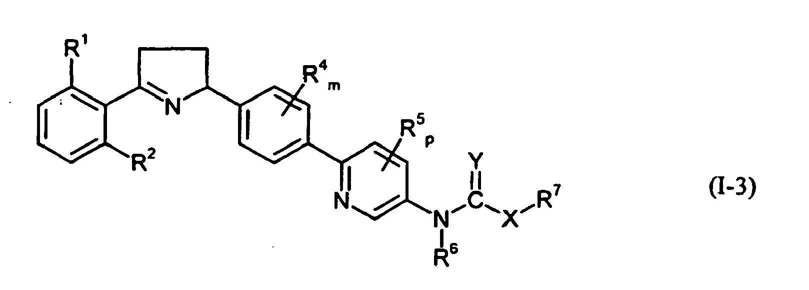 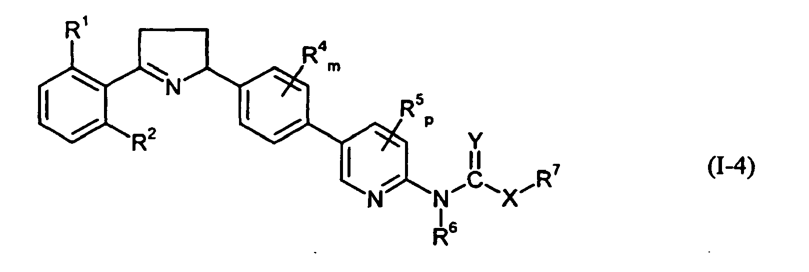 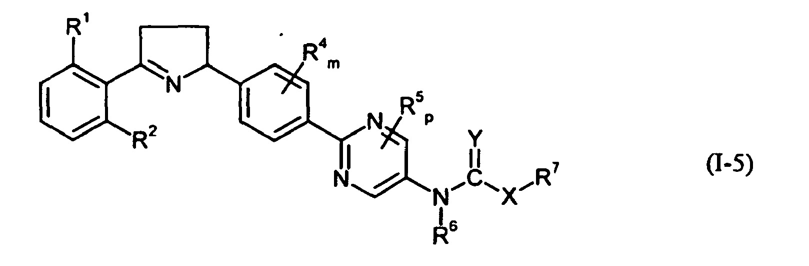 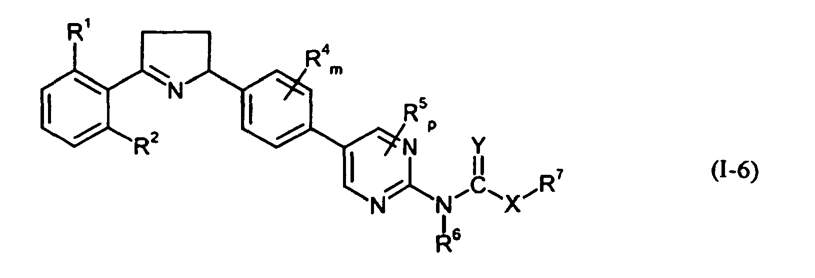 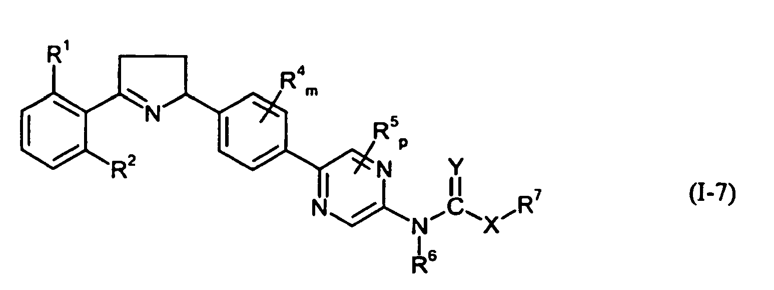 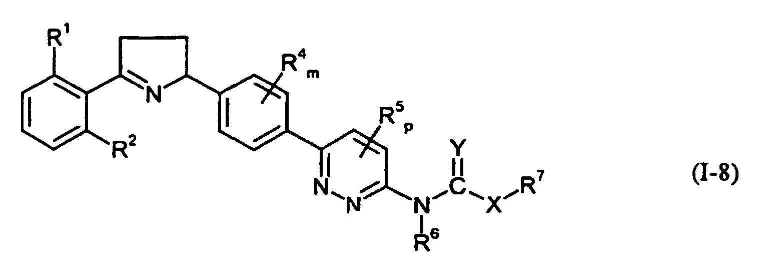 in welchen jeweils
- p: für 0, 1 oder 2 steht,

b)
   - Y: für O (Sauerstoff) oder S (Schwefel) steht, und
   - X: für S (Schwefel) oder NR⁸ steht,
   oder
c)
   - Y: für S (Schwefel) steht, und
   - X: für O (Sauerstoff) steht,
   oder
d)
   - Y: für O (Sauerstoff) steht, und
   - X: für O (Sauerstoff) steht,
und R¹, R², R⁴, R⁵, m, R⁶, R⁷ und R⁸ die oben angegebenen Bedeutungen haben.

In den Formeln (I-3) bis (I-8) stehen die Reste R¹, R², R⁴, R⁵, m, R⁶, R⁷, R⁸ jeweils bevorzugt, besonders bevorzugt bzw. ganz besonders bevorzugt für diejenigen Bedeutungen, die bei der Beschreibung der erfindungsgemäßen Stoffe der Formel (I) für diese Reste als bevorzugt, besonders bevorzugt etc. genannt wurden. Y und X stehen jeweils bevorzugt, besonders bevorzugt bzw. ganz besonders bevorzugt für diejenigen Bedeutungen die jeweils unter den entsprechenden Abschnitten "b)", "c)" und "d)" beschrieben wurden. p steht bevorzugt für 0, 1 oder 2, besonders bevorzugt für 0 oder 1, ganz besonders bevorzugt für 0.

Bevorzugt sind außerdem Verbindungen der Formel (I), in welcher A für Phenylen, bevorzugt 1,4-Phenylen steht.

Bevorzugt sind außerdem Verbindungen der Formel (I), in welcher A für Pyridinylen, Pyrimidinylen, Pyrazinylen oder Pyridazinylen, bevorzugt 2,5-Pyridinylen, 2,5-Pyrimidinylen, 2,5-Pyrazinylen oder 3,6-Pyridazinylen steht.

Bevorzugt sind außerdem Verbindungen der Formel (I), in welcher Y für O (Sauerstoff) steht.

Bevorzugt sind außerdem Verbindungen der Formel (I), in welcher X für O (Sauerstoff) oder NR⁸, bevorzugt für O (Sauerstoff) steht.

Bevorzugt sind außerdem Verbindungen der Formel (I), in welcher Y und X jeweils für O (Sauerstoff) stehen.

Bevorzugt sind außerdem Verbindungen der Formel (I), in welcher Y für O (Sauerstoff) und X für NR⁸ stehen.

Bevorzugt sind außerdem Verbindungen der Formel (I), in welcher A für Phenylen, bevorzugt 1,4-Phenylen, Y für O (Sauerstoff) oder S (Schwefel), bevorzugt O (Sauerstoff) und X für O (Sauerstoff), S (Schwefel) oder NR⁸, bevorzugt für O (Sauerstoff) oder NR⁸, besonders bevorzugt für O (Sauerstoff), stehen.

Bevorzugt sind außerdem Verbindungen der Formel (I), in welcher R¹ und R² jeweils für Fluor stehen.

Bevorzugt sind außerdem Verbindungen der Formel (I), in welcher R¹ für Methyl und R² für Wasserstoff stehen.

Bevorzugt sind außerdem Verbindungen der Formel (I), in welcher R¹ für Chlor und R² für Wasserstoff stehen.

Bevorzugt sind außerdem Verbindungen der Formel (I), in welcher R¹ für Chlor und R² für Fluor stehen.

Bevorzugt sind außerdem Verbindungen der Formel (I), in welcher R⁶ für Wasserstoff steht.

Weiterhin ganz besonders bevorzugt sind Verbindungen der Formel (I-a) mit (R)-Konfiguration in 5-Position des Pyrrolin-Ringes. 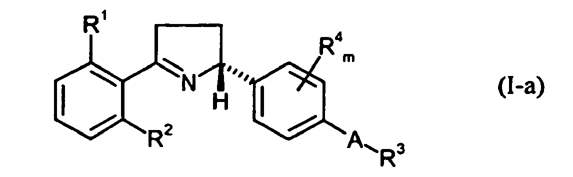 in welcher
R¹, R², A, R³, R⁴ und m die oben angegebenen Bedeutungen haben.

Auch die Verbindungen (I-1) bis (I-8) können entsprechend Formel (I-a) mit R-Konfiguration in 2-Position des 2H-Pyrrols (entspricht 5-Position bei Bezeichnung als Pyrrolin) vorliegen.

Verbindungen der Formel (I-a) erhält man durch übliche Verfahren zur Racematspaltung, wie zum Beispiel durch Chromatographie der entsprechenden Racemate an einer chiralen stationären Phase. Es ist möglich, sowohl die racemischen Endprodukte oder racemische Zwischenprodukte auf diese Weise in die beiden Enantiomere zu zerlegen.

Gesättigte KohlenWasserstoffreste wie Alkyl können, auch in Verbindung mit Heteroatomen, wie z.B. in Alkoxy, soweit möglich, jeweils geradkettig oder verzweigt sein.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Verwendet man 5-(2,6-Difluorphenyl)-2-(4-bromphenyl)-3,4-dihydro-2H-pyrrol, Methyl-4-bromphenylcarbamat und 4,4,4',4',5,5,5',5'-Octamethyl-2,2'-bis-1,3,2-dioxaborolan als Ausgangsstoffe sowie einen Palladiumkatalysator, so kann der Verlauf des erfindungsgemäßen Verfahrens (A) durch das folgende Formelschema veranschaulicht werden.

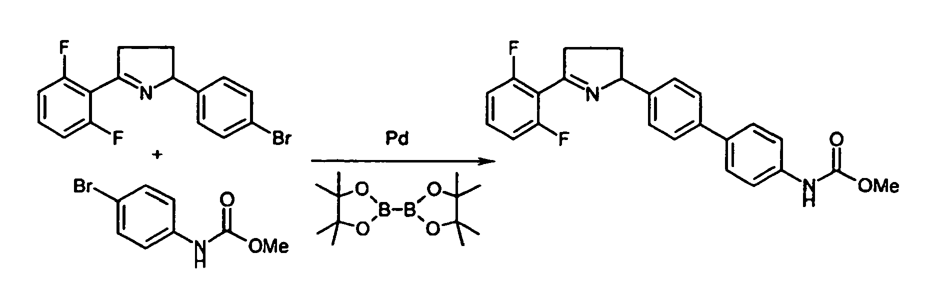

Verwendet man 5-(2,6-Difluorphenyl)-2-[4-(4,4,5,5-tetramethyl-1,3,2-dioxaborolan-2-yl)phenyl]-3,4-dihydro-2H-pyrrol und Methyl-4-bromphenylcarbamat als Ausgangsstoffe sowie einen Palladiumkatalysator, so kann der Verlauf des erfindungsgemäßen Verfahrens (B) durch das folgende Formelschema veranschaulicht werden.

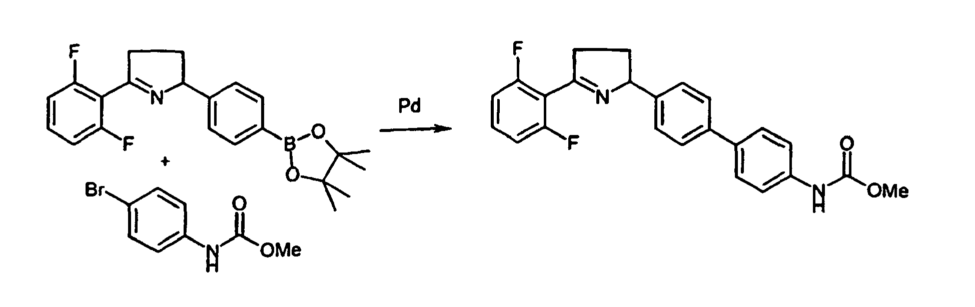

Verwendet man 5-(2,6-Difluorphenyl)-2-[4-(trifluormethylsulfonyloxy)phenyl]-3,4-dihydro-2H-pyrrol und Methyl-4-(4,4,5,5-tetramethyl-1,3,2-dioxaborolan-2-yl)phenylcarbamat als Ausgangsstoffe sowie einen Palladiumkatalysator, so kann der Verlauf des erfindungsgemäßen Verfahrens (C) durch das folgende Formelschema veranschaulicht werden.

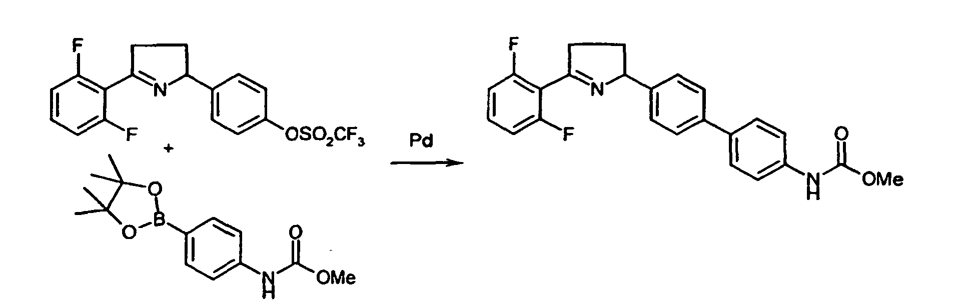

Verwendet man 5-(2,6-Difluorphenyl)-2-(4-bromphenyl)-3,4-dihydro-2H-pyrrol und Methyl-4-(tributylstannyl)phenylcarbamat als Ausgangsstoffe sowie einen Palladiumkatalysator, so kann der Verlauf des erfindungsgemäßen Verfahrens (D) durch das folgende Formelschema veranschaulicht werden.

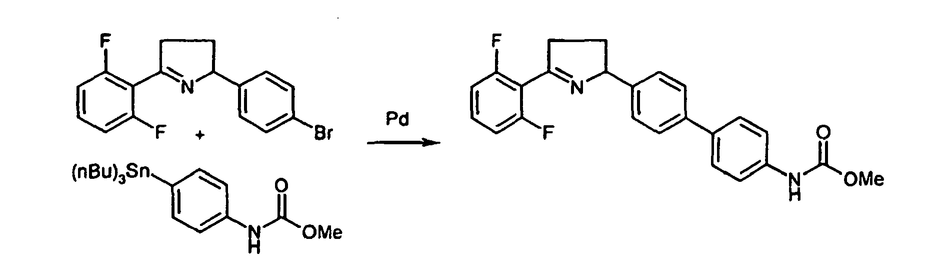

Verwendet man 4'-[5-(2,6-Difluorphenyl)-3,4-dihydro-2H-pyrrol-2-yl]-1,1'-biphenyl-4-amin und (Methylimino)(thioxo)methan als Ausgangsstoff, so kann der Verlauf des erfindungsgemäßen Verfahrens (E) durch das folgende Formelschema veranschaulicht werden.

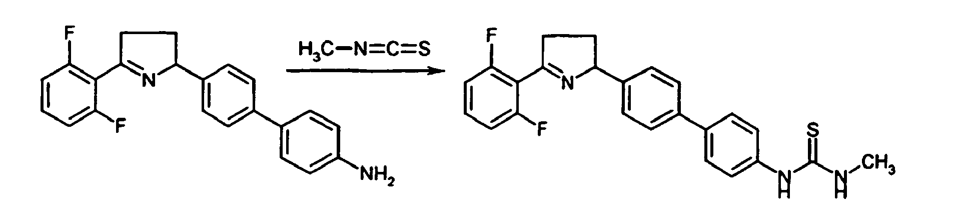

### Erläuterung der Verfahren und Zwischenprodukte

### Verfahren (A)

In einem ersten Reaktionsschritt wird eine Verbindung der Formel (II) mit einem Diboronsäureester in Gegenwart eines Palladium-Katalysators, gegebenenfalls in Gegenwart eines Säurebindemittels und gegebenenfalls in Gegenwart eines Lösungsmittels gekuppelt. Ohne Isolierung des Zwischenprodukts wird in demselben Reaktionsgefäß in einem zweiten Reaktionsschritt eine Verbindung der Formel (III) in Gegenwart eines Katalysators, gegebenenfalls in Gegenwart eines Säurebindemittels und gegebenenfalls in Gegenwart eines Lösungsmittels gekuppelt (vgl. z.B. Tetrahedron Lett. 1997, 38, 3841).

Das erfindungsgemäße Verfahren (A) kann in zwei Varianten durchgeführt werden. Es kann entweder eine Verbindung der Formel (II) oder eine Verbindung der Formel (III) vorgelegt werden. Verfahren (A) ist als Tandem-Reaktion der nachfolgend beschriebenen Verfahren (B) und (C) anzusehen.

Die bei der Durchführung des erfindungsgemäßen Verfahrens (A) als Ausgangsstoffe benötigten Δ¹-Pyrroline sind durch die Formel (II) allgemein definiert. In dieser Formel stehen R¹, R², R⁴ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Z¹ steht bevorzugt für Brom, Iod, -OSO₂CF₃ oder -OSO₂(CF₂)₃CF₃, besonders bevorzugt für Brom, -OSO₂CF₃ oder -OSO₂(CF₂)₃CF₃, ganz besonders bevorzugt für Brom oder -OSO₂CF₃.

Δ¹-Pyrroline der Formel (II) lassen sich nach bekannten Verfahren herstellen (vgl. WO 98/22438). Δ¹-Pyrroline der Formel (II) können auch nach einem unten beschriebenen Verfahren erhalten werden.

Die bei der Durchführung des erfindungsgemäßen Verfahrens (A) als Ausgangsstoffe benötigten (Hetero)cyclen sind durch die Formel (III) allgemein definiert. In dieser Formel steht Y¹ bevorzugt für O (Sauerstoff). X¹ steht bevorzugt für O (Sauerstoff) oder NR⁸. E steht bevorzugt für Brom, Chlor, Iod oder -OSO₂CF₃, besonders bevorzugt für Brom, Chlor oder Iod, ganz besonders bevorzugt für Brom oder Chlor. A, R⁶, R⁷ und R⁸ stehen bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden.

Die (Hetero)cyclen der Formel (III) sind teilweise bekannt.

### (Hetero)cyclen der Formel (III-a)

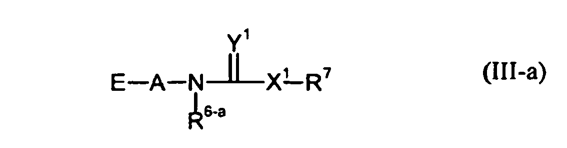

in welcher
E, A, Y¹, X¹ und R⁷ die oben angegebenen Bedeutungen haben,
- R^{6-a}: für Wasserstoff steht,
lassen sich beispielsweise herstellen, indem man
a) Isocyanate der Formel (X)

   E-A-N=C=O (X)

   in welcher
   E und A die oben angegebenen Bedeutungen haben,
   mit Alkoholen bzw. Aminen der Formel (XI)

   H-X¹-R⁷ (XI)

   in welcher
   X¹ und R⁷ die oben angegebenen Bedeutungen haben,
   oder mit Verbindungen der Formel (XII) 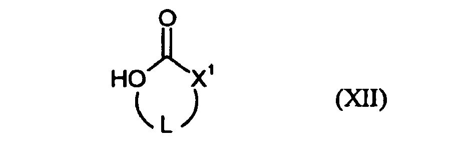 in welcher
   - X¹: die oben angegebenen Bedeutungen hat,
   - L: für gegebenenfalls einfach oder mehrfach durch Alkyl substituiertes Alkylen
gegebenenfalls in Gegenwart eines Verdünnungsmittels (z.B. Toluol, Dioxan, Dimethylsulfoxid) umsetzt.

Die nach Verfahren (a) herstellbaren (Hetero)cyclen sind durch die Formel (III-a) allgemein definiert. In dieser Formel stehen A und R⁷ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. E, Y¹ und X¹ stehen bevorzugt, besonders bevorzugt bzw. ganz besonders bevorzugt für diejenigen Bedeutungen, die bereits in Zusammenhang mit der Beschreibung der erfindungsgemäßen Stoffe der Formel (III) für diese Reste als bevorzugt, besonders bevorzugt etc. genannt wurden. R^{6-a} steht bevorzugt für Wasserstoff.

Die bei der Durchführung des Verfahrens (a) als Ausgangsstoffe benötigten Isocyanate sind durch die Formel (X) allgemein definiert. In dieser Formel steht A bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. E steht bevorzugt, besonders bevorzugt bzw. ganz besonders bevorzugt für diejenigen Bedeutungen, die bereits in Zusammenhang mit der Beschreibung der erfindungsgemäßen Stoffe der Formel (III) für diese Reste als bevorzugt, besonders bevorzugt etc. genannt wurden.

Isocyanate der Formel (X) sind bekannt.

Die bei der Durchführung des Verfahrens (a) als Ausgangsstoffe benötigten Alkohole bzw. Amine durch die Formel (XI) allgemein definiert. In dieser Formel steht R⁷ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. X¹ steht bevorzugt, besonders bevorzugt bzw. ganz besonders bevorzugt für diejenigen Bedeutungen, die bereits in Zusammenhang mit der Beschreibung der erfindungsgemäßen Stoffe der Formel (III) für diese Reste als bevorzugt, besonders bevorzugt etc. genannt wurden.

Alkohole bzw. Amine der Formel (XI) sind bekannt.

Die bei der Durchführung des Verfahrens (a) weiterhing als Ausgangsstoffe benötigten Verbindungen sind durch die Formel (XII) allgemein definiert. In dieser Formel steht X¹ bevorzugt, besonders bevorzugt bzw. ganz besonders bevorzugt für diejenigen Bedeutungen, die bereits in Zusammenhang mit der Beschreibung der erfindungsgemäßen Stoffe der Formel (III) für diese Reste als bevorzugt, besonders bevorzugt etc. genannt wurden. L steht bevorzugt für gegebenenfalls einfach bis vierfach durch C₁-C₄-Alkyl substituiertes C₂-C₄-Alkylen, besonders bevorzugt für gegebenenfalls einfach bis dreifach durch C₁-C₄-Alkyl substituiertes C₂-C₃-Alkylen, ganz besonders bevorzugt für jeweils gegebenenfalls einfach oder zweifach, gleich oder verschieden durch Methyl, Ethyl, n-Propyl oder i-Propyl substituiertes Methylen oder Ethylen.

Verbindungen der Formel (XII) sind bekannt.

### (Hetero)cyclen der Formel (III-b)

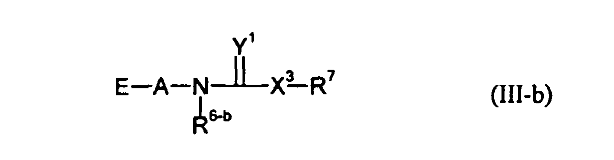

in welcher
E, A, Y¹ und R⁷ die oben angegebenen Bedeutungen haben,
- X³: für O (Sauerstoff) steht,
- R^{6-b}: für Alkyl steht,
lassen sich beispielsweise herstellen, indem man
b) Amine der Formel (XIII) 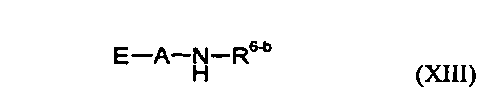 in welcher
   E, A und R^{6-b} die oben angegebenen Bedeutungen haben,
   mit einem Chlorameisensäureester der Formel (XIV) 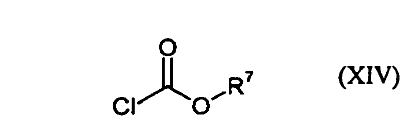 in welcher
   R⁷ die oben angegebenen Bedeutungen hat,
   in Gegenwart von N,O-Bis(trimethylsilyl)acetamid und gegebenenfalls in Gegenwart eines Verdünnungsmittels (z.B. Dichlormethan) umsetzt (vgl. Syn. Commun. 1985, 15, 1025-1031).

Die nach Verfahren (b) herstellbaren (Hetero)cyclen sind durch die Formel (III-b) allgemein definiert. In dieser Formel steht A bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. E steht bevorzugt, besonders bevorzugt bzw. ganz besonders bevorzugt für diejenigen Bedeutungen, die bereits in Zusammenhang mit der Beschreibung der erfindungsgemäßen Stoffe der Formel (III) für diese Reste als bevorzugt, besonders bevorzugt etc. genannt wurden. R^{6-b} steht bevorzugt für C₁-C₆-Alkyl, besonders bevorzugt für C₁-C₄-Alkyl, ganz besonders bevorzugt für Methyl, Ethyl, n-Propyl, i-Propyl, n-Butyl, i-Butyl, s-Butyl oder t-Butyl.

Die bei der Durchführung des Verfahrens (b) als Ausgangsstoffe benötigten Amine sind durch die Formel (XIII) allgemein definiert. In dieser Formel stehen E und A bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. R^{6-b} steht bevorzugt für C₁-C₆-Alkyl, besonders bevorzugt für C₁-C₄-Alkyl, ganz besonders bevorzugt für Methyl, Ethyl, n-Propyl, i-Propyl, n-Butyl, i-Butyl, s-Butyl oder t-Butyl.

Amine der Formel (XIII) sind bekannt.

Die bei der Durchführung des Verfahrens (b) als Ausgangsstoffe benötigten Chlorameisensäureester sind durch die Formel (XIV) allgemein definiert. In dieser Formel steht R⁷ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden.

Chlorameisensäureester der Formel (XIV) sind bekannt.

### (Hetero)cyclen der Formel (III-c)

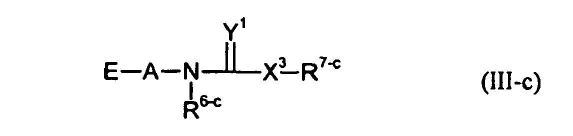

in welcher
E, A und Y¹ die oben angegebenen Bedeutungen haben,
- X³: für O (Sauerstoff) steht,
- R^{6-c} und R^{7-c}: gemeinsam für gegebenenfalls einfach oder mehrfach durch Alkyl substituiertes Alkylen stehen,
lassen sich beispielsweise herstellen, indem man
c) Isocyanate der Formel (X)

   E-A-N=C=O (X)

   in welcher
   E und A die oben angegebenen Bedeutungen haben,
   mit einem Dioxolan der Formel (XV) 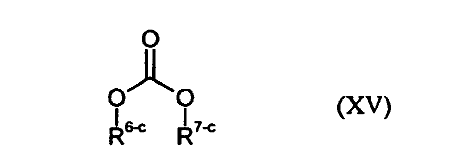 in welcher
   R^{6-c} und R^{7-c} die oben angegebenen Bedeutungen haben,
   gegebenenfalls in Gegenwart eines Säurebindemittels (z.B. Caesiumfluorid) und gegebenenfalls in Gegenwart eines Verdünnungsmittels (z.B. Dimethylsulfoxid) umsetzt (vgl. JP 2000-2902635).

Die nach Verfahren (c) herstellbaren (Hetero)cyclen sind durch die Formel (III-c) allgemein definiert. In dieser Formel steht A bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. E steht bevorzugt, besonders bevorzugt bzw. ganz besonders bevorzugt für diejenigen Bedeutungen, die bereits in Zusammenhang mit der Beschreibung der erfindungsgemäßen Stoffe der Formel (III) für diese Reste als bevorzugt, besonders bevorzugt etc. genannt wurden. R^{6-c} und R^{7-c} stehen gemeinsam bevorzugt für gegebenenfalls einfach bis vierfach durch C₁-C₄-Alkyl substituiertes C₂-C₄-Alkylen, besonders bevorzugt für gegebenenfalls einfach bis dreifach durch C₁-C₄-Alkyl substituiertes C₂-C₃-Alkylen, ganz besonders bevorzugt für einfach oder zweifach durch Methyl, Ethyl, n-Propyl oder i-Propyl substituiertes Methylen oder Ethylen.

Die bei der Durchführung des Verfahrens (c) als Ausgangsstoffe benötigten Isocyanate der Formel (X) wurden bereits in Zusammenhang mit der Erläuterung des Verfahrens (a) beschrieben.

Die bei der Durchführung des Verfahrens (c) als Ausgangsstoffe benötigten Dioxolane sind durch die Formel (XV) allgemein definiert. In dieser Formel stehen R^{6-c} und R^{7-c} bevorzugt, besonders bevorzugt bzw. ganz besonders bevorzugt für diejenigen Bedeutungen, die bereits in Zusammenhang mit der Beschreibung der Stoffe der Formel (III-c) für diese Reste als bevorzugt, besonders bevorzugt etc. genannt wurden.

Dioxolane der Formel (XV) sind bekannt.

Als Diboronsäureester kommen bei der Durchführung des erfindungsgemäßen Verfahrens (A) 4,4,4',4',5,5,5',5'-Octamethyl-2,2'-bi-1,3,2-dioxaborolan, 5,5,5',5'-Tetramethyl-2,2'-bi-1,3,2-dioxaborinan, 4,4,4',4',6,6'-Hexamethyl-2,2'-bi-1,3,2-dioxaborinan oder 2,2'-Bi-1,3,2-benzodioxaborol infrage. Bevorzugt verwendet man 4,4,4',4',-5,5,5',5'-Octamethyl-2,2'-bi-1,3,2-dioxaborolan, 5,5,5',5'-Tetramethyl-2,2'-bi-1,3,2-dioxaborinan oder 4,4,4',4',6,6'-Hexamethyl-2,2'-bi-1,3,2-dioxaborinan, besonders bevorzugt 4,4,4',4',5,5,5',5'-Octamethyl-2,2'-bi-1,3,2-dioxaborolan oder 5,5,5',5'-Tetramethyl-2,2'-bi-1,3,2-dioxaborinan, ganz besonders bevorzugt 4,4,4',4',5,5,5',5'-Octamethyl-2,2'-bi-1,3,2-dioxaborolan.

Bei der Durchführung des erfindungsgemäßen Verfahrens (A) setzt man auf 1 Mol an Verbindung der Formel (II) im allgemeinen 1 Mol oder einen leichten Überschuss eines Diboronesters und 1 Mol oder einen leichten Überschuss einer Verbindung der Formel (III), sowie 3 % eines Palladiumkatalysators ein. Es ist jedoch auch möglich, die Reaktionskomponenten in anderen Verhältnissen einzusetzen. Es kann wahlweise die Verbindung der Formel (II) oder die Verbindung der Formel (III) zuerst vorgelegt werden. Die Aufarbeitung erfolgt nach üblichen Methoden. Im allgemeinen verfährt man in der Weise, dass man das Reaktionsgemisch mit Wasser verdünnt und mit Essigsäureethylester extrahiert. Die organische Phase wird gewaschen, getrocknet, filtriert und eingeengt. Der Rückstand wird gegebenenfalls nach üblichen Methoden, wie Chromatographie oder Umkristallisation, von eventuell noch vorhandenen Verunreinigungen befreit.

### Verfahren (B)

Die bei der Durchführung des erfindungsgemäßen Verfahrens (B) als Ausgangsstoffe benötigten Δ¹-Pyrroline sind durch die Formel (IV) allgemein definiert. In dieser Formel stehen R¹, R², R⁴ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Z² steht bevorzugt für (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl, besonders bevorzugt für (4,4,5,5-Tetramethyl-1,3,2-dioxaborolan)-2-yl, (5,5-Dimethyl-1,3,2-dioxaborinan)-2-yl oder (4,4,6-Trimethyl-1,3,2-dioxaborinan)-2-yl, ganz besonders bevorzugt für (4,4,5,5-Tetramethyl-1,3,2-dioxaborolan)-2-yl, (5,5-Dimethyl-1,3,2-dioxaborinan)-2-yl.

Δ¹-Pyrroline der Formel (IV) lassen sich herstellen, indem man
d) Verbindungen der Formel (II) 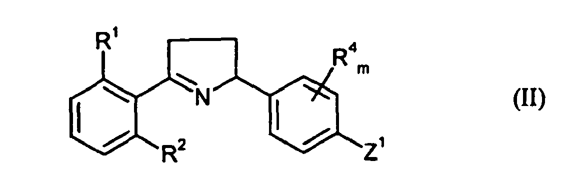 in welcher
   R¹, R², R⁴, m und Z¹ die oben angegebenen Bedeutungen haben,
   mit einem Diboronsäureester in Gegenwart eines Katalysators, gegebenenfalls in Gegenwart eines Säurebindemittels und gegebenenfalls in Gegenwart eines Verdünnungsmittels umsetzt (vgl. J. Org. Chem. 1995, 60, 7508; Tetrahedron Lett. 1997, 38, 3447).

Geeignete Diboronsäureester zur Durchführung des Verfahrens (d) sind bei der Beschreibung des erfindungsgemäßen Verfahrens (A) bereits genannt worden.

Die bei der Durchführung des erfindungsgemäßen Verfahrens (B) als Ausgangsstoffe benötigten Heterocyclen der Formel (III) wurden bereits oben bei der Beschreibung des Verfahrens (A) beschrieben.

Bei der Durchführung des erfindungsgemäßen Verfahrens (B) setzt man auf 1 Mol an Verbindung der Formel (V) im allgemeinen 1 Mol oder einen leichten Überschuss an einer Verbindung der Formel (III) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (C)

Die bei der Durchführung des erfindungsgemäßen Verfahrens (C) als Ausgangsstoffe benötigten Δ¹-Pyrroline der Formel (II) wurden bereits bei der Beschreibung des Verfahrens (A) beschrieben.

Die bei der Durchführung des erfindungsgemäßen Verfahrens (C) als Ausgangsstoffe benötigten Boronsäure-Derivate sind durch die Formel (V) allgemein definiert. In dieser Formel steht Y¹ bevorzugt für O (Sauerstoff). X¹ steht bevorzugt für O (Sauerstoff) oder NR⁸. Z² steht bevorzugt für (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl, besonders bevorzugt für (4,4,5,5-Tetramethyl-1,3,2-dioxaborolan)-2-yl, (5,5-Dimethyl-1,3,2-dioxaborinan)-2-yl oder (4,4,6-Trimethyl-1,3,2-dioxaborinan)-2-yl, ganz besonders bevorzugt für (4,4,5,5-Tetramethyl-1,3,2-dioxaborolan)-2-yl, (5,5-Dimethyl-1,3,2-dioxaborinan)-2-yl. A, R⁶, R⁷ und R⁸ stehen bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden.

Die Verbindungen der Formel (V) sind bekannt oder lassen sich nach bekannten Verfahren herstellen.

Bei der Durchführung des erfindungsgemäßen Verfahrens (C) setzt man auf 1 Mol an Verbindung der Formel (II) im allgemeinen 1 Mol oder einen leichten Überschuss einer Verbindung der Formel (V) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (D)

Die bei der Durchführung des erfindungsgemäßen Verfahrens (D) als Ausgangsstoffe benötigten Δ¹-Pyrroline sind durch die Formel (II-a) allgemein definiert. In dieser Formel stehen R¹, R², R⁴ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Z³ steht bevorzugt für Brom oder Iod.

Δ¹-Pyrroline der Formel (II-a) lassen sich nach bekannten Verfahren herstellen (vgl. WO 98/22438).
Die bei der Durchführung des erfindungsgemäßen Verfahrens (D) als Ausgangsstoffe benötigten organometallischen Verbindungen sind durch die Formel (VI) allgemein definiert. In dieser Formel steht Y¹ bevorzugt für O (Sauerstoff). X¹ steht bevorzugt für O (Sauerstoff) oder NR⁸. M steht bevorzugt für ZnCl, Sn(Me)₃ oder Sn(n-Bu)₃. A, R⁶, R⁷ und R⁸ stehen bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden.

Organometallische Verbindungen der Formel (VI) sind teilweise bekannt oder können nach bekannten Methoden hergestellt werden. Es ist z.B. möglich, Verbindungen der Formel (VI) aus den entsprechenden Verbindungen der Formel (III), in welchen X für -OSO₂CF₃ steht, in situ herzustellen (vgl. Tetrahedron Lett. 1995, 36, 9085).

Bei der Durchführung des erfindungsgemäßen Verfahrens (D) setzt man auf 1 Mol an Verbindung der Formel (II-a) im allgemeinen 1 Mol oder einen leichten Überschuss einer Verbindung der Formel (VI) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Herstellung von Ausgangsstoffen für die Verfahren (A), (B), (C) und (D)

Die bei der Durchführung der erfindungsgemäßen Verfahren (A), (B), (C) und (D) als Ausgangsstoffe benötigten Δ¹-Pyrroline der Formeln (II), (IV) und (II-a) lassen sich auch herstellen, indem man
e) Amide der Formel (XVI) 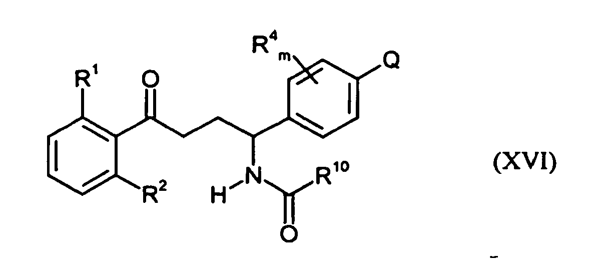
in welcher
- Q: für Z¹,Z² oder Z³ steht,
- R¹⁰: für Alkyl, Halogenalkyl, Phenyl oder Benzyl steht,
R¹, R², R⁴, m, Z¹, Z² und Z³ die oben angegebenen Bedeutungen haben,
mit einem N-Entacylierungsmittel in Gegenwart eines Verdünnungsmittels umsetzt.

Die bei der Durchführung des Verfahrens (e) als Ausgangsstoffe benötigten Amide sind durch die Formel (XVI) allgemein definiert. In dieser Formel stehen R¹, R², R⁴ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Q steht für Z¹, Z² oder Z³ bzw. für die bevorzugten, besonders bevorzugten und ganz besonders bevorzugten Bedeutungen dieser Reste, die bereits oben beschrieben wurden. R¹⁰ steht bevorzugt für C₁-C₄-Alkyl, C₁-C₄-Halogenalkyl, Phenyl oder Benzyl, besonders bevorzugt für Methyl, Ethyl, Phenyl oder Benzyl, ganz besonders bevorzugt Methyl, Phenyl oder Benzyl.

Die zur Durchführung des Verfahrens (e) als Ausgangsstoffe benötigten Amide der Formel (XVI) lassen sich herstellen, indem man
f) Cyclopropane der Formel (XVII) 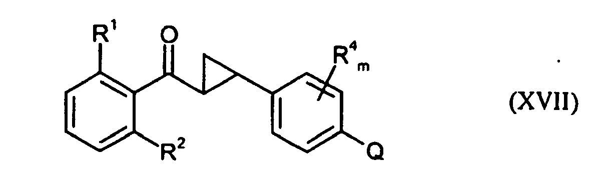
in welcher R¹, R², R⁴, m und Q die oben angegebenen Bedeutungen haben,
mit Nitrilen der Formel (XVIII)

R¹⁰-CN (XVIII)

in welcher R¹⁰ die oben angegebenen Bedeutungen hat,
und einer Protonsäure oder Trimethylsilyltetrafluorborat umsetzt.

Die bei der Durchführung des Verfahrens (f) als Ausgangsstoffe benötigten Cyclopropane sind durch die Formel (XVII) allgemein definiert. In dieser Formel stehen R¹, R², R⁴ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt bzw. ganz besonders bevorzugt genannt wurden. Q steht für Z¹, Z² oder Z³ bzw. für die bevorzugten, besonders bevorzugten und ganz besonders bevorzugten Bedeutungen dieser Reste, die bereits oben beschrieben wurden.

Die bei der Durchführung des Verfahrens (f) als Ausgangsstoffe benötigten Nitrile sind durch die Formel (XVIII) allgemein definiert. In dieser Formel steht R¹⁰ steht bevorzugt für C₁-C₄-Alkyl, C₁-C₄-Halogenalkyl, Phenyl oder Benzyl, besonders bevorzugt für Methyl, Ethyl, Phenyl oder Benzyl, ganz besonders bevorzugt Methyl, Phenyl oder Benzyl.

Als Protonsäuren kommen bei der Durchführung des Verfahrens (f) alle üblicherweise für diesen Zweck einsetzbaren Säuren in Betracht. Vorzugsweise verwendbar ist Schwefelsäure.

Als Trimethylsilyltetrafluorborat kommt bei der Durchführung des Verfahrens (f) die Verbindung der Formel (XIX)

Me₃Si-N=C⁺-CH₃ BF₄⁻ (XIX)

in Betracht. Das Reagenz der Formel (XIX) ist bekannt (vgl. Tetrahedron Lett. 1984, 25, 577-578).

Die Reaktionstemperaturen zur Durchführung des Verfahren (f) können in einem größeren Bereich variiert werden. Im allgemeinen arbeitet man bei Temperaturen zwischen -20°C und +60°C, vorzugsweise zwischen -10°C und 30°C.

Die zur Durchführung des Verfahrens (f) als Ausgangsstoffe benötigten Cyclopropane der Formel (XVII) lassen sich herstellen, indem man
g) Chalcone der Formel (XX) 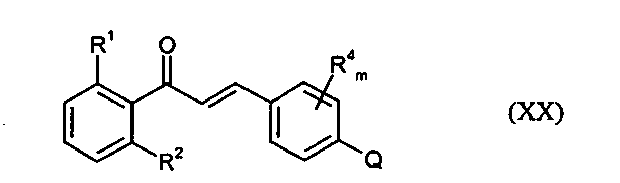
in welcher
R¹, R², R⁴, m und Q die oben angegebenen Bedeutungen haben,
mit einem Trialkylsulfoxoniumylid in Gegenwart einer Base und gegebenenfalls in Gegenwart eines Verdünnungsmittels umgesetzt.

Die bei der Durchführung des Verfahrens (g) als Ausgangsstoffe benötigten Chalcone sind durch die Formel (XX) allgemein definiert. In dieser Formel stehen R¹, R², R⁴ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt bzw. ganz besonders bevorzugt genannt wurden. Q steht für Z¹, Z² oder Z³ bzw. für die bevorzugten, besonders bevorzugten und ganz besonders bevorzugten Bedeutungen dieser Reste, die bereits oben beschrieben wurden.

Als Trialkylsulfoxoniumylid wird bei der Durchführung des Verfahrens (g) bevorzugt Trimethylsulfoxoniumylid eingesetzt.

Als Basen können bei der Durchführung des Verfahrens (g) Alkalimetall-hydride, -alkoholate und -hydroxide eingesetzt werden. Vorzugsweise verwendet man Natriumhydrid, Kalium-2-methyl-2-propanolat, Natriummethanolat oder Kaliumhydroxid, besonders bevorzugt Natriumhydrid.

Als Verdünnungsmittel kommen bei der Durchführung des Verfahrens (g) Dimethylsulfoxid, Tetrahydrofuran, Acetonitril, Toluol oder Diethylenglykol, sowie Gemische davon, in Frage. Vorzugsweise verwendet man Dimethylsulfoxid (vgl. Tetrahedron Asymmetry 1998, 9, 1035).

Die Reaktionstemperaturen zur Durchführung des erfindungsgemäßen Verfahren (g) können in einem größeren Bereich variiert werden. Im allgemeinen arbeitet man bei Temperaturen zwischen -20°C und +120°C, vorzugsweise zwischen 0°C und 60°C, besonders bevorzugt zwischen 20°C und 40°C.

Die zur Durchführung des Verfahrens (g) als Ausgangsstoffe benötigten Chalcone der Formel (XX) sind bekannt.

Bei der Durchführung des Verfahrens (e) werden zur N-Entacylierung der Amide der Formel (XVI) bei der Umsetzung zu Pyrrolinen der Formeln (II), (IV), und (II-a) Protonsäuren (vgl. J. Org. Chem. 1978, 43, 4593), anorganische Basen (vgl. J. Chem. Soc. 1964, 4142), Hydrazine (vgl. J. Org. Chem. 1978, 43, 3711) oder Biotransformationen mit Enzymen (vgl. Appl. Microbiol. Biotechnol. 1997, 47, 650) verwendet. Andere übliche Verfahren zur Entacylierung von Amiden sind beschrieben bei T. W. Greene, P. G. M. Wuts, Protective Groups in Organic Synthesis (Ed. 3, New York, Wiley 1999, S. 553-555).

Als N-Entacylierungsmittel werden bevorzugt Protonsäuren oder organische Säuren, besonders bevorzugt wässrige Salzsäure, wässrige Bromwasserstoffsäure oder Trifluoressigsäure, ganz besonders bevorzugt wässrige Salzsäure; bevorzugt anorganische Basen, besonders bevorzugt Bariumhydroxid [Ba(OH)₂] und Natriumhydroxid (NaOH) und bevorzugt Biotransformationen, besonders bevorzugt unter Einsatz von Acylasen, verwendet.

Bei der N-Entacylierung mittels Biotransformationen erhält man die Verbindungen der Formeln (II), (IV), und (II-a) mit einer der beiden Enantiomeren im Überschuss.

Als Verdünnungsmittel kommen bei der Durchführung des Verfahrens (e) Wasser oder Alkohole und Gemische von diesen in Betracht. Bevorzugt verwendet man Wasser, Methanol oder Ethanol oder Gemische aus zwei oder drei dieser drei Verdünnungsmittel.

Die Reaktionstemperaturen bei der Durchführung des Verfahrens (e) können in einem größeren Bereich variiert werden. Im allgemeinen arbeitet man bei Temperaturen zwischen 20°C und 200°C, vorzugsweise zwischen 60°C und 140°C, besonders bevorzugt zwischen 80°C und 120°C. Wird die N-Entacylierung enzymatisch unter Einsatz von Acylasen durchgeführt, arbeitet man im allgemeinen zwischen 20°C und 60°C, vorzugsweise zwischen 20°C und 40°C.

Bei der Durchführung des Verfahrens (e) setzt man im allgemeinen auf 1 Volumenanteil einer 10%-igen (w/v) alkoholischen Lösung an Amid der Formel (XVI) 2 Volumenanteile einer Protonsäure ein. Es können aber auch andere Verhältnisse der Reaktionskomponenten gewählt werden. Die Aufarbeitung erfolgt nach üblichen Methoden. Im allgemeinen geht man in der Weise vor, dass man mit Natronlauge neutralisiert und anschließend mit Essigsäureethylester extrahiert, die organische Phase trocknet, filtriert und einengt.

### Verfahren (E)

Die bei der Durchführung des erfindungsgemäßen Verfahrens (E) als Ausgangsstoffe benötigten Δ¹-Pyrroline sind durch die Formel (VII) allgemein definiert. In dieser Formel stehen R¹, R², A, R⁴ und m bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden.

Δ¹-Pyrroline der Formel (VII) sind neu. Sie lassen sich herstellen, indem man

### h) Δ¹-Pyrroline der Formel (I-b)

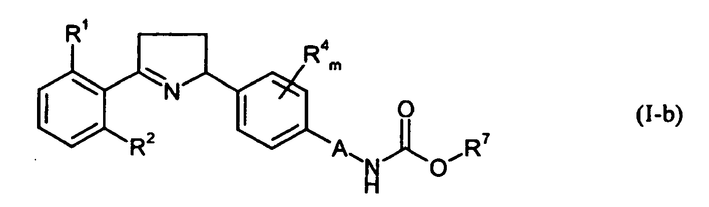

in welcher
R¹, R², A, R⁴, m und R⁷ die oben angegebenen Bedeutungen haben,
mit einer Mineralsäure (z.B. Salzsäure, Schwefelsäure) oder einer Base (z.B. Natriumhydroxid, Kaliumhydroxid) gegebenenfalls in Gegenwart eines Verdünnungsmittels (z.B. Alkohole wie Methanol, Ethanol oder Wasser oder Gemische aus diesen) umsetzt.

Die bei der Durchführung des Verfahrens (h) als Ausgangsstoffe benötigten Δ¹-Pyrroline der Formel (I-b) sind eine Untergruppe der erfindungsgemäßen Verbindungen der Formel (I). In der Formel (I-b) stehen R¹, R², A, R⁴, m und R⁷ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Δ¹-Pyrroline der Formel (I-b) können nach einem der erfindungsgemäßen Verfahren (A), (B), (C) oder (D) hergestellt werden.

Die bei der Durchführung des erfindungsgemäßen Verfahrens (E) als Ausgangsstoffe benötigten Iso(thio)cyanate sind durch die Formel (VIII) allgemein definiert. In dieser Formel stehen Y und R⁷ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden.

Iso(thio)cyanate der Formel (VIII) sind bekannt und/oder lassen sich nach bekannten Verfahren herstellen.

Die bei der Durchführung des erfindungsgemäßen Verfahrens (E) als Ausgangsstoffe benötigten (Thio)carbonate sind durch die Formel (IX) allgemein definiert. In dieser Formel stehen Y und R⁷ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. X² steht bevorzugt für O (Sauerstoff) oder S (Schwefel).

(Thio)carbonate der Formel (IX) sind bekannt und/oder lassen sich nach bekannten Verfahren herstellen.

Bei der Durchführung des erfindungsgemäßen Verfahrens (E) setzt man auf 1 Mol an Verbindung der Formel (VII) im allgemeinen 1 Mol oder einen leichten Überschuss einer Verbindung der Formel (VIII) bzw. 1 Mol oder einen leichten Überschuss einer Verbindung der Formel (IX) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit ° Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Chirale Verbindungen der Formel (I-a)

Zur Herstellung chiraler Verbindungen der Formel (I-a) können beispielsweise Δ¹-Pyrroline der Formel (II-b) 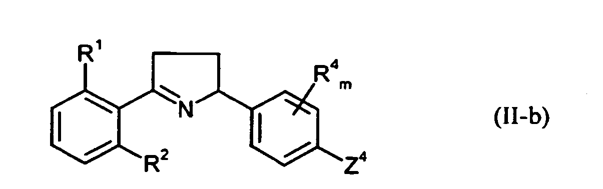 in welcher
R¹, R², R⁴ und m die oben angegebenen Bedeutungen haben,
- Z⁴: für Chlor, Brom oder Iod steht,
einer Racematspaltung unterzogen werden. Dabei arbeitet man beispielsweise nach Methoden der präparativen Chromatographie, vorzugsweise nach der Methode der High Performance Liquid Chromatography (HPLC). Dabei wird eine chirale stationäre Kieselgelphase verwendet. Als besonders geeignet für die Trennung der Verbindungen der Formel (II-b) in die beiden Enantiomere hat sich ein mit Tris(3,5-di-methylphenylcarbamat)-cellulose modifiziertes Kieselgel erwiesen. Dieses Trennmaterial ist kommerziell erhältlich. Es ist aber auch möglich, andere stationäre Phasen zu verwenden. Als Eluenten kommen alle üblichen inerten, organischen Solventien sowie Gemische von diesen in Frage. Vorzugsweise verwendbar sind gegebenenfalls halogenierte aliphatische, alicyclische oder aromatische KohlenWasserstoffe, wie Petrolether, Hexan, Heptan, Cyclohexan; Dichlormethan, Chloroform; Alkohole, wie Methanol, Ethanol, Propanol; Nitrile, wie Acetonitril; Ester wie Essigsäuremethylester oder Essigsäureethylester. Besonders bevorzugt verwendet man aliphatische Kohlen Wasserstoffe, wie Hexan oder Heptan, und Alkohole, wie Methanol oder Propanol, ganz besonders bevorzugt n-Heptan und Isopropanol bzw. Gemische von diesen. Im allgemeinen arbeitet man bei Temperaturen zwischen 10°C und 60°C, vorzugsweise zwischen 10°C und 40°C, besonders bevorzugt bei Raumtemperatur. Die auf diesem Wege erhaltenen (R)-konfigurierten Enantiomere werden dann als Ausgangsstoffe für die Verfahren (A), (C) oder (D) eingesetzt.

Bei der Durchführung der erfindungsgemäßen Verfahren (A), (B), (C) und (D) setzt man jeweils einen Palladium-Katalysator ein, der wiederum mit oder ohne Zusatz von weiteren Liganden verwendet werden kann. Vorzugsweise verwendet man als Katalysator PdCl₂(dppf) [dppf = 1,1'-Bis(diphenylphosphino)ferrocene], Pd(PPh₃)₄, PdCl₂(PPh₃)₂, PdCl₂(CH₃CN)₂, Pd₂(dba)₃ [dba = Dibenzylidenaceton] oder Pd(OAc)₂, besonders bevorzugt PdCl₂(dppf), Pd(PPh₃)₄, PdCl₂(PPh₃)₂, oder Pd(OAc)₂, ganz besonders bevorzugt PdCl₂(dppf) oder PdCi₂(PPh₃)₂.

Als Liganden kommen Triarylphosphine, Trialkylphosphine oder Arsine in Frage. Vorzugsweise verwendet man dppf, PPh₃, P(t-Bu)₃, Pcy₃ oder AsPh₃, besonders bevorzugt dppf.

Als Verdünnungsmittel kommen bei der Durchführung der erfindungsgemäßen Verfahren (A), (B) und (C) jeweils alle üblichen inerten, organischen Solventien in Frage. Vorzugsweise verwendbar sind gegebenenfalls halogenierte aliphatische, alicyclische oder aromatische Kohlen Wasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan. Besonders bevorzugt verwendet man Aceton, Dimethoxyethan, Dioxan, Tetrahydrofuran, Dimethylformamid, Dimethylacetamid, Dimethylsulfoxid, Ethanol, Toluol oder gegebenenfalls Gemische dieser genannten Verdünnungsmittel mit Wasser.

Als Verdünnungsmittel kommen bei der Durchführung der erfindungsgemäßen Verfahren (D) und (E) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol. Besonders bevorzugt verwendet man bei der Durchführung des erfindungsgemäßen Verfahrens (D) Dioxan, Tetrahydrofuran oder Toluol.

Als Säurebindemittel kommen bei der Durchführung der erfindungsgemäßen Verfahren (A), (B), (C) und (D) jeweils alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, Alkalimetallfluoride, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert. Besonders bevorzugt verwendet man Bariumhydroxid, Natriumhydroxid, Kaliumhydroxid, Trikaliumphosphat, Caesiumcarbonat, Kaliumcarbonat, Natriumcarbonat, Kaliumacetat, Triethylamin, Kalium-tert-butanolat, Caesiumfluorid oder Kaliumfluorid.

Als Säurebindemittel kommen bei der Durchführung des erfindungsgemäßen Verfahren (E) jeweils alle für derartige Reaktionen üblichen anorganischen und organischen Basen in Betracht. Vorzugsweise verwendbar sind Alkalimetallcarbonate, wie Natriumcarbonat, Kaliumcarbonat, Kaliumhydrogencarbonat, Natriumhydrogencarbonat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten.

Die Reaktionstemperaturen können bei der Durchführung der erfindungsgemäßen Verfahren (A), (B) und (C) jeweils in einem größeren Bereich variiert werden. Im allgemeinen arbeitet man bei Temperaturen zwischen 0°C und 140°C, vorzugsweise zwischen 20°C und 120°C, besonders bevorzugt zwischen 60°C und 100°C.

Die Reaktionstemperaturen können bei der Durchführung des erfindungsgemäßen Verfahrens (D) jeweils in einem größeren Bereich variiert werden. Im allgemeinen arbeitet man bei Temperaturen zwischen 0°C und 140°C, vorzugsweise zwischen 20°C und 120°C.

Die Reaktionstemperaturen können bei der Durchführung des erfindungsgemäßen Verfahrens (E) jeweils in einem größeren Bereich variiert werden. Im allgemeinen arbeitet man bei Temperaturen zwischen 0°C und 100°C, vorzugsweise zwischen 20°C und 50°C.

Bei der Durchführung aller erfindungsgemäßen Verfahren arbeitet man im allgemeinen unter Atmosphärendruck. Es ist aber auch möglich, jeweils unter erhöhtem oder vermindertem Druck zu arbeiten.

Die erfindungsgemäßen Wirkstoffe eignen sich bei guter Pflanzenverträglichkeit und günstiger Warrnblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca. spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen der Formel (I) zeichnen sich insbesondere durch eine hervorragende Wirkung gegen Raupen, Käferlarven, Spinnmilben, Blattläuse und Minierfliegen aus.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der erfindungsgemäßen Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische KohlenWasserstoffe, wie Chlorbenzole, Chlorethylene oder Methylenchlorid, aliphatische KohlenWasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln;
als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate;
als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.
Die Formulierungen enthalten im allgemeinen zwischen 0,1 und 95 Gew.% Wirkstoff, vorzugsweise zwischen 0,5 und 90 %.

Die erfindungsgemäßen Wirkstoffe können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlen Wasserstoffe, Phenylharnstoffe, durch Mikroorganismen hergestellte Stoffe u.a.

Besonders günstige Mischpartner sind z.B. die folgenden:

### Fungizide:

Aldimorph, Ampropylfos, Ampropylfos-Kaliurn, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Guazatin, Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb,
Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G, OK-8705, OK-8801,
α-(1,1-Dimethylethyl)-β-(2-phenoxyethyl)-1H-1 1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)-ethyl]-amino]-carbonyl]-propyl} -carbaminsäure-1 - isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-0-(4-0-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid, 2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1 H-inden-2-carbonitril,
3-(2-(4-Chlorphenyl)-5-ethoxy-3-isoxazolidinyl]-pyridin,
4-Chlor-2-cyan-N,N-dimethyl-5-(4-methylphenyl)-1 H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin, 8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-metharumidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2, 1'(3'H)-isobenzofuran]-3'-on,

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermedirin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine, Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Eprinomectin, Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene, Imidacloprid, Isazofos, Isofenphos, Isoxathion, Ivermectin,
Kempolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos, Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Selamectin, Silafluofen, Spinosad, Sulfotep, Sulprofos, Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenytethoxy)-phenyl]-amino]-carbonyl]-benzamid
2-Chlor-N-[[[4-(2,2-dichlor-1, 1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(l,l-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat N-(3,4,4-Trifluor-1-oxo-3-butenyl)-glycin
N-(4-Chlorphenyl)-3-[4-(difluorimethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der erfindungsgemäßen Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch über additive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.. Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..
Beispielsweise zeigen sie eine hervorragende Wirksamkeit gegen die Entwicklungsstadien von Zecken wie zum Beispiel Amblyomma hebraeum, gegen parasitierende Fliegen wie zum Beispiel gegen Lucilia cuprina, gegen Flöhe wie zum Beispiel Ctenocephalides felis

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die erfindungsgemäßen Wirkstoffe der Formel (I) als Formulierungen (beispielsweise Pulver, Emulsionen, fließfähige Mittel), die die erfindungsgemäßen Wirkstoffe in einer Menge von 1 bis 80 Gew.-% enthalten, direkt oder nach 100 bis 10 000-facher Verdünnung anwenden oder sie als chemisches Bad verwenden. Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die erfindungsgemäßen Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der erfindungsgemäßen Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische KohlenWasserstoffe mit einem Siedebereich von 180 bis 210°C oder hochsiedende Gemische von aromatischen und aliphatischen KohlenWasserstoffen mit einem Siedebereich von 180 bis 220°C und/oder Spindelöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, KohlenWasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-lod-2-propinyl-butyl-carbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.
Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-lod-2-propinyl-butylcarbamat, Tolylfluanid und Azole wie
   Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb; oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten des weiteren die üblichen Bestandteile wie z.B. in Ungerer, *Chem. Ind.* **1985**, 37, 730-732 und Williams, Antifouling Marine Coatings, Noyes, Park Ridge, **1973** beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die erfindungsgemäßen Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga camaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die Herstellung und die Verwendung der erfindungsgemäßen Stoffe geht aus den folgenden Beispielen hervor.

### Herstellungsbeispiele

### Beispiel 1

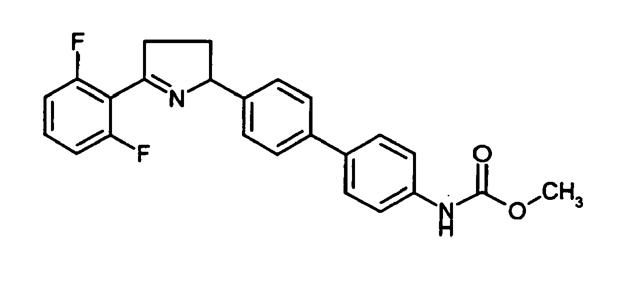

5-(2,6-Difluorphenyl)-2-[4-(4,4,5,5-tetramethyl-1,3,2-dioxaborolan-2-yl)phenyl]-3,4-dihydro-2H-pyrrol (0.96 g, 2.50 mmol) wird in 10 ml N,N-Dimethylacetamid gelöst. Man gibt nacheinander Methyl-4-bromphenylcarbamat (III-1) (0.69 g, 3.00 mmol), PdCl₂[dppf] (0.05 g, 0.07 mmol) und 3.75 ml Natriumcarbonat-Lösung (2 M) zu und rührt anschließend 16 h bei 80°C. Man lässt auf Raumtemperatur abkühlen, extrahiert mit Wasser/Essigsäureethylester, trennt die organische Phase ab, trocknet über Magnesiumsulfat, filtriert und engt unter vermindertem Druck ein. Das Rohprodukt wird durch Chromatographie an Kieselgel (Cyclohexan/Essigsäureethylester 91:9 → 80:20, jeweils v/v) aufgereinigt.

Man erhält 0.35 g (32% d. Th.) an Methyl-4'-[5-(2,6-difluorophenyl)-3,4-dihydro-2H-pyrrol-2-yl]-1,1'-biphenyl-4-yl-carbamat.
- HPLC:: Log P (pH 2.3) = 2.31 (Reinheit: 93 %)
Log P (pH 7.5) = 3.52

### Beispiel 2

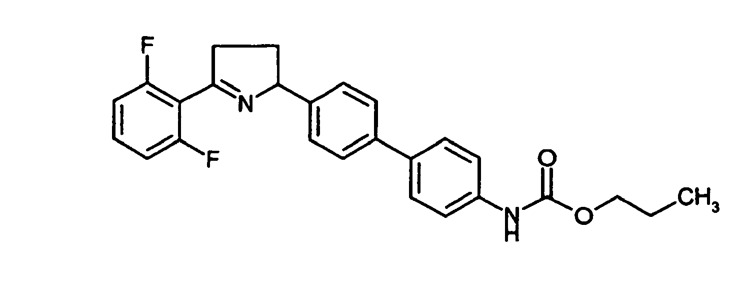

5-(2,6-Difluorphenyl)-2-[4-(4,4,5,5-tetramethyl-1,3,2-dioxaborolan-2-yl)phenyl]-3,4-dihydro-2H-pyrrol (0.96 g, 2.50 mmol) und n-Propyl-4-bromphenylcarbamat (III-2) (0.77 g, 3.00 mmol) werden in 10 ml 1,2-Dimethoxyethan gelöst. Man gibt nacheinander PdCl₂[dppf] (0.05 g, 0.07 mmol) und 3.75 ml Natriumcarbonat-Lösung (2 M) zu und rührt anschließend 16 h bei 80°C. Man lässt auf Raumtemperatur abkühlen, extrahiert mit Wasser/Essigsäureethylester, trennt die organische Phase ab, trocknet über Magnesiumsulfat, filtriert und engt unter vermindertem Druck ein. Das Rohprodukt wird durch Chromatographie an Kieselgel (CyclohexanlEssigsäureethylester 20:1 → 6:1 → 2:1, jeweils v/v) aufgereinigt.

Man erhält 0.28 g (25% d. Th.) an n-Propyl-4'-[5-(2,6-difluorophenyl)-3,4-dihydro-2H-pyrrol-2-yl]-1,1'-biphenyl-4-yl-carbamat.
- HPLC:: Log P (pH 2.3) = 3.02 (Reinheit: 98 %)
Log P (pH 7.5) = 4.24

### Beispiel 3

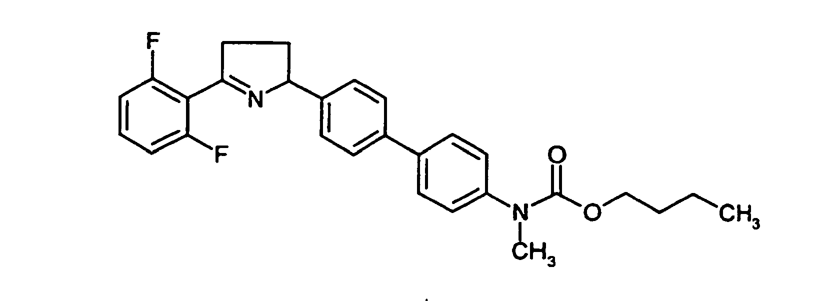

5-(2,6-Difluorphenyl)-2-[4-(4,4,5,5-tetramethyl-1,3,2-dioxaborolan-2-yl)phenyl]-3,4-dihydro-2H-pyrrol (0.96 g, 2.50 mmol)) wird in 20 ml N,N-Dimethylacetamid gelöst. Man gibt nacheinander n-Butyl-4-bromphenyl(methyl)carbamat (III-3) (0.86 g, 3.00 mmol), PdCl₂[dppf] (0.05 g, 0.07 mmol) und 3.75 ml Natriumcarbonat-Lösung (2 M) zu und rührt anschließend 16 h bei 90°C. Man lässt auf Raumtemperatur abkühlen und engt das Reaktionsgemisch unter vermindertem Druck ein. Der Rückstand wird in Essigsäureethylester aufgenommen und mit Wasser gewaschen. Die organische Phase wird abgetrennt, über Magnesiumsulfat getrocknet, filtriert und unter vermindertem Druck eingeengt. Das Rohprodukt wird durch Chromatographie an Kieselgel (Cyclohexan/Essigsäureethylester 10:1 → 6:1, jeweils v/v) aufgereinigt.

Man erhält 0.48 g (41% d. Th.) an n-Butyl-4'-[5-(2,6-difluorphenyl)-3,4-dihydro-2H-pyrrol-2-yl]-1,1'-biphenyl-4-yl(methyl)carbamat.
- HPLC:: Log P (pH 2.3) = 3.76 (Reinheit: 98 %)
Log P (pH 7.5) = 4.92

### Beispiel 4

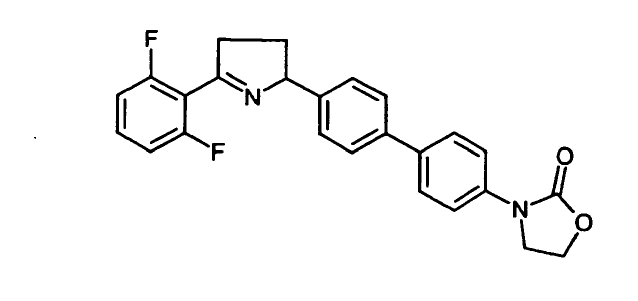

5-(2,6-Difluorphenyl)-2-[4-(4,4,5,5-tetramethyl-1,3,2-dioxaborolan-2-yl)phenyl]-3,4-dihydro-2H-pyrrol (0.96 g, 2.50 mmol) wird in 20 ml N,N-Dimethylacetamid gelöst. Man gibt nacheinander 3-(4-Bromphenyl)-1,3-oxazolidin-2-on (III-4) (0.73 g, 3.00 mmol), PdCl₂[dppf] (0.05 g, 0.07 mmol) und 3.75 ml Natriumcarbonat-Lösung (2 M) zu und rührt anschließend 16 h bei 90°C. Man lässt auf Raumtemperatur abkühlen, gibt Wasser zu und saugt den ausfallenden Niederschlag ab. Das Rohprodukt wird durch Chromatographie an Kieselgel (Cyclohexan/Aceton 20:1 → 10:1, jeweils v/v) aufgereinigt.

Man erhält 0.75 g (66% d. Th.) an 3-{4'-[5-(2,6-Difluorphenyl)-3,4-dihydro-2H-pyrrol-2-yl]-1,1'-biphenyl-4-yl}-1,3-oxazolidin-2-on.
- HPLC:: Log P (pH 2.3) = 2.13 (Reinheit: 92 %)
Log P (pH 7.5) = 3.28

### Beispiel 5

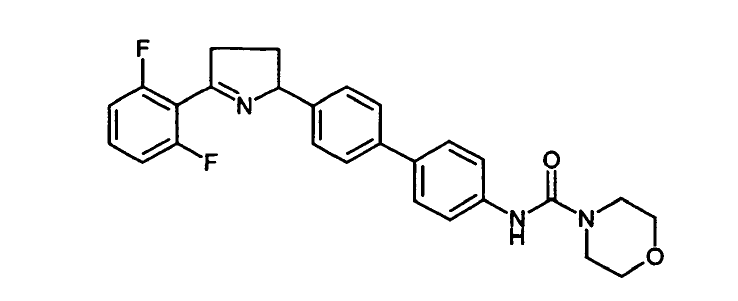

5-(2,6-Difluorphenyl)-2-[4-(4,4,5,5-tetramethyl-1,3,2-dioxaborolan-2-yl)phenyl]-3,4-dihydro-2H-pyrrol (0.96 g, 2.50 mmol) und N-(4-Bromphenyl)-4-morpholin-carboxamid (III-5) (0.86 g, 3.00 mmol) werden in 10 ml 1,2-Dimethoxyethan gelöst. Man gibt nacheinander PdCl₂[dppf] (0.05 g, 0.07 mmol) und 3.75 ml Natriumcarbonat-Lösung (2 M) zu und rührt anschließend 16 h bei 80°C. Man lässt auf Raumtemperatur abkühlen, extrahiert mit Wasser/Essigsäureethylester, trennt die organische Phase ab, trocknet über Magnesiumsulfat, filtriert und engt unter vermindertem Druck ein. Das Rohprodukt wird durch Chromatographie an Kieselgel (Cyclohexan/Essigsäureethylester 2:1 → 1:1, jeweils v/v) aufgereinigt.

Man erhält 0.35 g (30% d. Th.) an N-{4'-[5-(2,6-Difluorphenyl)-3,4-dihydro-2H-pyrrol-2-yl]-1,1'-biphenyl-4-yl}-4-morpholincarboxamid.
- HPLC:: Log P (pH 2.3) = 1.87 (Reinheit: 100 %)
Log P (pH 7.5) = 2.96

Analog den vorstehenden Beispielen 1 bis 5 bzw. gemäß den allgemeinen Angaben zur Herstellung werden die in der folgenden Tabelle angegebenen Verbindungen der Formel (I) erhalten.

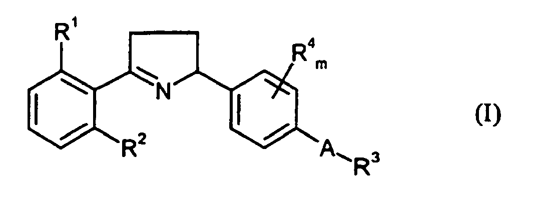

| **Nr.** | **R¹** | **R²** | **R⁴ₘ** | **-A-R³** | **Log P** |
|---|---|---|---|---|---|
| 6 | F | F | - | 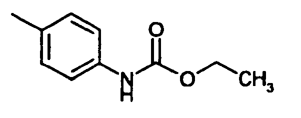 | 2.62^{a)} |
| | | | | | 3.85^{b)} |
| 7 | F | F | - | 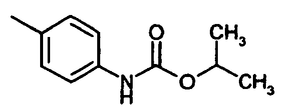 | 3.00^{a)} |
| | | | | | 4.19^{b)} |
| 8 | F | F | - | 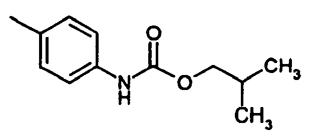 | 3.32^{a)} |
| | | | | | 4.54^{b)} |
| 9 | F | F | - | 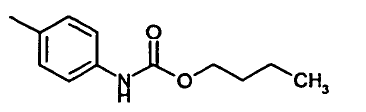 | 3.44^{a)} |
| | | | | | 4.60^{b)} |
| 10 | F | F | - | 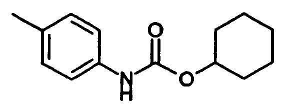 | 3.88^{a)} |
| | | | | | 1.68^{b)} |
| 11 | F | F | - | 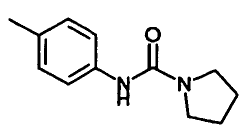 | 2.11^{a)} |
| 12 | F | F | - | 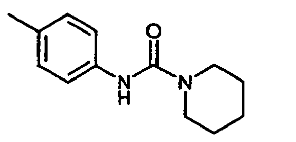 | 2.53^{a)} |
| | | | | | 3.71^{b)} |
| 13 | F | F | - | 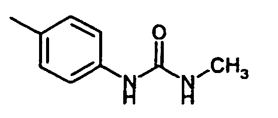 | 1.71^{a)} |
| | | | | | 2.78^{b)} |
| 14 | F | F | - | 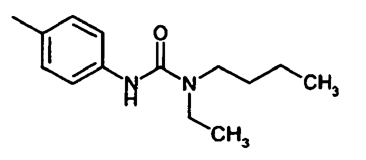 | 3.18^{a)} |
| | | | | | 4.36^{b)} |
| 15 | F | F | - | 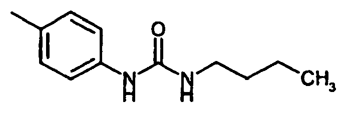 | 2.48^{a)} |
| | | | | | 3.47^{b)} |
| 16 | F | F | - | 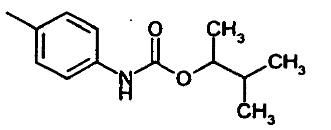 | 3.82^{a)} |
| | | | | | 4.77^{b)} |
| 17 | F | F | - | 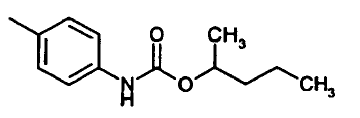 | 4.82^{b)} |
| 18 | F | F | - | 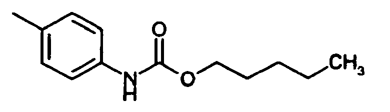 | 3.87^{a)} |
| | | | | | 4.89^{b)} |
| 19 | F | F | - | 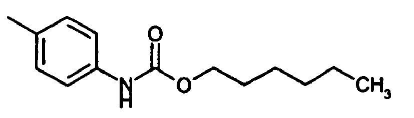 | 4.46^{a)} |
| | | | | | 5.26^{b)} |
| 20 | F | F | - | 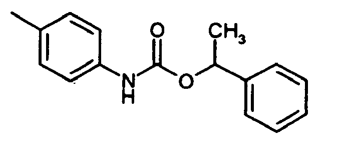 | 3.63^{a)} |
| 21 | F | F | - | 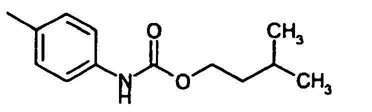 | 3.72^{a)} |
| 22 | F | F | - | 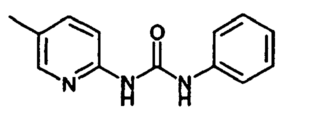 | 2.71^{a)} |
| | | | | | 3.98^{b)} |
| 23 | F | F | - | 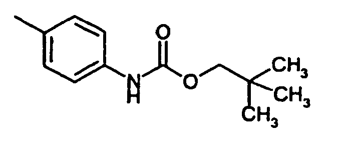 | 3.76^{a)} |
| 24 | F | F | - | 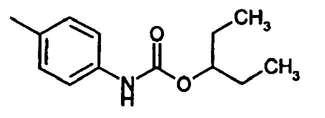 | 3.76^{a)} |
| 25 | F | F | - | 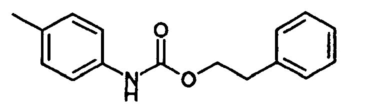 | 3.62^{a)} |
| | | | | | 4.64^{b)} |
| 26 | F | F | - | 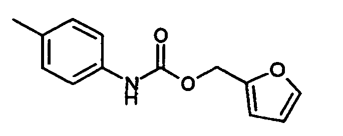 | 3.01^{a)} |
| | | | | | 4.10^{b)} |
| 27 | F | F | - | 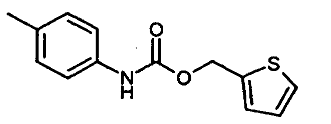 | 3.28^{a)} |
| | | | | | 4.36^{b)} |
| 28 | F | F | - | 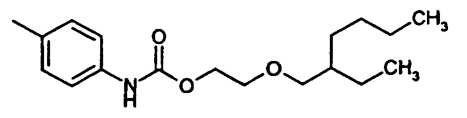 | 5.13^{a)} |
| | | | | | 5.97^{b)} |
| 29 | F | F | - | 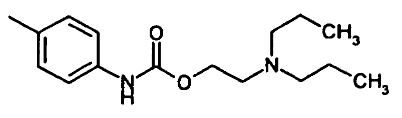 | 1.74^{a)} |
| | | | | | 5.52^{b)} |
| 30 | F | F | - | 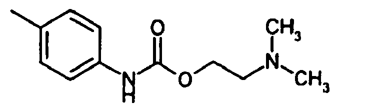 | 1.37^{a)} |
| 31 | F | F | - | 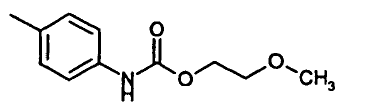 | 2.24^{a)} |
| | | | | | 3.45^{b)} |
| 32 | F | F | - | 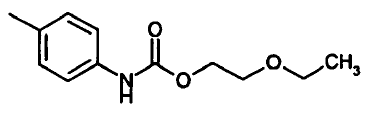 | 2.52^{a)} |
| | | | | | 3.75^{b)} |
| 33 | F | F | - | 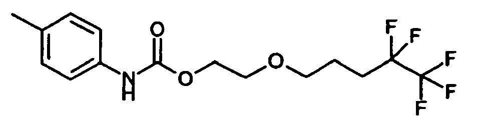 | 3.82^{a)} |
| 34 | F | F | - | 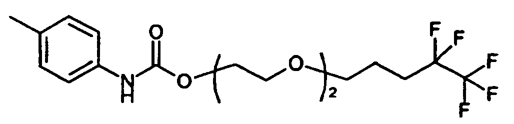 | 3.70^{a)} |
| | | | | | 4.73^{b)} |
| 35 | F | F | - | 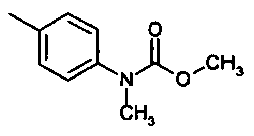 | 2.58^{a)} |
| | | | | | 3.79^{b)} |
| 36 | F | F | - | 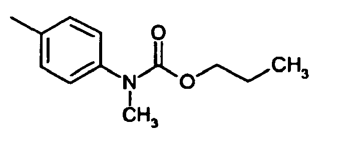 | 3.32^{a)} |
| | | | | | 4.52^{b)} |
| 37 | F | F | - | 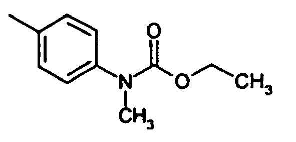 | 2.92^{a)} |
| | | | | | 4.16^{b)} |
| 38 | F | F | - | 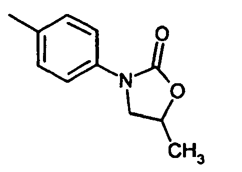 | 2.40^{a)} |
| 39 | F | F | - | 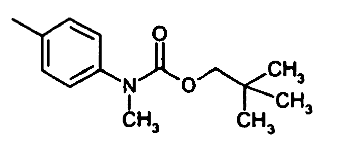 | 4.11^{a)} |
| | | | | | 5.19^{b)} |
| 40 | F | F | - | 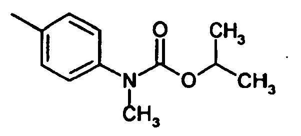 | 3.30^{a)} |
| | | | | | 4.50^{b)} |
| 41 | F | F | - | 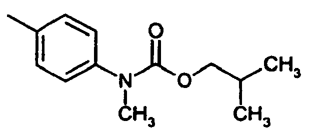 | 3.79^{a)} |
| | | | | | 4.89^{b)} |
| 42 | F | F | - | 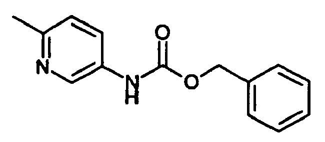 | 2.54^{a)} |
| | | | | | 3.88^{b)} |

### Herstellung von Ausgangsstoffen der Formel (III)

### Beispiel (III-1)

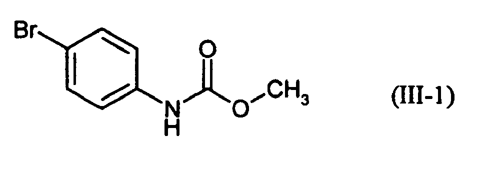

Zu 1 ml Methanol und 20 ml Toluol tropft man bei Raumtemperatur eine Lösung von 4-Bromphenylisocyanat (3.26 g, 16.46 mmol) in 15 ml Toluol zu und rührt für weitere 16 Stunden bei Raumtemperatur nach. Das Reaktionsgemisch wird unter vermindertem Druck eingeengt. Das Rohprodukt wird durch Chromatographie an Kieselgel (Cyclohexan/Essigsäureethylester 2:1, v/v) aufgereinigt.

Man erhält 3.65 g (88% d. Th.) an Methyl-4-bromphenylcarbamat.
- HPLC:: Log P (pH 2.3) = 2.27 (Reinheit: 92 %)

### Beispiel (III-2)

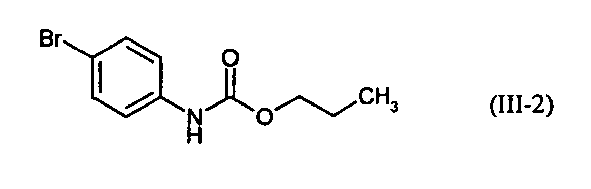

Unter Argonatmosphäre legt man 1.85 ml n-Propanol in 10 ml Toluol vor. Man gibt bei Raumtemperatur 4-Bromphenylisocyanat (3.26 g, 16.46 mmol) portionsweise zu und rührt für weitere 16 Stunden bei Raumtemperatur nach. Das Reaktionsgemisch wird unter vermindertem Druck eingeengt. Das Rohprodukt wird durch Chromatographie an Kieselgel (Cyclohexan/Essigsäureethylester 2:1, v/v) aufgereinigt.

Man erhält 4.13 g (95% d. Th.) an n-Propyl-4-bromphenylcarbamat.
- HPLC:: Log P (pH 2.3) = 3.12 (Reinheit: 98 %)

### Beispiel (III-3)

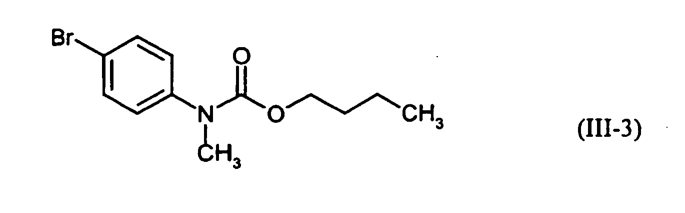

Unter Argonatmosphäre legt man N-(4-Bromphenyl)-N-methylamin (1.50 g, 8.06 mmol) in 15 ml Dichlormethan vor, gibt anschließend N,O-Bis(trimethylsilyl)-acetamid (1.97 g, 9.67 mmol) zu und rührt bei Raumtemperatur 1 Stunde nach. Danach wird auf 0°C abgekühlt und Chlorameisensäurebutylester (1.23 ml, 9.67 mmol) zugetropft. Man lässt auf Raumtemperatur erwärmen und rührt für weitere 16 Stunden bei dieser Temperatur. Man kühlt erneut auf 0°C, quencht mit Kaliumdihydrogenphosphat/Dinatriumhydrogenphosphat-Puffer pH 7, extrahiert mit Dichlormethan, trennt die organische Phase ab, wäscht mit gesättigter Natriumchlorid-Lösung, trocknet über Magnesiumsulfat, filtriert und engt unter vermindertem Druck ein.

Man erhält 1.13 g (98% d. Th.) an n-Butyl-4-bromphenyl(methyl)carbamat.
- HPLC:: Log P (pH 2.3) = 3.88 (Reinheit: 96 %)

### Beispiel (III-4)

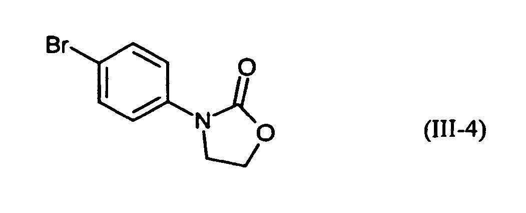

Unter Argonatmosphäre legt man 4-Bromphenylisocyanat (2.00 g, 10.10 mmol) in 15 ml Dimethylsulfoxid vor und gibt anschließend Dioxolan-2-on (0.89 g, 10.10 mmol) und Caesiumfluorid (0.15 g, 1.01 mmol) zu, worauf sich ein weißer Niederschlag bildet. Unter Argon wird 2 Stunden bei 140°C gerührt. Man kühlt ab, gießt das Reaktionsgemisch auf Eiswasser, saugt den entstehenden Niederschlag ab, trocknet und kristallisiert aus Isopropanol um.

Man erhält 1.55 g (61% d. Th.) an 3-(4-Bromphenyl)-1,3-oxazolidin-2-on.
- HPLC:: Log P (pH 2.3) = 2.02 (Reinheit: 96 %)

### Beispiel (III-5)

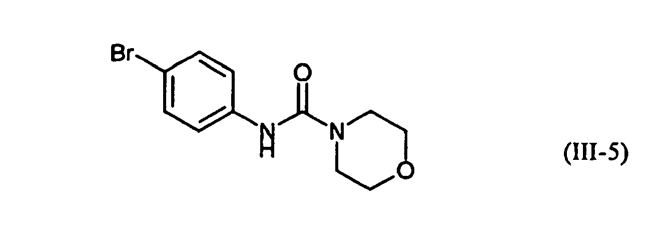

Unter Argonatmosphäre legt man 1.96 ml Morpholin in 15 ml Dioxan vor. Man gibt bei Raumtemperatur 4-Bromphenylisocyanat (2.97 g, 15.00 mmol) portionsweise zu und rührt für weitere 16 Stunden bei Raumtemperatur nach. Anschließend saugt man ab und isoliert das Rohprodukt durch Chromatographie an Kieselgel (Cyclohexan/Essigsäureethylester 2:1, v/v).

Man erhält 3.13 g (66% d. Th.) an N-(4-Bromphenyl)-4-morpholincarboxamid.
- HPLC:: Log P (pH 2.3) = 1.75 (Reinheit: 91 %)

### Herstellung von Ausgangsstoffen der Formel (VII)

### Beispiel (VII-1)

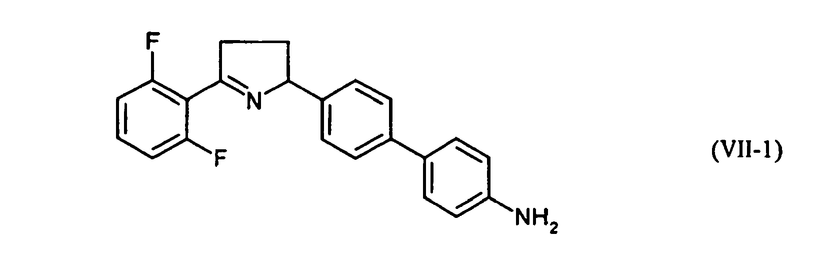

Methyl-4'-[5-(2,6-difluorophenyl)-3,4-dihydro-2H-pyrrol-2-yl]-1,1'-biphenyl-4-yl-carbamat (Beispiel 1) (0.23 g, 0.53 mmol) werden in 2 ml Ethanol gelöst. Nach Zugabe von Kaliumhydroxid (0.03 g, 0.53 mmol) rührt man 16 Stunden unter Rückfluss. Der ausfallende Niederschlag wird abgesaugt und das Rohprodukt durch Chromatographie an Kieselgel (Cyclohexan/Essigsäureethylester 2:1, v/v) aufgereinigt.

Man erhält 0.13 g (69% d. Th.) an 4`-[5-(2,6-Difluorphenyl)-3,4-dihydro-2H-pyrrol-2-yl]-1,1'-biphenyl-4-amin.
- HPLC:: Log P (pH 2.3) = 1.25 (Reinheit: 97 %)

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10% Acetonitril bis 90% Acetonitril. Entsprechende Messergebnisse sind in den Tabellen mit "a)" markiert.

Die Bestimmung erfolgt im neutralen Bereich bei pH 7.5 mit 0,01-molare wässriger Phosphatpuffer-Lösung und Acetonitril als Eluenten; linearer Gradient von 10% Acetonitril bis 90 % Acetonitril. Entsprechende Messergebnisse sind in den Tabellen mit "b)" markiert.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

### Anwendungsbeispiele

### Beispiel A

### Heliothis virescens-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojatriebe (Glycine max) werden durch Tauchen in die Wirkstoffzubereitung der gewünschten Konzentration behandelt und mit *Heliothis virescens*-Raupen besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen bei einer Wirkstoffkonzentration von 100 ppm z.B. die Verbindungen 2 und 6-8 der Herstellungsbeispiele eine gute Wirksamkeit von mindestens 95%. Die Versuchsergebnisse gehen im Detail aus der folgenden Tabelle A hervor.

**Tabelle A**

| pflanzenschädigende Insekten | | |
|---|---|---|
| **Heliothis virescens-Test** | | |
| Wirkstoffe | Wirkstoffkonzentration in ppm | Abtötungsgrad in % nach 7^{d} |
| 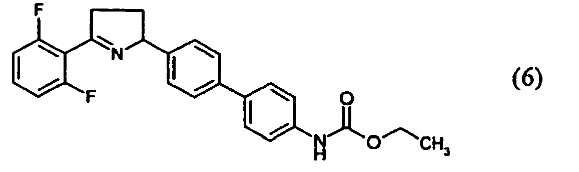 | 100 | 100 |
| 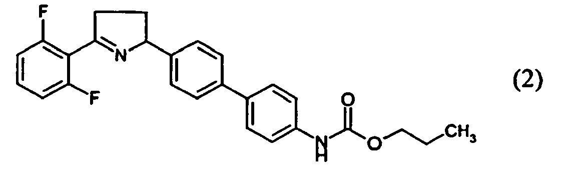 | 100 | 100 |
| 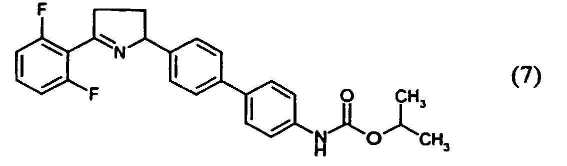 | 100 | 100 |
| 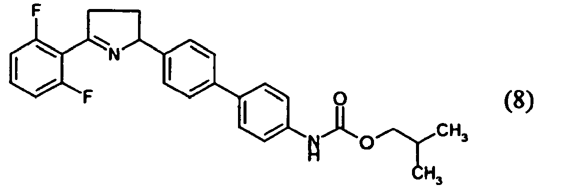 | 100 | 100 |

### Beispiel B

### Phaedon-Larven-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea)* werden durch Tauchen in die Wirkstoffzubereitung der gewünschten Konzentration behandelt und mit Larven des Meerrettichblattkäfers *(Phaedon cochleariae)* besetzt, solange die Blätter noch feucht sind.

Nach der gewünschten Zeit wird die Abtötung in % bestimmt. Dabei bedeutet 100%, dass alle Käferlarven abgetötet wurden; 0% bedeutet, dass keine Käferlarven abgetötet wurden.

Bei diesem Test zeigen bei einer Wirkstoffkonzentration von 100 ppm z.B. die Verbindungen 1, 2, 6, 7, 16, 18-21, 24, 25, 27, 29, 32, 35, 37 der Herstellungsbeispiele eine gute Wirksamkeit von mindestens 85%. Die Versuchsergebnisse gehen im Detail aus der folgenden Tabelle B hervor.

**Tabelle B**

| pflanzenschädigende Insekten | | |
|---|---|---|
| **Phaedon-Larven-Test** | | |
| Wirkstoffe | Wirkstoffkonzentration in ppm | Abtötungsgrad in % nach 7^{d} |
| 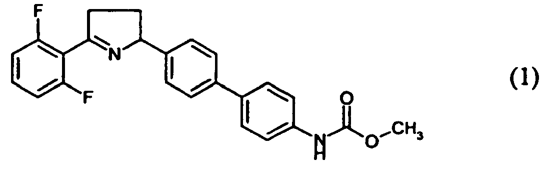 | 100 | 100 |
| 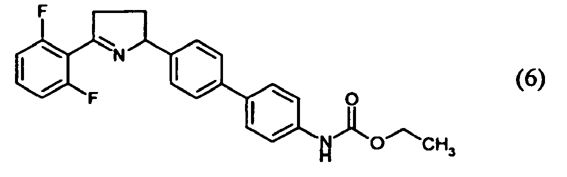 | 100 | 100 |
| 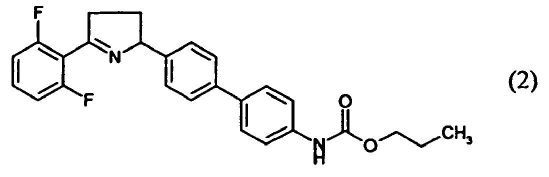 | 100 | 100 |
| 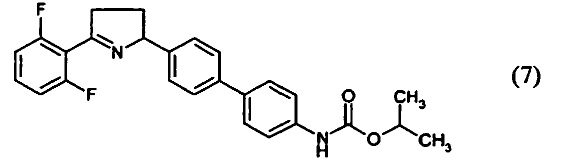 | 100 | 100 |
| 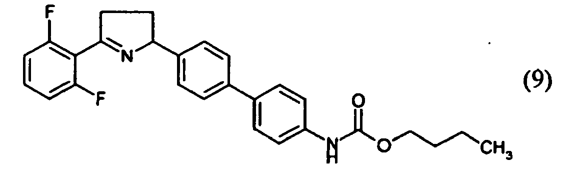 | 100 | 100 |
| 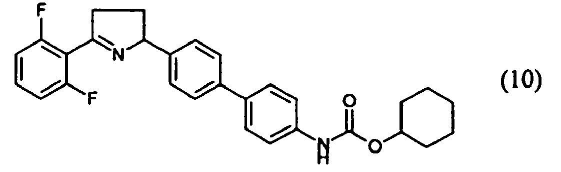 | 100 | 100 |
| 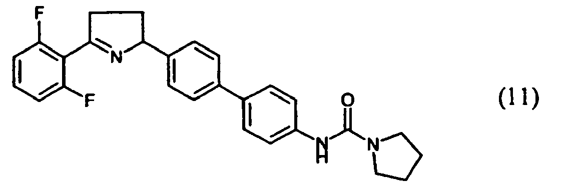 | 100 | 100 |
| 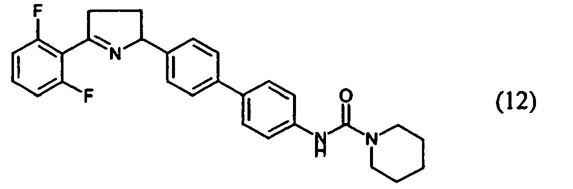 | 100 | 100 |
| 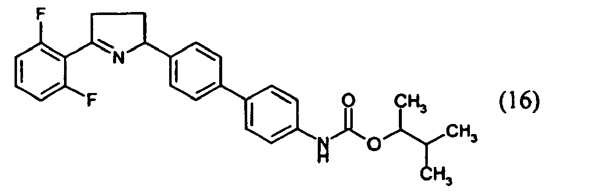 | 100 | 100 |
| 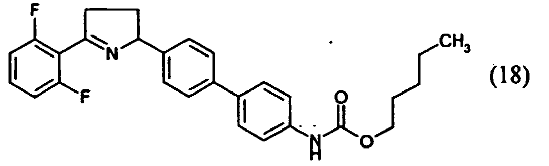 | 100 | 100 |
| 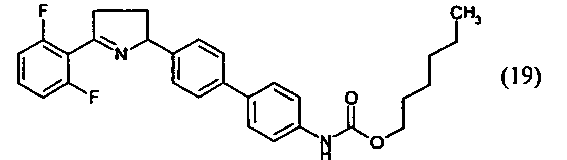 | 100 | 100 |
| 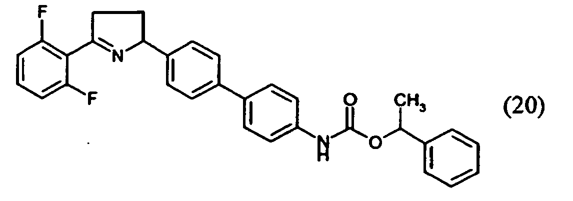 | 100 | 100 |
| 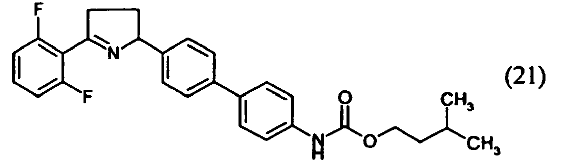 | 100 | 100 |
| 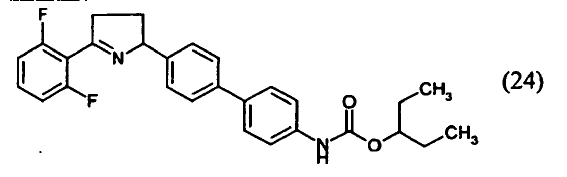 | 100 | 100 |
| 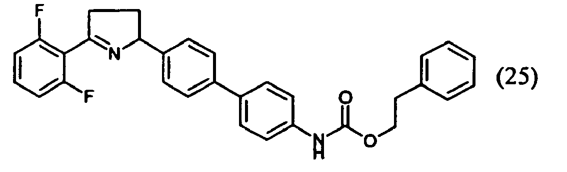 | 100 | 100 |
| 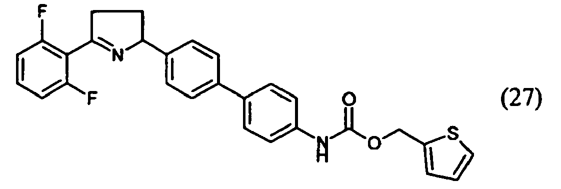 | 100 | 100 |
| 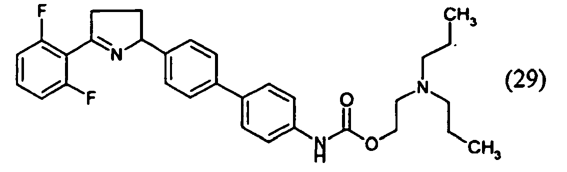 | 100 | 100 |
| 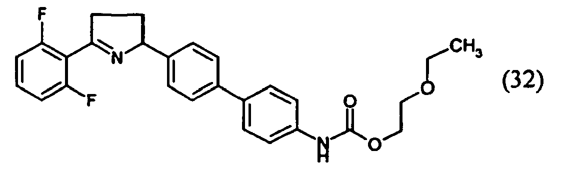 | 100 | 100 |
| 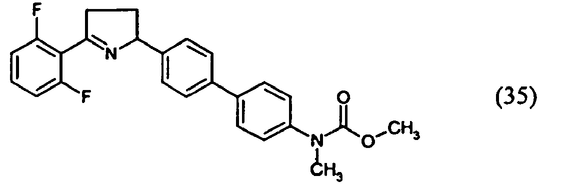 | 100 | 100 |
| 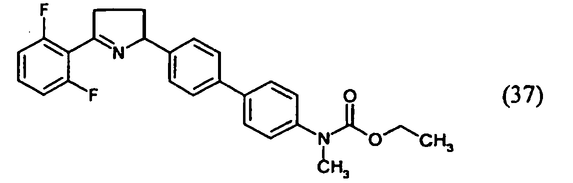 | 100 | 90 |

### Beispiel C

### Plutella-Test, sensibler Stamm

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen der Kohlschabe *(Plutella xylostella,* sensibler Stamm) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurde.

Bei diesem Test zeigen bei einer Wirkstoffkonzentration von 100 ppm z.B. die Verbindungen 2, 6-8 und 23 der Herstellungsbeispiele eine gute Wirksamkeit von mindestens 95%. Die Versuchsergebnisse gehen im Detail aus der folgenden Tabelle C hervor.

**Tabelle C**

| pflanzenschädigende Insekten | | |
|---|---|---|
| **Plutella-Test, sensibler Stamm** | | |
| Wirkstoffe | Wirkstoffkonzentration in ppm | Abtötungsgrad in % nach 7^{d} |
| 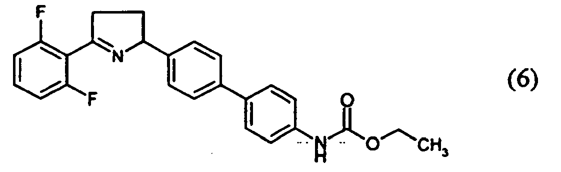 | 100 | 100 |
| 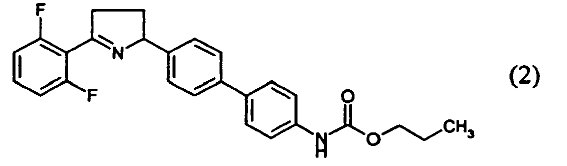 | 100 | 100 |
| 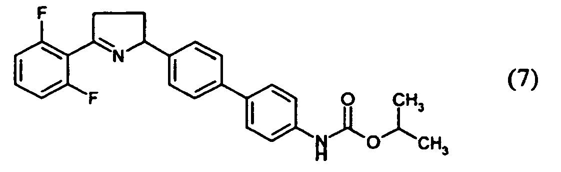 | 100 | 100 |
| 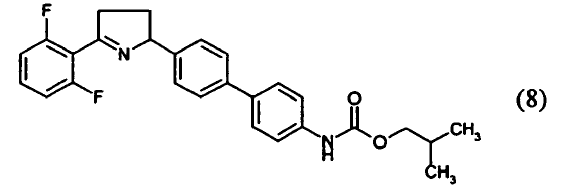 | 100 | 100 |
| 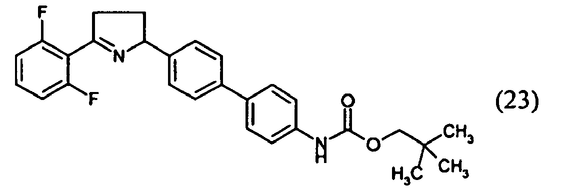 | 100 | 100 |

### Beispiel D

### Plutella-Test, resistenter Stamm

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen der Kohlschabe *(Plutella xylostella,* resistenter Stamm) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurde.

Bei diesem Test zeigen bei einer Wirkstoffkonzentration von 100 ppm z.B. die Verbindungen 2, 6-8 und 23 der Herstellungsbeispiele eine gute Wirksamkeit von mindestens 95%. Die Versuchsergebnisse gehen im Detail aus der folgenden Tabelle D hervor.

**Tabelle D**

| pflanzenschädigende Insekten | | |
|---|---|---|
| **Plutella-Test, resistenter Stamm** | | |
| Wirkstoffe | Wirkstoffkonzentration in ppm | Abtötungsgrad in % nach 7^{d} |
| 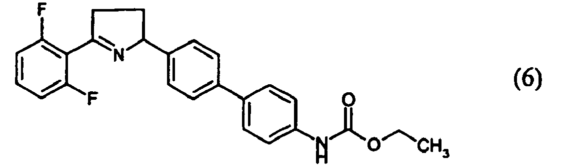 | 100 | 100 |
| 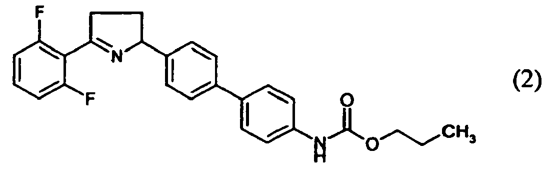 | 100 | 100 |
| 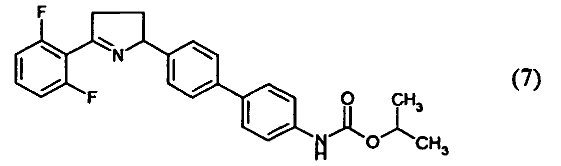 | 100 | 100 |
| 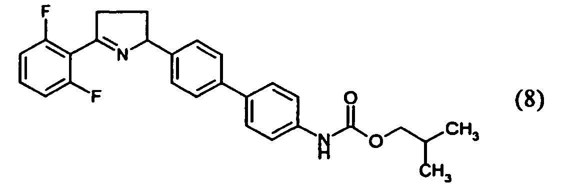 | 100 | 100 |
| 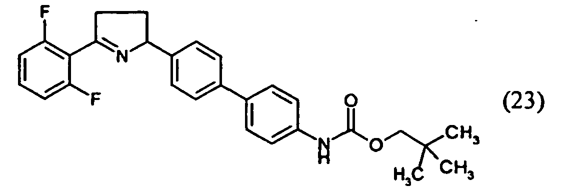 | 100 | 100 |

### Beispiel E

### Spodoptera exigua-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen des Heerwurms *(Spodoptera exigua)* besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen bei einer Wirkstoffkonzentration von 100 ppm z.B. die Verbindungen 2, 6-8 und 23 der Herstellungsbeispiele eine gute Wirksamkeit von mindestens 95%. Die Versuchsergebnisse gehen im Detail aus der folgenden Tabelle E hervor.

**Tabelle E**

| pflanzenschädigende Insekten | | |
|---|---|---|
| **Spodoptera exigua-Test** | | |
| Wirkstoffe | Wirkstoffkonzentration in ppm | Abtötungsgrad in % nach 7^{d} |
| 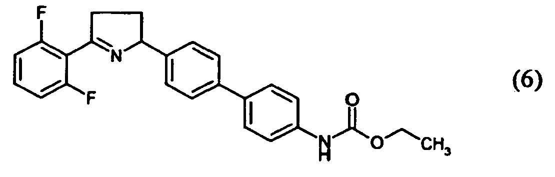 | 100 | 100 |
| 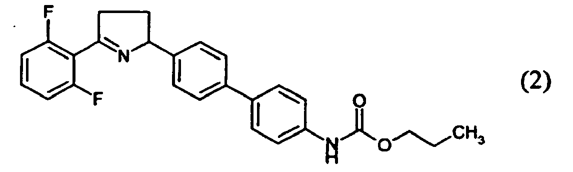 | 100 | 100 |
| 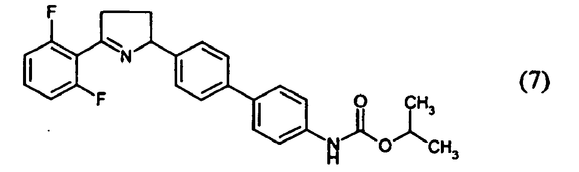 | 100 | 100 |
| 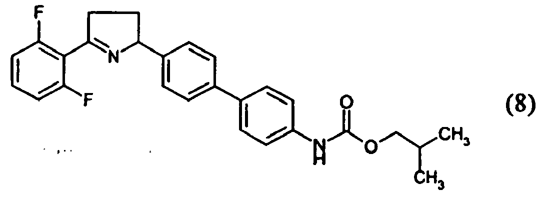 | 100 | 100 |
| 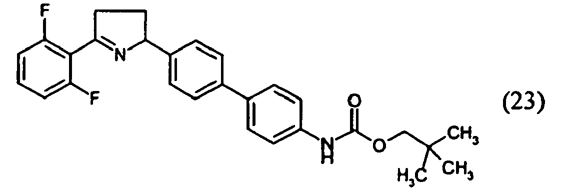 | 100 | 100 |

### Beispiel F

### Spodoptera frugiperda-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen des Heerwurms *(Spodoptera frugiperda)* besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen bei einer Wirkstoffkonzentration von 100 ppm z.B. die Verbindungen 1, 2, 4-7, 9, 10, 12, 14, 17, 18, 20-23, 25-28, 30, 31, 33, 34 und 37 der Herstellungsbeispiele eine gute Wirksamkeit von mindestens 95%. Die Versuchsergebnisse gehen im Detail aus der folgenden Tabelle F hervor.

**Tabelle F**

| pflanzenschädigende Insekten | | |
|---|---|---|
| **Spodoptera frugiperda-Test** | | |
| Wirkstoffe | Wirkstoffkonzentration in ppm | Abtötungsgrad in % nach 7^{d} |
| 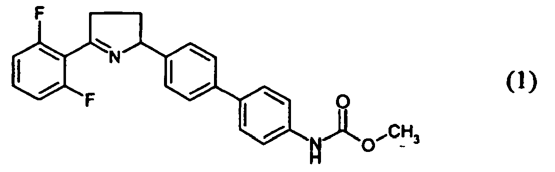 | 100 | 100 |
| 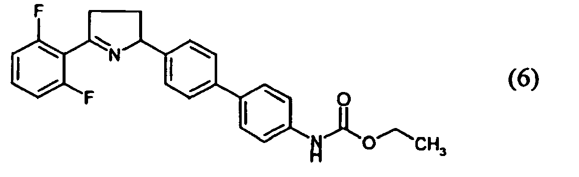 | 100 | 100 |
| 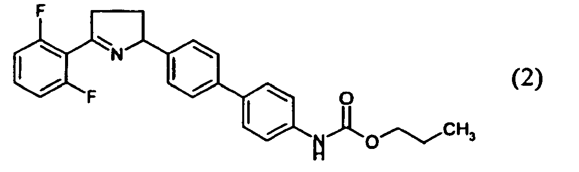 | 100 | 100 |
| 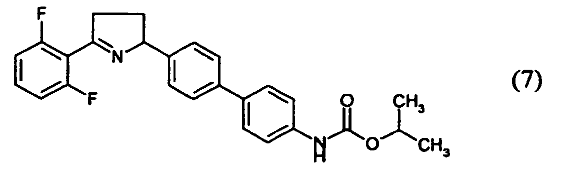 | 100 | 100 |
| 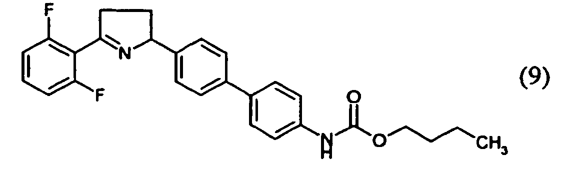 | 100 | 100 |
| 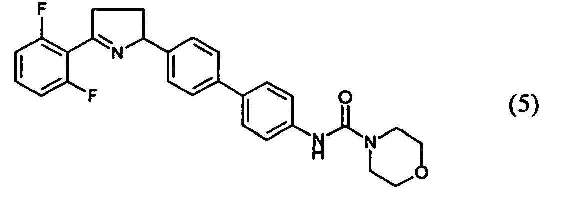 | 100 | 100 |
| 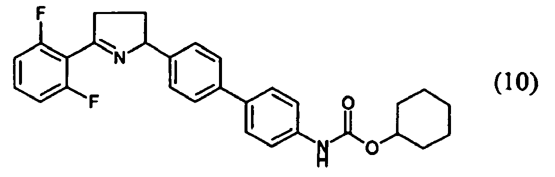 | 100 | 100 |
| 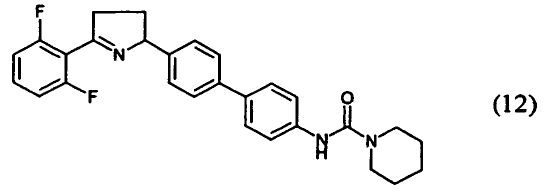 | 100 | 100 |
| 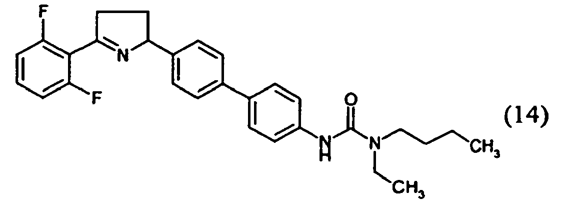 | 100 | 100 |
| 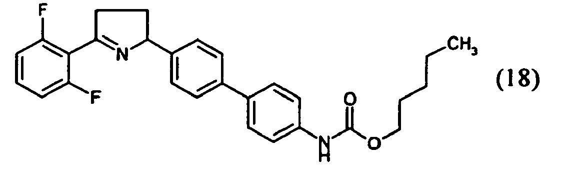 | 100 | 100 |
| 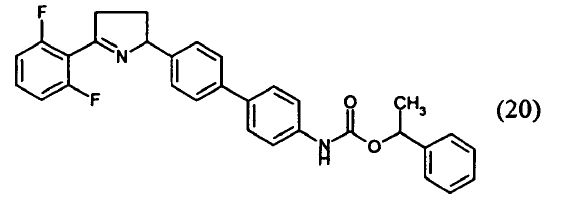 | 100 | 100 |
| 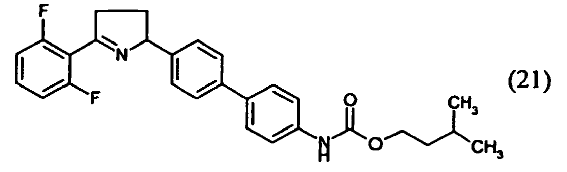 | 100 | 100 |
| 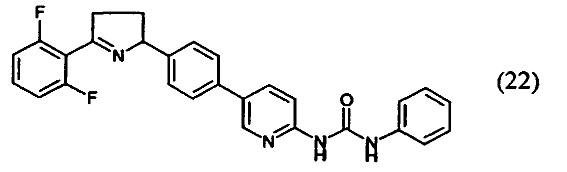 | 100 | 100 |
| 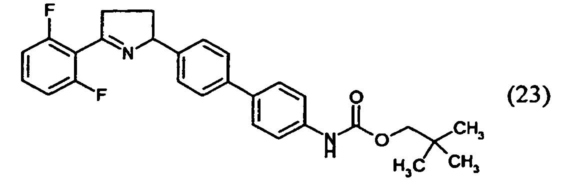 | 100 | 100 |
| 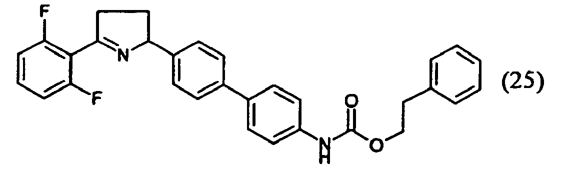 | 100 | 100 |
| 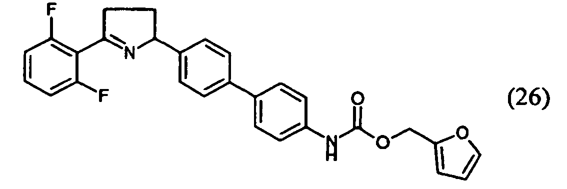 | 100 | 100 |
| 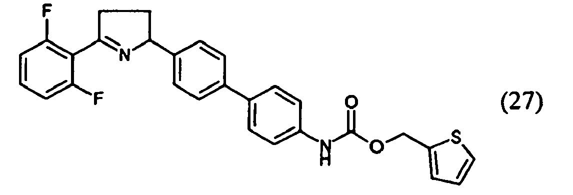 | 100 | 100 |
| 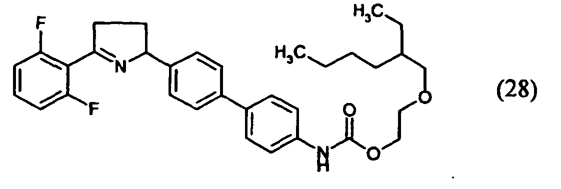 | 100 | 100 |
| 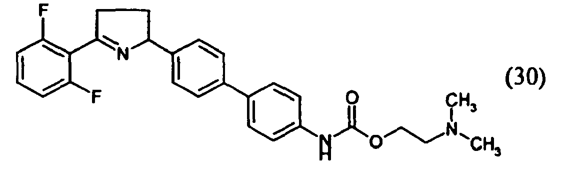 | 100 | 100 |
| 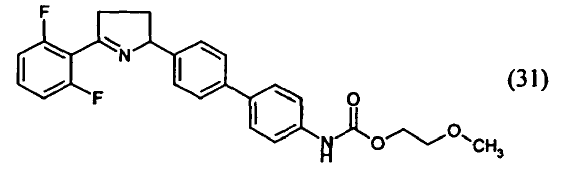 | 100 | 100 |
| 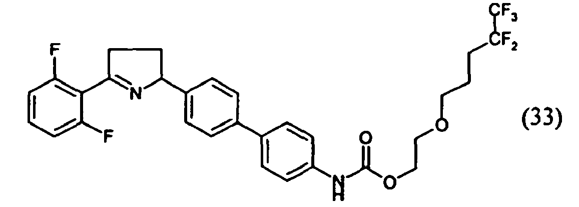 | 100 | 100 |
| 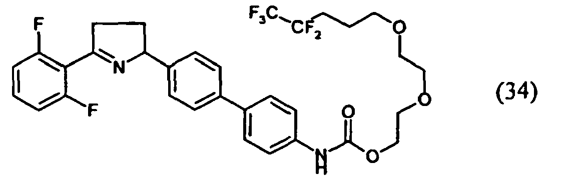 | 100 | 100 |
| 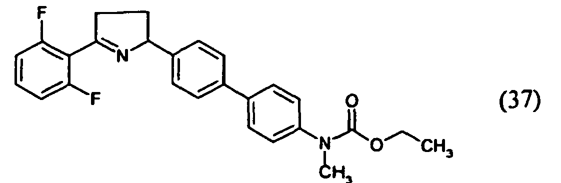 | 100 | 100 |
| 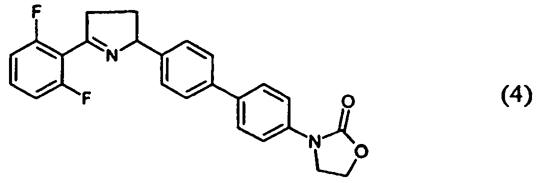 | 100 | 100 |

### Beispiel G

### Tetranychus-Test (OP-resistent/Tauchbehandlung)

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen *(Phaseolus vulgaris),* die stark von allen Stadien der gemeinen Spinnmilbe *(Tetranychus urticae)* befallen sind, werden in eine Wirkstoffzubereitung der gewünschten Konzentration getaucht.

Nach der gewünschten Zeit wird die Wirkung in % bestimmt. Dabei bedeutet 100%, dass alle Spinnmilben abgetötet wurden; 0% bedeutet, dass keine Spinnmilben abgetötet wurden.

Bei diesem Test zeigen bei einer Wirkstoffkonzentration von 100 ppm z.B. die Verbindungen 1, 2, 4, 6, 7, 17, 19, 22-24, 26-30 und 32-35 der Herstellungsbeispiele eine gute Wirksamkeit von mindestens 85%. Die Versuchsergebnisse gehen im Detail aus der folgenden Tabelle G hervor.

**Tabelle G**

| pflanzenschädigende Insekten | | |
|---|---|---|
| **Tetranychus-Test** (OP-resistent/Tauchbehandlung) | | |
| Wirkstoffe | Wirkstoffkonzentration in ppm | Abtötungsgrad in % nach 7^{d} |
| 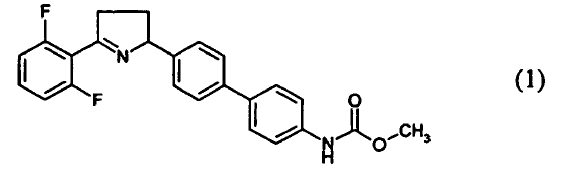 | 100 | 98 |
| 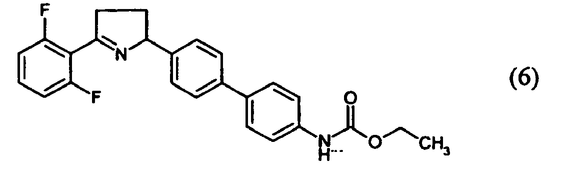 | 100 | 98 |
| 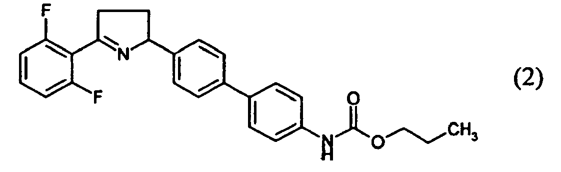 | 100 | 98 |
| 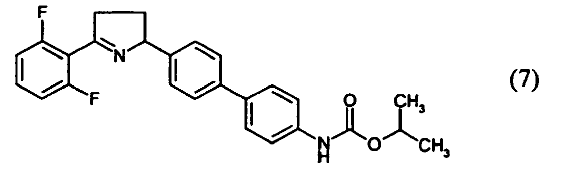 | 100 | 98 |
| 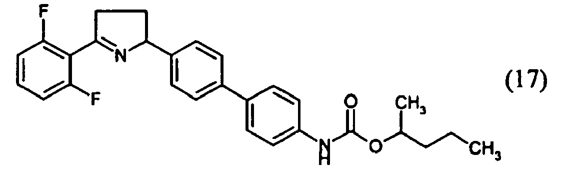 | 100 | 90 |
| 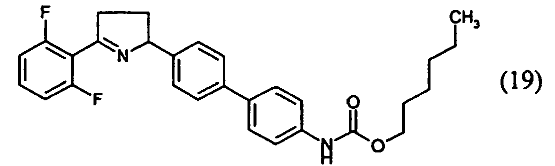 | 100 | 90 |
| 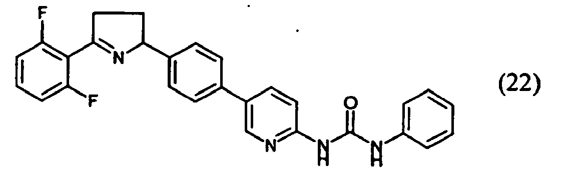 | 100 | 90 |
| 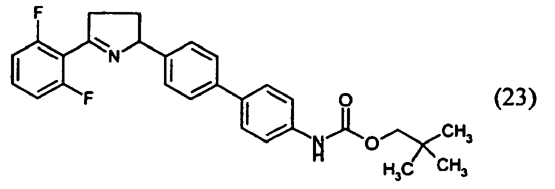 | 100 | 95 |
| 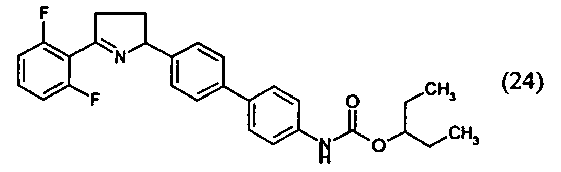 | 100 | 95 |
| 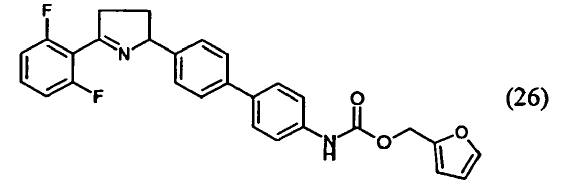 | 100 | 98 |
| 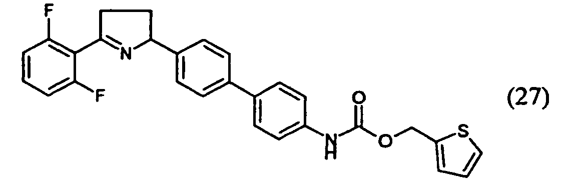 | 100 | 98 |
| 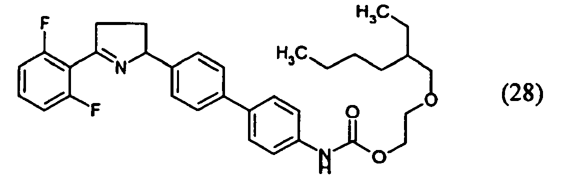 | 100 | 95 |
| 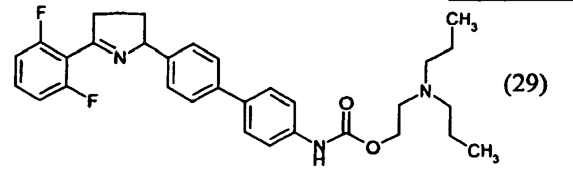 | 100 | 98 |
| 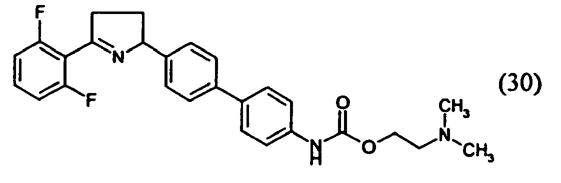 | 100 | 95 |
| 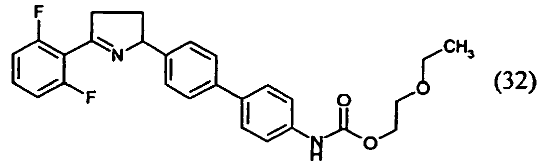 | 100 | 98 |
| 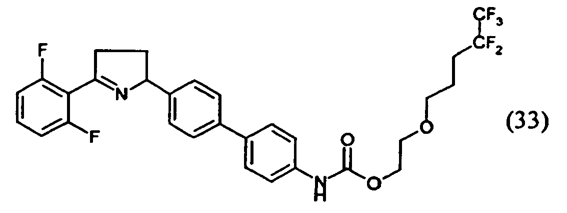 | 100 | 100 |
| 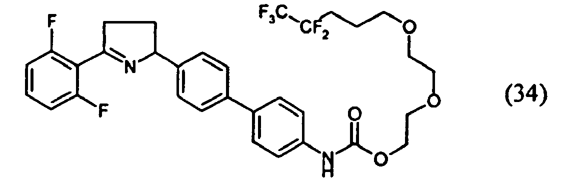 | 100 | 90 |
| 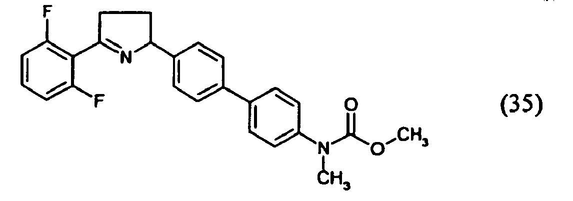 | 100 | 98 |
| 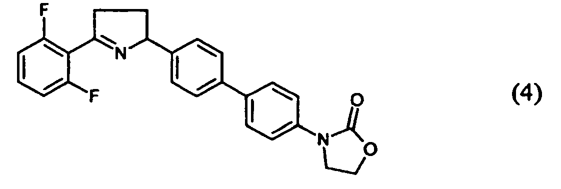 | 100 | 90 |

### Beispiel H

### Diabrotica balteata - Test (Larven im Boden)

### Grenzkonzentrations-Test / Bodeninsekten - Behandlung transgener Pflanzen

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20% Wirkung).

### Beispiel J

### Heliothis virescens - Test (Behandlung transgener Pflanzen)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

### Beispiel K

### Test mit Boophilus microplus resistent/SP-resistenter Parkhurst-Stamm

| | |
|---|---|
| Testtiere: | adulte gesogene Weibchen |
| Lösungsmittel: | Dimethylsulfoxid |

20 mg Wirkstoff werden in 1 ml Dimethylsulfoxid gelöst. Geringere Konzentrationen werden durch Verdünnen in demselben Lösungsmittel hergestellt.

Der Test wir in 5-fach-Bestimmung durchgeführt. 1 µl der Lösungen wird in das Abdomen injiziert, die Tiere werden in Schalen überführt und in einem klimatisierten Raum aufbewahrt. Die Wirkungskontrolle erfolgt nach 7 Tagen auf Ablage fertiler Eier. Eier, deren Fertilität nicht äußerlich sichtbar ist, werden in Glasröhrchen bis zum Larvenschlupf im Klimaschrank aufbewahrt. Eine Wirkung von 100 % bedeutet, dass keine Zecke fertile Eier gelegt hat.

Bei diesem Test zeigen z.B. die folgenden Verbindungen der Herstellungsbeispiele sehr gute Wirksamkeit:

**Tabelle K**

| **Test mit Boophilus microplus resistent/SP-resistenter Parkhurst-Stamm** | | |
|---|---|---|
| Wirkstoffe | Konzentration in µg/Tier | Wirkung/ Abtötung in % |
| 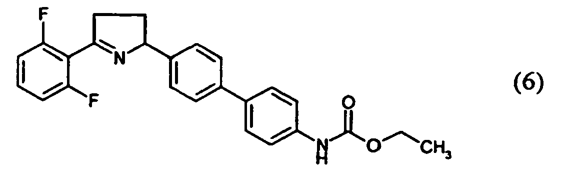 | 100/20 | 20/20 |
| 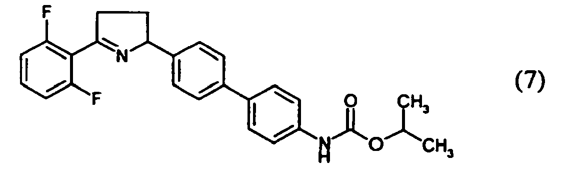 | 100/20 | 40/0 |
| 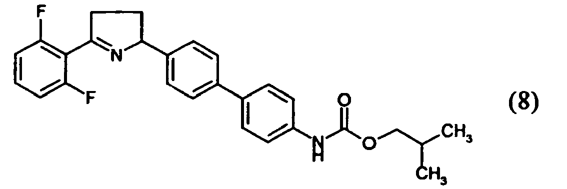 | 100/2014 | 80/40/0 |

### Beispiel L

### Test mit Fliegen (Musca domestica)

| | |
|---|---|
| Testtiere: | adulte Musca domestica, Stamm Reichswald (OP, SP, Carbamat-resistent) |
| Lösungsmittel: | Dimethylsulfoxid |

20 mg Wirkstoff werden in 1 ml Dimethylsulfoxid gelöst. Geringere Konzentrationen werden durch Verdünnen mit destilliertem Wasser hergestellt.

2 ml dieser Wirkstoffzubereitung werden auf Filterpapierschalen (∅ 9.5 cm) pipettiert, die sich in Petrischalen entsprechender Größe befinden. Nach Trocknung der Filterscheiben werden 25 Testtiere in die Petrischalen überführt und abgedeckt.

Nach 1, 3, 5, 24 und 48 Stunden wird die Wirksamkeit der Wirkstoffzubereitung ermittelt. Dabei bedeutet 100 %, dass alle Fliegen abgetötet wurden, 0 % bedeutet, dass keine Fliege abgetötet wurde.

Bei diesem Test zeigt z.B. die folgende Verbindung der Herstellungsbeispiele sehr gute Wirksamkeit:

**Tabelle L**

| **Test mit Fliegen (Musca domestica)** | | |
|---|---|---|
| Wirkstoff | Konzentration in ppm | Wirkung/ Abtötung in % |
| 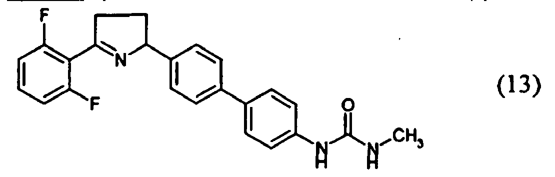 | 100/20 | 30/0 |

## Patentansprüche

1. Δ¹-Pyrroline der Formel (I) 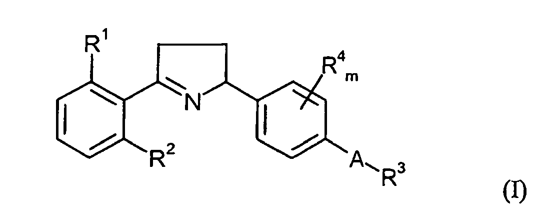 in welcher
R¹ für Halogen oder Methyl steht,
R² für Wasserstoff oder Halogen steht,
R³ für -N(R⁶)-C(=Y)-X-R⁷ steht,
und
a)
A für jeweils gegebenenfalls einfach bis vierfach, gleich oder verschieden durch R⁵ substituiertes Arylen oder 5-gliedriges Heteroarylen mit 1 bis 3 Heteroatomen, welches 0 bis 3 Stickstoffatome, 0 bis 1 Sauerstoffatom und/oder 0 bis 1 Schwefelatom enthält, oder 6-gliedriges Heteroarylen mit 3 Stickstoffatomen oder 6-gliedriges Heteroarylen mit 1 Stickstoffatom und 1 bis 2 weiteren Heteroatomen, wovon 0 bis 2 Sauerstoffatome und/oder 0 bis 2 Schwefelatome sein können, steht, und
Y für O (Sauerstoff) oder S (Schwefel) steht, und
X für O (Sauerstoff), S (Schwefel) oder NR⁸ steht,
oder
b)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für O (Sauerstoff) oder S (Schwefel) steht, und
X für S (Schwefel) oder NR⁸ steht,
oder
c)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für S (Schwefel) steht, und
X für O (Sauerstoff) steht,
oder
d)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für O (Sauerstoff) steht, und
X für O (Sauerstoff) steht,
und
R⁴ und R⁵ unabhängig voneinander für Halogen, Alkyl, Alkoxy, Alkylthio, Halogenalkyl, Halogenalkoxy oder Halogenalkylthio stehen,
m für 0, 1, 2, 3 oder 4 steht,
R⁶ für Wasserstoff oder Alkyl steht,
R⁷ und R⁸ unabhängig voneinander für Wasserstoff, für jeweils gegebenenfalls einfach oder mehrfach, gleich oder verschieden durch Halogen, Alkylcarbonyl, Alkylcarbonyloxy, Alkylamino, Dialkylamino, Alkoxy, Alkylthio, Alkoxyalkoxy, Halogenalkoxy, Halogenalkylthio und/oder Halogenalkoxyalkoxy substituiertes Alkyl oder Alkenyl;
für jeweils gegebenenfalls einfach oder mehrfach, gleich oder verschieden durch Halogen, Alkyl, Halogenalkyl, Alkoxy, Halogenalkoxy, Alkylthio, Halogenalkylthio, Alkylcarbonyl und/oder Alkoxycarbonyl substituiertes Cycloalkyl, Cycloalkylalkyl, Aryl, Arylalkyl, gesättigtes oder ungesättigtes, 5- bis 10-gliedriges Heterocyclyl oder Heterocyclylalkyl stehen,
R⁶ und R⁷ außerdem gemeinsam für gegebenenfalls einfach oder mehrfach durch Alkyl substituiertes Alkylen stehen, oder
R⁷ und R⁸ außerdem gemeinsam mit dem Stickstoffatom, an das sie gebunden sind, für einen gesättigten oder ungesättigten 5- bis 10-gliedrigen Heterocyclus stehen, der gegebenenfalls eine weitere Heteroatomgruppierung aus der Reihe -O-, -S- oder -NR⁹- enthalten kann und der gegebenenfalls einfach oder mehrfach, gleich oder verschieden durch Halogen, Alkyl, Alkoxy, Alkylthio, Halogenalkyl, Halogenalkoxy und/oder Halogenalkylthio substituiert sein kann, und
R⁹ für Wasserstoff, Alkyl oder Alkenyl steht.

2. Δ¹-Pyrroline der Formel (I) gemäß Anspruch 1, in welcher
R¹ für Fluor, Chlor, Brom oder Methyl steht,
R² für Wasserstoff, Fluor, Chlor oder Brom steht,
R³ für -N(R⁶)-C(=Y)-X-R⁷ steht,
und
a)
A für jeweils gegebenenfalls einfach bis dreifach, gleich oder verschieden durch R⁵ substituiertes Arylen (insbesondere Phenylen) oder 5-gliedriges Heteroarylen mit 1 bis 3 Heteroatomen, welches 0 bis 3 Stickstoffatome, 0 bis 1 Sauerstoffatom und/oder 0 bis 1 Schwefelatom enthält (insbesondere aus der Reihe Pyrrolylen, Furylen, Thienylen, Pyrazylen, Imidazylen, Triazylen, Thiazylen oder Oxazylen), oder 6-gliedriges Heteroarylen mit 3 Stickstoffatomen (insbesondere Triazinylen) oder 6-gliedriges Heteroarylen mit 1 Stickstoffatom und 1 bis 2 weiteren Heteroatomen, wovon 0 bis 2 Sauerstoffatome und/oder 0 bis 2 Schwefelatome sein können (insbesondere aus der Reihe Oxazinylen oder Thiazinylen), steht, und
Y für O (Sauerstoff) oder S (Schwefel) steht, und
X für O (Sauerstoff), S (Schwefel) oder NR⁸ steht,
oder
b)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für O (Sauerstoff) oder S (Schwefel) steht, und
X für S (Schwefel) oder NR⁸ steht,
oder
c)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für S (Schwefel) steht, und
X für O (Sauerstoff) steht,
oder
d)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für O (Sauerstoff) steht, und
X für O (Sauerstoff) steht,
und
R⁴ und R⁵ unabhängig voneinander für Fluor, Chlor, Brom, C₁-C₆-Alkyl, C₁-C₆-Alkoxy, C₁-C₆-Alkylthio, C₁-C₆-Halogenalkyl, C₁-C₆-Halogenalkoxy oder C₁-C₆-Halogenakylthio stehen,
m für 0, 1, 2 oder 3 steht,
R⁶ für Wasserstoff oder C₁-C₆-Alkyl steht,
R⁷ und R⁸ unabhängig voneinander für Wasserstoff, für jeweils gegebenenfalls einfach oder mehrfach, gleich oder verschieden durch Fluor, Chlor, Brom, C₁-C₆-Allcylcarbonyl, C₁-C₆-Allcylcarbonyloxy, C₁-C₆-Alkylamino, Di-(C₁-C₆-alkyl)amino, C₁-C₁₀-Alkoxy, C₁-C₁₀-Alkylthio, C₁-C₁-Alkoxy-C₁-C₁₀-alkoxy, C₁-C₁₀-Halogenalkoxy, C₁-C₁₀-Halogenalkylthio und/oder C₁-C₁₀-Halogenalkoxy-C₁C₁₀-alkoxy substituiertes C₁-C₂₀-Alkyl oder C₂-C₂₀-Alkenyl ;
für jeweils gegebenenfalls einfach bis vierfach, gleich oder verschieden durch Fluor, Chlor, Brom, C₁-C₆-Allcyl, C₁-C₆-Alkoxy, C₁-C₆-Alkylthio, C₁-C₆-Halogenalkyl, C₁-C₆-Halogenalkoxy, C₁-C₆-Halogenalkylthio, C₁-C₆-Allcylcarbonyl und/oder C₁-C₆-Alkoxycarbonyl substituiertes C₃-C₁₂-Cycloalkyl, C₃-C₇-Cycloikyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) stehen,
R⁶ und R⁷ außerdem gemeinsam für gegebenenfalls einfach bis vierfach durch C₁-C₄-Alkyl substituiertes C₂-C₄-Alkylen stehen, oder
R⁷ und R⁸ außerdem gemeinsam mit dem Stickstoffatom, an das sie gebunden sind, für einen gesättigten oder ungesättigten 5- bis 10-gliedrigen Heterocyclus stehen, der gegebenenfalls eine weitere Heteroatomgruppierung aus der Reihe -O-, -S- oder -NR⁹- enthalten kann und der gegebenenfalls einfach bis vierfach, gleich oder verschieden durch Fluor, Chlor, Brom, C₁-C₆-Alkyl, C₁-C₆-Alkoxy, C₁-C₆-Alkylthio, C₁-C₆-Halogenalkyl, C₁-C₆-Halogenalkoxy und/oder C₁-C₆-Halogenalkylthio substituiert sein kann, und
R⁹ für Wasserstoff, C₁-C₆-Alkyl oder C₂-C₆-Alkenyl steht.

3. Δ¹-Pyrroline der Formel (I) gemäß Anspruch 1, in welcher
R¹ für Fluor, Chlor oder Methyl steht,
R² für Wasserstoff, Fluor oder Chlor steht,
R³ für -N(R⁶)-C(=Y)-X-R⁷ steht,
und
a)
A für jeweils gegebenenfalls einfach oder zweifach, gleich oder verschieden durch R⁵ substituiertes Phenylen, Pyrrolylen, Furylen, Thienylen, Pyrazylen, Imidazylen, Triazylen, Thiazylen oder Oxazylen steht, und
Y für O (Sauerstoff) oder S (Schwefel) steht, und
X für O (Sauerstoff), S (Schwefel) oder NR⁸ steht,
oder
b)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für O (Sauerstoff) oder S (Schwefel) steht, und
X für S (Schwefel) oder NR⁸ steht,
oder
c)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für S (Schwefel) steht, und
X für O (Sauerstoff) steht,
oder
d)
A für jeweils gegebenenfalls einfach oder zweifach, gleich oder verschieden durch R⁵ substituiertes Pyridinylen, Pyrimidinylen, Pyrazinylen oder Pyridazinylen steht, und
Y für O (Sauerstoff) steht, und
X für O (Sauerstoff steht,
und
R⁴ und R⁵ unabhängig voneinander für Fluor, Chlor, C₁-C₄-Alkyl, C₁-C₄-Alkoxy, C₁-C₄-Alkylthio ; C₁-C₄-Halogenalkyl, C₁-C₄-Halogenalkoxy oder C₁-C₄-Halogenalkylthio mit jeweils 1 bis 9 Fluor-, Chlor- und/oder Bromatomen stehen,
m für 0, 1 oder 2 steht,
R⁶ für Wasserstoff oder C₁-C₄-Alkyl steht,
R⁷ und R⁸ unabhängig voneinander für Wasserstoff, für jeweils gegebenenfalls einfach oder mehrfach, gleich oder verschieden durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₁₀-Alkoxy, C₁-C₁₀-Alkylthio, C₁-C₁₀-Alkoxy-C₁-C₆-alkoxy, C₁-C₁₀-Halogenalkoxy, C₁-C₁₀-Halogenalkylthio und/oder C₁-C₁₀-Halogenalkoxy-C₁-C₆-alkoxy mit jeweils 1 bis 21 Fluor-, Chlor- und/oder Bromatomen substituiertes C₁-C₁₆-Alkyl oder C₂-C₁₆-Alkenyl;
für jeweils gegebenenfalls einfach bis dreifach, gleich oder verschieden durch Fluor, Chlor, Brom, C₁-C₄-Akyl, C₁-C₄-Alkoxy, C₁-C₄-Alkylthio, C₁-C₄-Halogenalkyl, C₁-C₄-Halogenalkoxy, C₁-C₄-Halogenalkylthio
mit jeweils 1 bis 9 Fluor-, Chlor- und/oder Bromatomen, C₁-C₄-Alkylcarbonyl und/oder C₁-C₄-Alkoxycarbonyl substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁶ und R⁷ außerdem gemeinsam für gegebenenfalls einfach bis dreifach durch C₁-C₄-Alkyl substituiertes C₂-C₃-Alkylen stehen, oder
R⁷ und R⁸ außerdem gemeinsam mit dem Stickstoffatom, an das sie gebunden sind, für einen gesättigten oder ungesättigten 5- bis 7-gliedrigen Heterocyclus stehen, der gegebenenfalls eine weitere Heteroatomgruppierung aus der Reihe -O-, -S- oder -NR⁹- enthalten kann (insbesondere aus der Reihe Piperidino, Morpholino, Thiomorpholino, Piperazino, Pyrrolidino, Oxazolidino, Thiazolidino, 4H-1-Oxazinyl, 4H-1-Thiazinyl) und der gegebenenfalls einfach bis vierfach, gleich oder verschieden durch Fluor, Chlor, C₁-C₄-Alkyl, C₁-C₄-Alkoxy, C₁-C₄-Alkylthio, C₁-C₄-Halogenalkyl, C₁-C₄-Halogenalkoxy und/oder C₁-C₄-Halogenalkylthio mit jeweils 1 bis 9 Fluor-, Chlor- und/oder Bromatomen substituiert sein kann, und
R⁹ für Wasserstoff, C₁-C₄-Alkyl oder C₂-C₄-Alkenyl steht.

4. Δ¹-Pyrroline der Formel (1) gemäß Anspruch 1, in welcher
R¹ für Fluor oder Chlor steht,
R² für Wasserstoff oder Fluor steht,
R³ für -N(R⁶)-C(=Y)-X-R⁷ steht,
und
a)
A für jeweils gegebenenfalls einfach durch R⁵ substituiertes 1,2-Phenylen, 1,4-Phenylen, 2,5-Pyrrolylen, 2,5-Furylen, 2,4-Furylen, 2,5-Thienylen, 2,4-Thienylen, 2,5-Thiazylen, 2,4-Thiazylen, 2,5-Oxazylen oder 2,4-Oxazylen steht, und
Y für O (Sauerstoff) oder S (Schwefel) steht, und
X für O (Sauerstoff, S (Schwefel) oder NR⁸ steht,
oder
b)
A für jeweils gegebenenfalls einfach durch R⁵ substituiertes 2,5-Pyridinylen, 2,5-Pyrimidinylen, 2,5-Pyrazinylen oder 3,6-Pyridazinylen steht, und
Y für O (Sauerstoff) oder S (Schwefel) steht, und
X für S (Schwefel) oder NR⁸ steht,
oder
c)
A für jeweils gegebenenfalls einfach durch R⁵ substituiertes 2,5-Pyridinylen, 2,5-Pyrimidinylen, 2,5-Pyrazinylen oder 3,6-Pyridazinylen steht, und
Y für S (Schwefel) steht, und
X für O (Sauerstoff) steht,
oder
d)
A für jeweils gegebenenfalls einfach durch R⁵ substituiertes 2,5-Pyridinylen, 2,5-Pyrimidinylen, 2,5-Pyrazinylen oder 3,6-Pyridazinylen steht, und
Y für O (Sauerstoff) steht, und
X für O (Sauerstoff) steht,
und
R⁴ und R⁵ unabhängig voneinander für Fluor, Chlor, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy, Methylthio, Ethylthio, n-Propylthio, i-Propylthio, n-Butylthio, i-Butylthio, s-Butylthio, t-Butylthio, Trifluormethyl, Trifluorethyl, Trifluormethoxy, Trifluorethoxy, Trifluormethylthio oder Trifluorethylthio stehen,
m für 0 oder 1 steht,
R⁶ für Wasserstoff, Methyl, Ethyl, n-Propyl, i-Propyl, n-Butyl, i-Butyl, s-Butyl oder t-Butyl steht,
R⁷ und R⁸ unabhängig voneinander für Wasserstoff, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-akyl)amino, C₁-C₁₀-Alkoxy, C₁-C₈-Alkoxy-C₁-C₆-alkoxy, C₁-C₁₀-Alkylthio, C₁-C₁₀-Halogenalkoxy, C₁-C₁₀-Halogenalkylthio mit jeweils 1 bis 21 Fluor-, Chlor- und/oder Bromatomen, C₁-C₈-Halogenalkoxy-C₁-C₆-alkoxy mit 1 bis 17 Fluor-, Chlor- und/oder Bromatomen substituiertes C₁-C₁₀-Alkyl oder C₂-C₁₀-Alkenyl (insbesondere Methyl, Ethyl, n-Propyl, 1-Propyl, n-Butyl, i-Butyl, s-Butyl, t-Butyl, die isomeren Pentyle, die isomeren Hexyle);
für jeweils gegebenenfalls einfach bis dreifach, gleich oder verschieden durch Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio mit jeweils 1 bis 9 Fluor-, Chlor- und/oder Bromatomen, C₁-C₄-Alkylcarbonyl und/oder C₁-C₄-Alkoxycarbonyl substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁶ und R⁷ außerdem gemeinsam für jeweils gegebenenfalls einfach oder zweifach, gleich oder verschieden durch Methyl, Ethyl, n-Propyl oder i-Propyl substituiertes Methylen oder Ethylen stehen, oder
R⁷ und R⁸ außerdem gemeinsam mit dem Stickstoffatom, an das sie gebunden sind, für einen 5- bis 6-gliedrigen Heterocyclus aus der Reihe Piperidino, Morpholino, Thiomorpholino, Piperazino, Pyrrolidino, Oxazolidino, Thiazolidino, 4H-1-Oxazinyl, 4H-1-Thiazinyl stehen, der gegebenenfalls einfach bis vierfach, gleich oder verschieden durch Fluor, Chlor, Brom, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy, Methylthio, Ethylthio, n-Propylthio, i-Propylthio, n-Butylthio, i-Butylthio, s-Butylthio, t-Butylthio, C₁-C₄-Halogenalkyl, C₁-C₄-Halogenalkoxy, C₁-C₄-Halogenalkylthio mit jeweils 1 bis 9 Fluor-, Chlor- und/oder Bromatomen substituiert sein kann, wobei der Rest Piperazino am zweiten Stickstoffatom durch R⁹ substituiert ist, und
R⁹ für Wasserstoff, Methyl, Ethyl, n-Propyl, i-Propyl, n-Butyl, i-Butyl, s-Butyl, t-Butyl, Vinyl oder Allyl steht.

5. Δ¹-Pyrroline der Formeln (I-1) und (1-2) 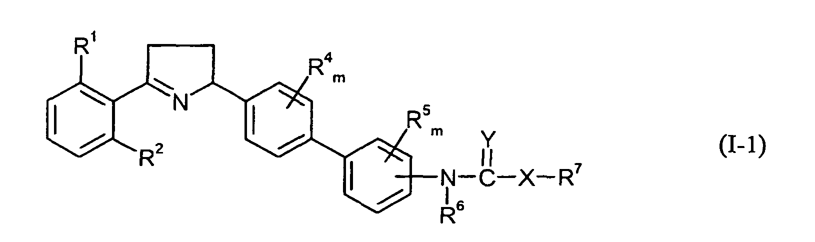 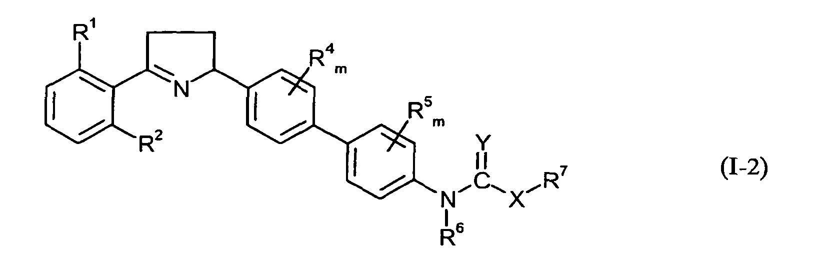 gemäß Anspruch 1, in welchen jeweils
a)
Y für O (Sauerstoff) oder S (Schwefel) steht, und
X für O (Sauerstoff), S (Schwefel) oder NR⁸ steht,
und R¹, R², R⁴, R⁵, m, R⁶, R⁷, R⁸ die in Anspruch 1 angegebenen Bedeutungen haben.

6. Δ¹-Pyrroline der Formeln (I-3) bis (I-8) 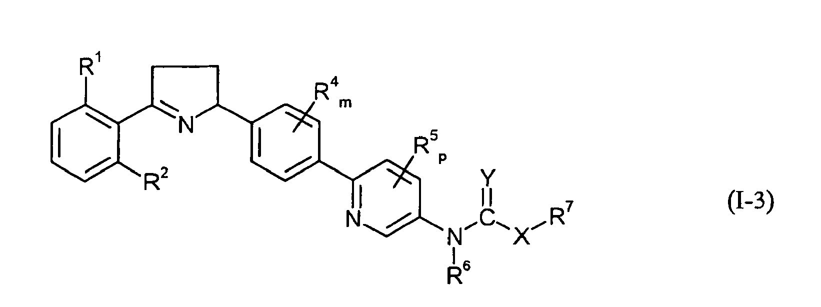 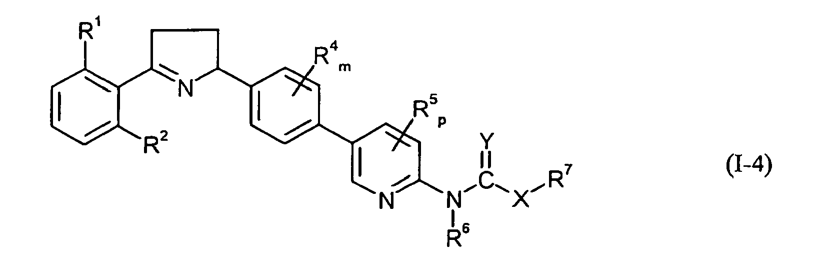 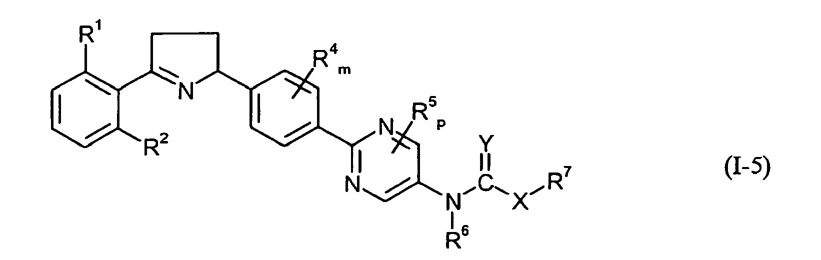 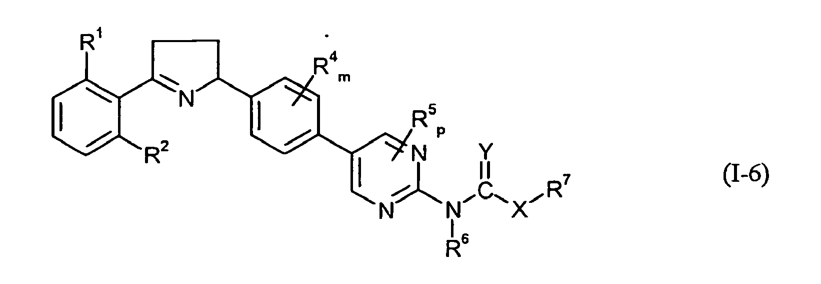 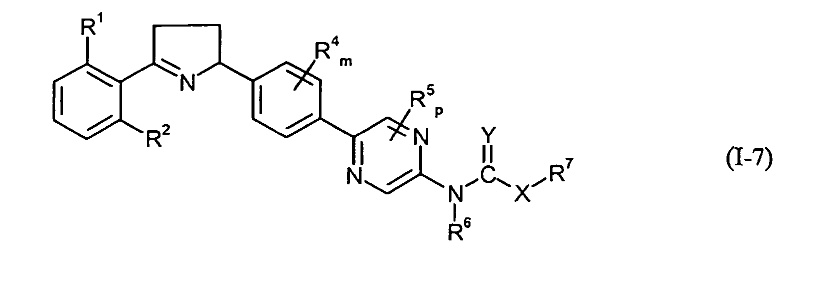 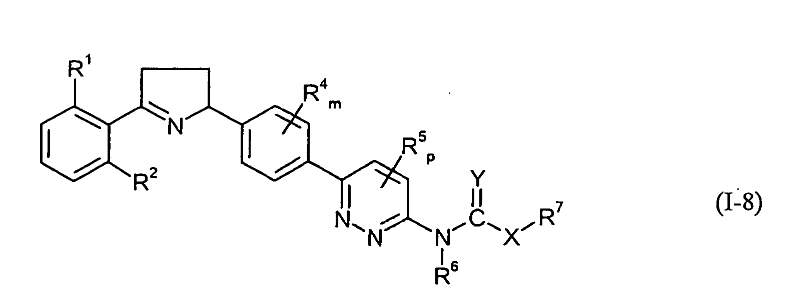 gemäß Anspruch 1, in welchen jeweils
p für 0, 1 oder 2 steht,
b)
Y für O (Sauerstoff) oder S (Schwefel) steht, und
X für S (Schwefel) oder NR⁸ steht,
oder
c)
Y für S (Schwefel) steht, und
X für O (Sauerstoff) steht,
oder
d)
Y für O (Sauerstoff) steht, und
X für O (Sauerstoff) steht,
und R¹, R², R⁴, R⁵, m, R⁶, R⁷ und R⁸ die in Anspruch 1 angegebenen Bedeutungen haben.

7. Δ¹-Pyrroline der Formel (I) gemäß Anspruch 1, in welcher A für Phenylen, bevorzugt 1,4-Phenylen steht.

8. Δ¹-Pyrroline der Formel (I) gemäß Anspruch 1, in welcher A für Pyridinylen, Pyrimidinylen, Pyrazinylen oder Pyridazinylen, bevorzugt 2,5-Pyridinylen, 2,5-Pyrimidinylen, 2,5-Pyrazinylen oder 3,6-Pyridazinylen steht.

9. Δ¹-Pyrroline der Formel (I) gemäß Anspruch 1, in welcher Y für O (Sauerstoff) steht.

10. Δ¹-Pyrroline der Formel (I) gemäß Anspruch 1, in welcher X für O (Sauerstoff) oder NR⁸, bevorzugt für O (Sauerstoff) steht.

11. Δ¹-Pyrroline der Formel (I) gemäß Anspruch 1, in welcher Y und X jeweils für O (Sauerstoff) stehen.

12. Δ¹-Pyrroline der Formel (I) gemäß Anspruch 1, in welcher Y für O (Sauerstoff) und X für NR⁸ stehen.

13. Δ¹-Pyrroline der Formel (I) gemäß Anspruch 1, in welcher A für Phenylen, bevorzugt 1,4-Phenylen, Y für O (Sauerstoff) oder S (Schwefel), bevorzugt O (Sauerstoff) und X für O (Sauerstoff), S (Schwefel) oder NR⁸, bevorzugt für O (Sauerstoff) oder NR⁸, besonders bevorzugt für O (Sauerstoff), stehen.

14. Δ¹-Pyrroline der Formel (I) gemäß Anspruch 1, in welcher R¹ und R² jeweils für Fluor stehen.

15. Δ¹-Pyrroline der Formel (I) gemäß Anspruch 1, in welcher R¹ für Methyl und R² für Wasserstoff stehen.

16. Δ¹-Pyrroline der Formel (I) gemäß Anspruch 1, in welcher R¹ für Chlor und R² für Wasserstoff stehen.

17. Δ¹-Pyroline der Formel (I) gemäß Anspruch 1, in welcher R¹ für Chlor und R² für Fluor stehen.

18. Δ¹-Pyrroline der Formel (I) gemäß Anspruch 1, in welcher R⁶ für Wasserstoff steht.

19. Δ¹-Pyrroline der Formel (I-a) mit (R)-Konfiguration in 5-Position des Pyrrolin-Ringes. 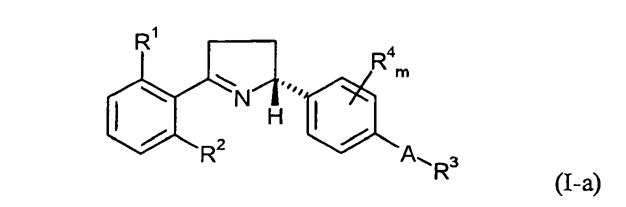 in welcher
R¹, R², A, R³, R⁴ und m die in Anspruch 1 angegebenen Bedeutungen haben.

20. Verfahren zum Herstellen von Verbindungen der Formel (I) gemäß Anspruch 1, **dadurch gekennzeichnet, dass** man
A) Δ¹-Pyrroline der Formel (II) 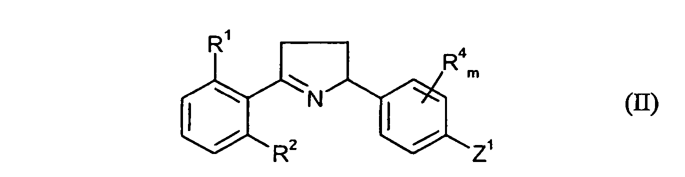 in welcher
R¹, R², R⁴ und m die in Anspruch 1 angegebenen Bedeutungen haben, und
Z¹ für Chlor, Brom, Iod, -OSO₂CF₃ oder -OSO₂(CF₂)₃CF₃ steht,
mit (Hetero)cyclen der Formel (ID) 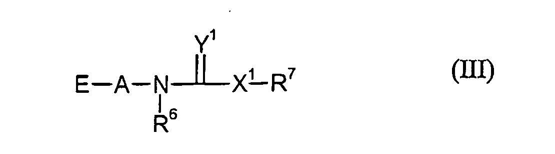 in welcher
A, R⁶ und R⁷ die in Anspruch 1 angegebenen Bedeutungen haben,
Y¹ für O (Sauerstoff) steht,
X¹ für O (Sauerstoff) oder NR⁸ steht,
E für Chlor, Brom, Iod, -OSO₂CF₃ oder -OSO₂(CF₂)₃CF₃ steht,
in Gegenwart eines Katalysators, in Gegenwart eines Diboronsäureesters und gegebenenfalls in Gegenwart eines Säurebindemittels und gegebenenfalls in Gegenwart eines Verdünnungsmittels in einer Tandem-Reaktion umsetzt,
oder
B) Δ¹-Pyrroline der Formel (IV) 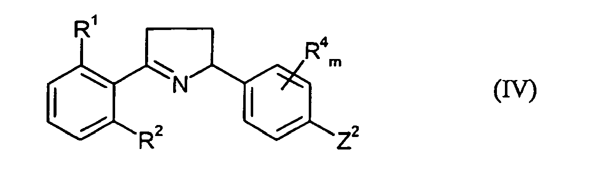 in welcher
R¹, R², R⁴ und m die in Anspruch 1 angegebenen Bedeutungen haben,
und
Z² für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl steht,
mit (Hetero)cyclen der Formel (III) 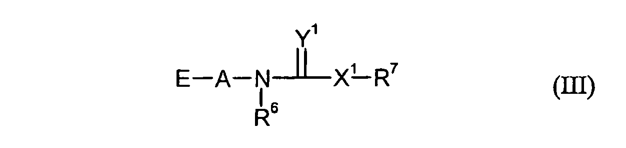 in welcher
A, R⁶ und R⁷ die in Anspruch 1 angegebenen Bedeutungen haben,
E, Y¹ und X¹ die oben angegebenen Bedeutungen haben,
in Gegenwart eines Katalysators, gegebenenfalls in Gegenwart eines Säurebindemittels und gegebenenfalls in Gegenwart eines Verdünnungsmittels umsetzt,
oder
C) Δ¹-Pyrroline der Formel (II) 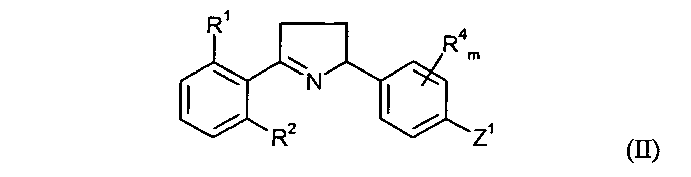 in welcher
R¹, R², R⁴ und m die in Anspruch 1 angegebenen Bedeutungen haben,
Z¹ die oben angegebenen Bedeutungen hat,
mit Boronsäure-Derivaten der Formel (V) 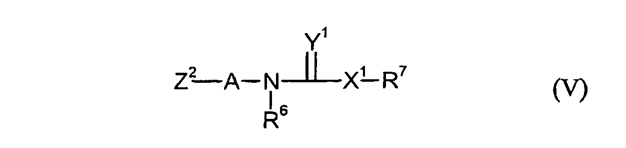 in welcher
A, R⁶ und R⁷ die und die in Anspruch 1 angegebenen Bedeutungen haben,
Z², Y¹ und X¹ die und die oben angegebenen Bedeutungen haben,
in Gegenwart eines Katalysators, gegebenenfalls in Gegenwart eines Säurebindemittels und gegebenenfalls in Gegenwart eines Verdünnungsmittels umsetzt,
oder
D) Δ¹-Pyrroline der Formel (II-a) 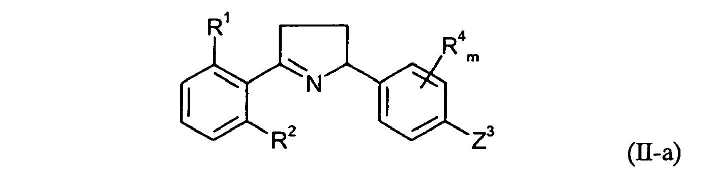 in welcher
R¹, R², R⁴ und m die in Anspruch 1 angegebenen Bedeutungen haben,
Z³ für Brom oder Iod steht,
mit metallorganischen Verbindungen der Formel (VI) 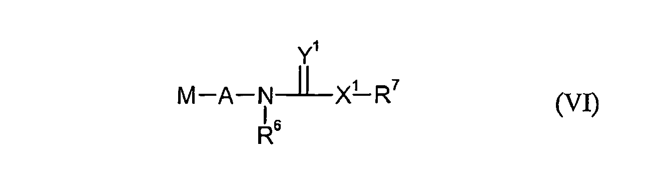 in welcher
A, R⁶ und R⁷ die in Anspruch 1 angegebenen Bedeutungen haben,
Y¹ und X¹ die oben angegebenen Bedeutungen haben,
M für ZnCl, Sn(Me)₃ oder Sn(n-Bu)₃ steht,
in Gegenwart eines Katalysators, gegebenenfalls in Gegenwart eines Säurebindemittels und gegebenenfalls in Gegenwart eines Verdünnungsmittels umsetzt,
oder
E) Δ¹-Pyrroline der Formel (VII) 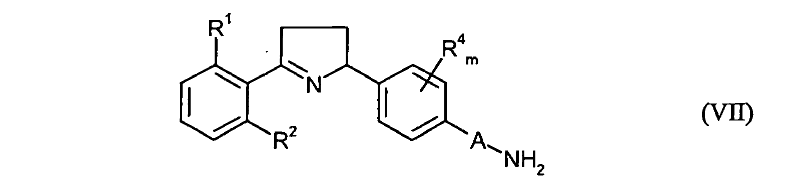
in welcher
R¹, R², A, R⁴ und m die in Anspruch 1 angegebenen Bedeutungen haben,
entweder mit einem Iso(thio)cyanat der Formel (VIII)
R⁷-N=C=Y (VIII)
in welcher
Y und R⁷ die in Anspruch 1 angegebenen Bedeutungen haben
oder mit einem (Thio)carbonat der Formel (IX) 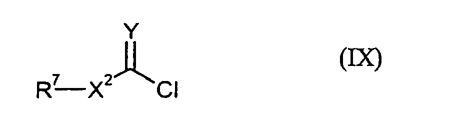 in welcher
Y und R⁷ die in Anspruch 1 angegebenen Bedeutungen haben,
X² für O (Sauerstoff) oder S (Schwefel) steht,
jeweils gegebenenfalls in Gegenwart eines Verdünnungsmittels und gegebenenfalls in Gegenwart eines Säurebindemittels umsetzt.

21. Schädlingsbekämpfungsmittel, **gekennzeichnet durch** einen Gehalt an mindestens einer Verbindung gemäß irgendeinem der Ansprüche 1 bis 19 neben Streckmitteln und/oder oberflächenaktiven Stoffen.

22. Verwendung von Verbindungen gemäß irgendeinem der Ansprüche 1 bis 19 zur Bekämpfung von Schädlingen, ausgenommen zur therapeutischen Behandlung des menschlichen oder tierischen Körpers.

23. Verfahren zur Bekämpfung von Schädlingen, **dadurch gekennzeichnet, dass** man Verbindungen gemäß irgendeinem der Ansprüche 1 bis 19 auf Schädlinge und/oder ihren Lebensraum einwirken lässt, ausgenommen zur therapeutischen Behandlung des menschlichen oder tierischen Körpers.

24. Verwendung von Verbindungen gemäß irgendeinem der Ansprüche 1 bis 19 zur Herstellung eines Tierarzneimittels gegen tierische Parasiten, insbesondere Ektoparasiten.

25. Verfahren zur Herstellung von Schädlingsbekämpfungsmitteln, **dadurch gekennzeichnet, dass** man Verbindungen der Formel (I) gemäß irgendeinem der Ansprüche 1 bis 19 mit Streckmitteln und/oder oberflächenaktiven Stoffen vermischt.

## Claims

1. Δ¹-Pyrrolines of the formula (I) 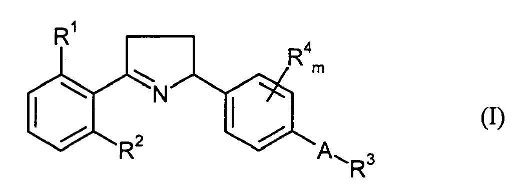 in which
R¹ represents halogen or methyl,
R² represents hydrogen or halogen,
R³ represents -N(R⁶)-C(=Y)-X-R⁷,
and
a)
A represents arylene or 5-membered heteroarylene having 1 to 3 heteroatoms, containing 0 to 3 nitrogen atoms, 0 to 1 oxygen atom and/or 0 to 1 sulphur atom, or 6-membered heteroarylene having 3 nitrogen atoms or 6-membered heteroarylene having 1 nitrogen atom and 1 to 2 further heteroatoms, of which 0 to 2 may be oxygen atoms and/or 0 to 2 may be sulphur atoms, each arylene or heteroarylene being optionally substituted from one to four times by identical or different substituents R⁵, and
Y represents O (oxygen) or S (sulphur), and
X represents O (oxygen), S (sulphur) or NR⁸,
or
b)
A represents pyridinylene, pyrimidinylene, pyrazinylene or pyridazinylene each optionally substituted once or twice by identical or different substituents R⁵, and
Y represents O (oxygen) or S (sulphur), and
X represents S (sulphur) or NR⁸,
or
c)
A represents pyridinylene, pyrimidinylene, pyrazinylene or pyridazinylene each optionally substituted once or twice by identical or different substituents R⁵, and
Y represents S (sulphur), and
X represents O (oxygen),
or
d)
A represents pyridinylene, pyrimidinylene, pyrazinylene or pyridazinylene each optionally substituted once or twice by identical or different substituents R⁵, and
Y represents O (oxygen), and
X represents O (oxygen),
and
R⁴ and R⁵ independently of one another represent halogen, alkyl, alkoxy, alkylthio, haloalkyl, haloalkoxy or haloalkylthio,
m represents 0, 1, 2, 3 or 4,
R⁶ represents hydrogen or alkyl,
R⁷ and R⁸ independently of one another represent hydrogen or represent alkyl or alkenyl each optionally substituted one or more times by identical or different substituents selected from the group consisting of halogen, alkylcarbonyl, alkylcarbonyloxy, alkylamino, dialkylamino, alkoxy, alkylthio, alkoxyalkoxy, haloalkoxy, haloalkylthio and halogenalkoxyalkoxy;
or represent cycloalkyl, cycloalkylalkyl, aryl, arylalkyl, saturated or unsaturated 5- to 10-membered heterocyclyl or heterocyclylalkyl each of which is optionally substituted one or more times by identical or different substitutents selected from the group consisting of halogen, alkyl, haloalkyl, alkoxy, haloalkoxy, alkylthio, haloalkylthio, alkylcarbonyl and alkoxycarbonyl,
R⁶ and R⁷ further together represent alkylene optionally substituted one or more times by alkyl, or
R⁷ and R⁸ further, together with the nitrogen atom to which they are attached, represent a saturated or unsaturated 5- to 10-membered heterocycle which may optionally contain a further heteroatom group from the series -O-, -S- and -NR⁹- and which may optionally be substituted one or more times by identical or different substituents selected from the group consisting of halogen, alkyl, alkoxy, alkylthio, haloalkyl, haloalkoxy and haloalkylthio, and
R⁹ represents hydrogen, alkyl or alkenyl.

2. Δ¹-Pyrrolines of the formula (I) according to Claim 1, in which
R¹ represents fluorine, chlorine, bromine or methyl,
R² represents hydrogen, fluorine, chlorine or bromine,
R³ represents -N(R⁶)-C(=Y)-X-R⁷,
and
a)
A represents arylene (especially phenylene) or 5-membered heteroarylene having 1 to 3 heteroatoms, containing 0 to 3 nitrogen atoms, 0 to 1 oxygen atom and/or 0 to 1 sulphur atom (in particular from the series pyrrolylene, furylene, thienylene, pyrazylene, imidazylene, triazylene, thiazylene or oxazylene), or 6-membered heteroarylene having 3 nitrogen atoms (especially triazinylene) or 6-membered heteroarylene having 1 nitrogen atom and 1 to 2 further heteroatoms, of which 0 to 2 may be oxygen atoms and/or 0 to 2 may be sulphur atoms (in particular from the series oxazinylene or thiazinylene), each being optionally substituted from one to three times by identical or different substituents R⁵, and
Y represents O (oxygen) or S (sulphur) and
X represents O (oxygen), S (sulphur) or NR⁸,
or
b)
A represents pyridinylene, pyrimidinylene, pyrazinylene or pyridazinylene each optionally substituted once or twice by identical or different substituents R⁵, and
Y represents O (oxygen) or S (sulphur), and
X represents S (sulphur) or NR⁸,
or
c)
A represents pyridinylene, pyrimidinylene, pyrazinylene or pyridazinylene each optionally substituted once or twice by identical or different substituents R⁵, and
Y represents S (sulphur), and
X represents O (oxygen),
or
d)
A represents pyridinylene, pyrimidinylene, pyrazinylene or pyridazinylene each optionally substituted once or twice by identical or different substituents R⁵, and
Y represents O (oxygen), and
X represents O (oxygen),
and
R⁴ and R⁵ independently of one another represents fluorine, chlorine, bromine, C₁-C₆-alkyl, C₁-C₆-alkoxy, C₁-C₆-alkylthio, C₁-C₆-haloalkyl, C₁-C₆-haloalkoxy or C₁-C₆-haloalkylthio,
m represents 0, 1, 2 or 3,
R⁶ represents hydrogen or C₁-C₆-alkyl,
R⁷ and R⁸ independently of one another represent hydrogen or represent C₁-C₂₀-alkyl or C₂-C₂₀-alkenyl each optionally substituted one or more times by identical or different substituents selected from the group consisting of fluorine, chlorine, bromine, C₁-C₆-alkylcarbonyl, C₁-C₆-alkylcarbonyloxy, C₁-C₆-alkylamino, di-(C₁-C₆-alkyl)amino, C₁-C₁₀-alkoxy, C₁-C₁₀-alkylthio, C₁-C₁₀-alkoxy-C₁-C₁₀-alkoxy, C₁-C₁₀-haloalkoxy, C₁-C₁₀-haloalkylthio and C₁-C₁₀-haloalkoxy-C₁-C₁₀-alkoxy;
or represent C₃-C₁₂-cycloalkyl, C₃-C₇-cycloalkyl-C₁-C₄-alkyl, aryl, aryl-C₁-C₄-alkyl, saturated or unsaturated 5- to 10-membered heterocyclyl or heterocyclyl-C₁-C₄-alkyl having 1 to 4 heteroatoms, containing 0 to 4 nitrogen atoms, 0 to 2 non-adjacent oxygen atoms and/or 0 to 2 non-adjacent sulphur atoms (especially tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl) each optionally substituted from one to four times by identical or different substituents selected from the group consisting of fluorine, chlorine, bromine, C₁-C₆-alkyl, C₁-C₆-alkoxy, C₁-C₆-alkylthio, C₁-C₆-haloalkyl, C₁-C₆-haloalkoxy, C₁-C₆-haloalkylthio, C₁-C₆-alkylearbonyl and C₁-C₆-alkoxycarbonyl,
R⁶ and R⁷ further together represent C₂-C₄-alkylene optionally substituted from one to four times by C₁-C₄-alkyl, or
R⁷ and R⁸ further represent, together with the nitrogen atom to which they are attached, a saturated or unsaturated 5- to 10-membered heterocycle which may optionally contain a further heteroatom group from the series -O-, -S- or -NR⁹- and which may optionally be substituted from one to four times by identical or different substituents selected from the group consisting of fluorine, chlorine, bromine, C₁-C₆-alkyl, C₁-C₆-alkoxy, C₁-C₆-alkylthio, C₁-C₆-haloalkyl, C₁-C₆-haloalkoxy and/or C₁-C₆-haloalkylthio, and
R⁹ represents hydrogen, C₁-C₆-alkyl or C₂-C₆-alkenyl.

3. Δ¹-Pyrrolines of the formula (I) according to Claim 1, in which
R¹ represents fluorine, chlorine or methyl,
R² represents hydrogen, fluorine or chlorine,
R³ represents -N(R⁶)-C(=Y)-X-R⁷,
and
a)
A represents phenylene, pyrrolylene, furylene, thienylene, pyrazylene, imidazylene, triazylene, thiazylene or oxazylene each optionally substituted once or twice by identical or different substituents R⁵, and
Y represents O (oxygen) or S (sulphur), and
X represents O (oxygen), S (sulphur) or NR⁸,
or
b)
A represents pyridinylene, pyrimidinylene, pyrazinylene or pyridazinylene each optionally substituted once or twice by identical or different substituents R⁵, and
Y represents O (oxygen) or S (sulphur), and
X represents S (sulphur) or NR⁸,
or
c)
A represents pyridinylene, pyrimidinylene, pyrazinylene or pyridazinylene each optionally substituted once or twice by identical or different substituents R⁵, and
Y represents S (sulphur), and
X represents O (oxygen),
or
d)
A represents pyridinylene, pyrimidinylene, pyrazinylene or pyridazinylene each optionally substituted once or twice by identical or different substituents R⁵, and
Y represents O (oxygen), and
X represents O (oxygen),
and
R⁴ and R⁵ independently of one another represent fluorine, chlorine, C₁-C₄-alkyl, C₁-C₄-alkoxy, C₁-C₄-alkylthio; C₁-C₄-haloalkyl, C₁-C₄-haloalkoxy or C₁-C₄-haloalkylthio having in each case 1 to 9 fluorine, chlorine and/or bromine atoms,
m represents 0, 1 or 2,
R⁶ represents hydrogen or C₁-C₄-alkyl,
R⁷ and R⁸ independently of one another represent hydrogen or represent C₁-C₁₆-alkyl or C₂-C₁₆-alkenyl each optionally substituted one or more times by identical or different substituents selected from the group consisting of fluorine, chlorine, bromine, C₁-C₄-alkylcarbonyl, C₁-C₄-alkylcarbonyloxy, C₁-C₄-alkylamino, di-(C₁-C₄-alkyl)amino, C₁-C₁₀-alkoxy, C₁-C₁₀-alkylthio, C₁-C₁₀-alkoxy-C₁-C₆-alkoxy, C₁-C₁₀-haloalkoxy, C₁-C₁₀-haloalkylthio and C₁-C₁₀-haloalkoxy-C₁-C₆-alkoxy having in each case 1 to 21 fluorine, chlorine and/or bromine atoms;
or represent C₃-C₁₀-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl each optionally substituted from one to three times by identical or different substituents selected from the group consisting of fluorine, chlorine, bromine, C₁-C₄-alkyl, C₁-C₄-alkoxy, C₁-C₄-alkylthio, C₁-C₄-haloalkyl, C₁-C₄-haloalkoxy, C₁-C₄-haloalkylthio having in each case 1 to 9 fluorine, chlorine and/or bromine atoms, C₁-C₄-alkylcarbonyl and C₁-C₄-alkoxycarbonyl,
R⁶ and R⁷ further together represent C₂-C₃-alkylene optionally substituted from one to three times by C₁-C₄-alkyl, or
R⁷ and R⁸ further represent, together with the nitrogen atom to which they are attached, a saturated or unsaturated 5- to 7-membered heterocycle which may optionally contain a further heteroatom group from the series -O-, -S- or -NR⁹- (in particular from the series piperidino, morpholino, thiomorpholino, piperazino, pyrrolidino, oxazolidino, thiazolidino, 4H-1-oxazinyl, 4H-1-thiazinyl) and which may optionally be substituted from one to four times by identical or different substituents selected from the group consisting of fluorine, chlorine, C₁-C₄-alkyl, C₁-C₄-alkoxy, C₁-C₄-alkylthio, C₁-C₄-haloalkyl, C₁-C₄-haloalkoxy and/or C₁-C₄-haloalkylthio having in each case 1 to 9 fluorine, chlorine and/or bromine atoms, and
R⁹ represents hydrogen, C₁-C₄-alkyl or C₂-C₄-alkenyl.

4. Δ¹-Pyrrolines of the formula (I) according to Claim 1, in which
R¹ represents fluorine or chlorine,
R² represents hydrogen or fluorine,
R³ represents -N(R⁶)-C(=Y)-X-R⁷,
and
a)
A represents 1,2-phenylene, 1,4-phenylene, 2,5-pyrrolylene, 2,5-furylene, 2,4-furylene, 2,5-thienylene, 2,4-thienylene, 2,5-thiazylene, 2,4-thiazylene, 2,5-oxazylene or 2,4-oxazylene each optionally substituted once by R⁵, and
Y represents O (oxygen) or S (sulphur), and
X represents O (oxygen), S (sulphur) or NR⁸,
or
b)
A represents 2,5-pyridinylene, 2,5-pyrimidinylene, 2,5-pyrazinylene or 3,6-pyridazinylene each optionally substituted once by R⁵, and
Y represents O (oxygen) or S (sulphur), and
X represents S (sulphur) or NR⁸,
or
c)
A represents 2,5-pyridinylene, 2,5-pyrimidinylene, 2,5-pyrazinylene or 3,6-pyridazinylene each optionally substituted once by R⁵, and
Y represents S (sulphur), and
X represents O (oxygen),
or
d)
A represents 2,5-pyridinylene, 2,5-pyrimidinylene, 2,5-pyrazinylene or 3,6-pyridazinylene each optionally substituted once by R⁵, and
Y represents O (oxygen), and
X represents O (oxygen),
and
R⁴ and R⁵ independently of one another represent fluorine, chlorine, methyl, ethyl, n-propyl, i-propyl, n-butyl, i-butyl, s-butyl, t-butyl, methoxy, ethoxy, n-propoxy, i-propoxy, n-butoxy, i-butoxy, s-butoxy, t-butoxy, methylthio, ethylthio, n-propylthio, i-propylthio, n-butylthio, i-butylthio, s-butylthio, t-butylthio, trifluoromethyl, trifluoroethyl, trifluoromethoxy, trifluoroethoxy, trifluoromethylthio or trifluoroethylthio,
m represents 0 or 1,
R⁶ represents hydrogen, methyl, ethyl, n-propyl, i-propyl, n-butyl, i-butyl, s-butyl or t-butyl,
R⁷ and R⁸ independently of one another represent hydrogen or represent C₁-C₁₀-alkyl or C₂-C₁₀-alkenyl (especially methyl, ethyl, n-propyl, i-propyl, n-butyl, i-butyl, s-butyl, t-butyl, the isomeric pentyls, the isomeric hexyls) each optionally substituted one or more times by identical or different substituents selected from the group consisting of fluorine, chlorine, bromine, C₁-C₄-alkylcarbonyl, C₁-C₄-alkylcarbonyloxy, C₁-C₄-alkylamino, di-(C₁-C₄-alkyl)amino, C₁-C₁₀-alkoxy, - C₁-C₈-alkoxy-C₁-C₆-alkoxy, C₁-C₁₀-alkylthio, C₁-C₁₀-haloalkoxy, C₁-C₁₀-haloalkylthio having in each case 1 to 21 fluorine, chlorine and/or bromine atoms, C₁-C₈-haloalkoxy-C₁-C₆-alkoxy having 1 to 17 fluorine, chlorine and/or bromine atoms;
or represent C₃-C₈-cycloalkyl, cyclopropylmethyl, cyclopentylmethyl, cyclohexylmethyl, cyclopropylethyl, cyclopentylethyl, cyclohexylethyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl each optionally substituted from one to three times by identical or different substituents selected from the group consisting of fluorine, chlorine, bromine, C₁-C₄-alkyl, C₁-C₄-alkoxy, C₁-C₄-alkylthio, C₁-C₄-haloalkyl, C₁-C₄-haloalkoxy, C₁-C₄-haloalkylthio having in each case 1 to 9 fluorine, chlorine and/or bromine atoms, C₁-C₄-alkylcarbonyl and C₁-C₄-alkoxycarbonyl,
R⁶ and R⁷ further together represent methylene or ethylene each optionally substituted once or twice by identical or different methyl, ethyl, n-propyl or i-propyl substituents, or
R⁷ and R⁸ further represent, together with the nitrogen atom to which they are attached, a 5- to 6-membered heterocycle from the series piperidino, morpholino, thiomorpholino, piperazino, pyrrolidino, oxazolidino, thiazolidino, 4H-1-oxazinyl, 4H-1-thiazinyl which may optionally be substituted from one to four times by identical or different substituents selected from the group consisting of fluorine, chlorine, bromine, methyl, ethyl, n-propyl, i-propyl, n-butyl, i-butyl, s-butyl, t-butyl, methoxy, ethoxy, n-propoxy, i-propoxy, n-butoxy, i-butoxy, s-butoxy, t-butoxy, methylthio, ethylthio, n-propylthio, i-propylthio, n-butylthio, i-butylthio, s-butylthio, t-butylthio, C₁-C₄-haloalkyl, C₁-C₄-haloalkoxy, C₁-C₄-haloalkylthio having in each case 1 to 9 fluorine, chlorine and/or bromine atoms, the piperazino radical being substituted on the second nitrogen atom by R⁹, and
R⁹ represents hydrogen, methyl, ethyl, n-propyl, i-propyl, n-butyl, i-butyl, s-butyl, t-butyl, vinyl or allyl.

5. Δ¹-Pyrrolines of the formulae (I-1) and (I-2) 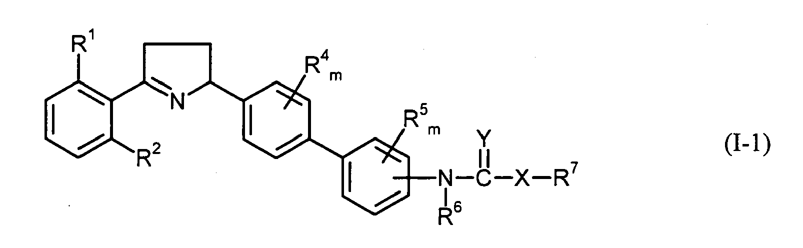 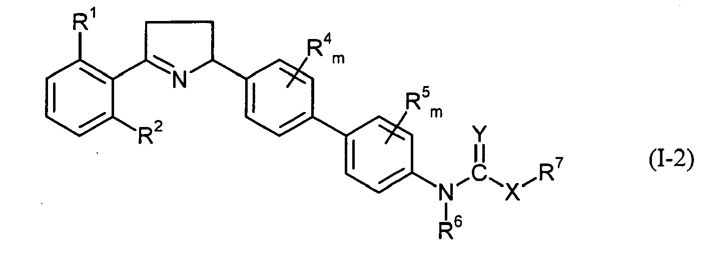 according to Claim 1, in each of which
a)
Y represents O (oxygen) or S (sulphur), and
X represents O (oxygen), S (sulphur) or NR⁸,
and R¹, R², R⁴, R⁵, m, R⁶, R⁷, R⁸ have the meanings given in Claim 1.

6. Δ¹-Pyrrolines of the formulae (I-3) to (I-8) 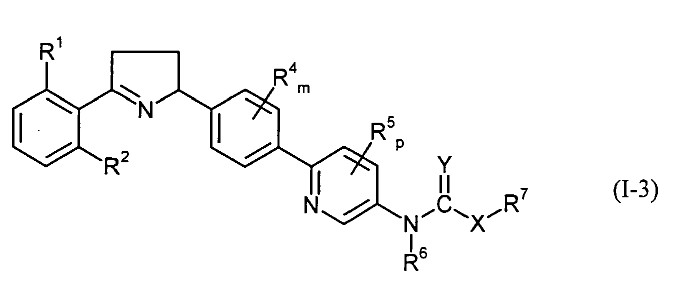 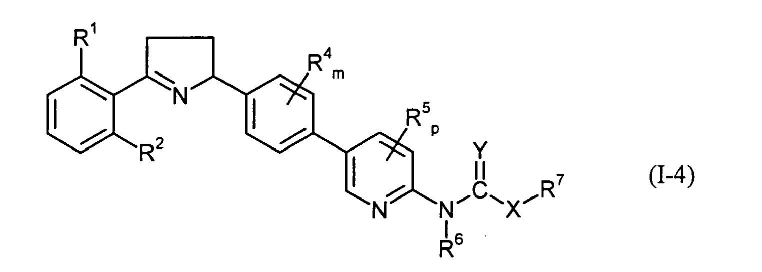 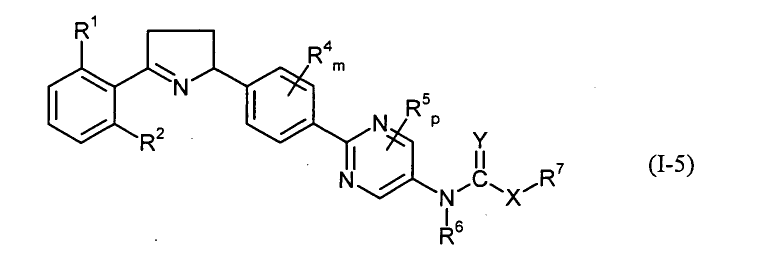 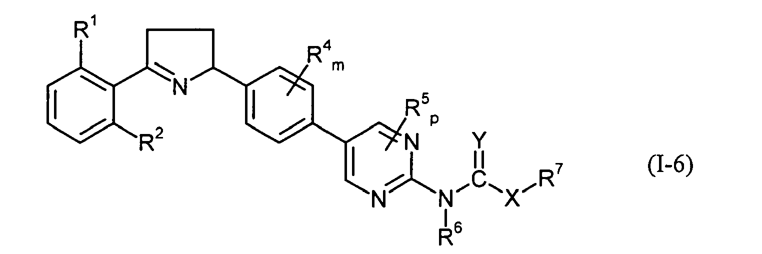 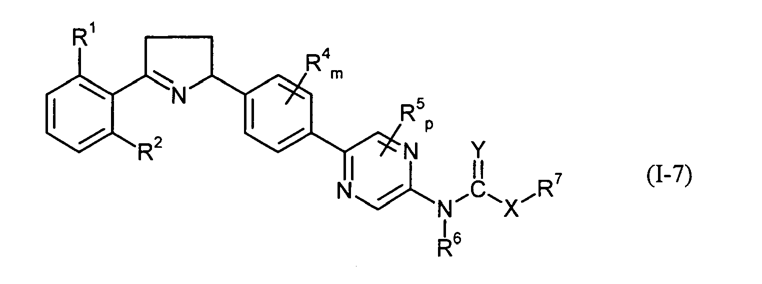 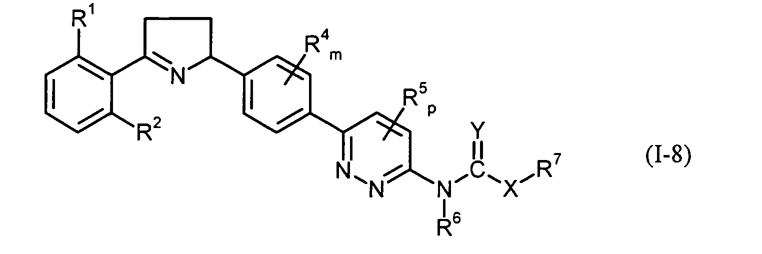 according to Claim 1,
in each of which
p represents 0, 1 or 2,
b)
Y represents O (oxygen) or S (sulphur), and
X represents S (sulphur) or NR⁸,
or
c)
Y represents S (sulphur), and
X represents O (oxygen),
or
d)
Y represents O (oxygen), and
X represents O (oxygen),
and R¹, R², R⁴, R⁵, m, R⁶, R⁷ and R⁸ have the meanings given in Claim 1.

7. Δ¹-Pyrrolines of the formula (I) according to Claim 1, in which A represents phenylene, preferably 1,4-phenylene.

8. Δ¹-Pyrrolines of the formula (I) according to Claim 1, in which A represents pyridinylene, pyrimidinylene, pyrazinylene or pyridazinylene, preferably 2,5-pyridinylene, 2,5-pyrimidinylene, 2,5-pyrazinylene or 3,6-pyridazinylene.

9. Δ¹-Pyrrolines of the formula (I) according to Claim 1, in which Y represents O (oxygen).

10. Δ¹-Pyrrolines of the formula (I) according to Claim 1, in which X represents O (oxygen) or NR⁸, preferably represents O (oxygen).

11. Δ¹-Pyrrolines of the formula (I) according to Claim 1, in which Y and X each represent O (oxygen).

12. Δ¹-Pyrrolines of the formula (I) according to Claim 1, in which Y represents O (oxygen) and X represents NR⁸.

13. Δ¹-Pyrrolines of the formula (I) according to Claim 1, in which A represents phenylene, preferably 1,4-phenylene, Y represents O (oxygen) or S (sulphur), preferably O (oxygen), and X represents O (oxygen), S (sulphur) or NR⁸, preferably O (oxygen) or NR⁸, with particular preference O (oxygen).

14. Δ¹-Pyrrolines of the formula (I) according to Claim 1, in which R¹ and R² each represent fluorine.

15. Δ¹-Pyrrolines of the formula (I) according to Claim 1, in which R¹ represents methyl and R² represents hydrogen.

16. Δ¹-Pyrrolines of the formula (I) according to Claim 1, in which R¹ represents chlorine and R² represents hydrogen.

17. Δ¹-Pyrrolines of the formula (I) according to Claim 1, in which R¹ represents chlorine and R² represents fluorine.

18. Δ¹-Pyrrolines of the formula (I) according to Claim 1, in which R⁶ represents hydrogen.

19. Δ¹-Pyrrolines of the formula (I-a) with (R) configuration in position 5 of the pyrroline ring. 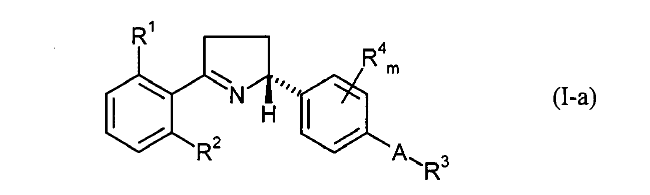 in which
R¹, R², A, R³, R⁴ and m have the meanings given in Claim 1.

20. Process for preparing compounds of the formula (I) according to Claim 1, **characterized by**
A) reacting Δ¹-pyrrolines of the formula (II) 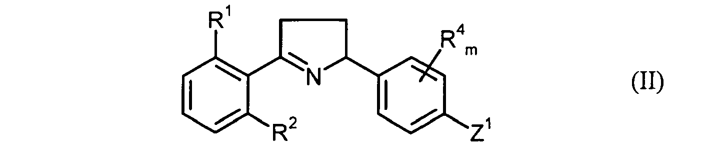 in which
R¹, R², R⁴ and m have the meanings given in Claim 1 and
Z¹ represents chlorine, bromine, iodine, -OSO₂CF₃ or -OSO₂(CF₂)₃CF₃,
in a tandem reaction with (hetero)cycles of the formula (III) 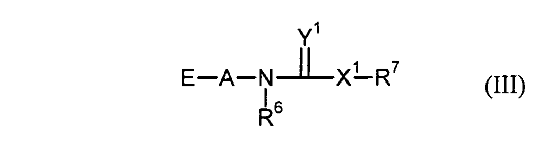 in which
A, R⁶ and R⁷ have the meanings given in Claim 1,
Y¹ represents O (oxygen),
X¹ represents O (oxygen) or NR⁸,
E represents chlorine, bromine, iodine, -OSO₂CF₃ or -OSO₂(CF₂)₃CF₃,
in the presence of a catalyst, in the presence of a diboronic ester and, where appropriate, in the presence of an acid binder and, where appropriate, in the presence of a diluent,
or
B) reacting Δ¹-pyrrolines of the formula (IV) 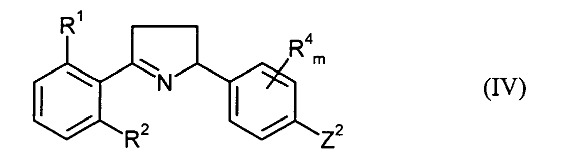 in which
R¹, R², R⁴ and m have the meanings given in Claim 1,
Z² represents -B(OH)₂, (4,4,5,5-tetramethyl-1,3,2-dioxaborolan)-2-yl, (5,5-dimethyl-1,3,2-dioxaborinan)-2-yl, (4,4,6-trimethyl-1,3,2-dioxaborinan)-2-yl or 1,3,2-benzodioxaborol-2-yl,
with (hetero)cycles of the formula (III) 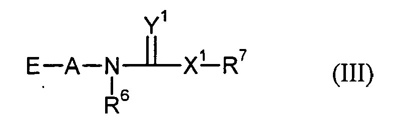 in which
A, R⁶ and R⁷ have the meanings given in Claim 1,
E, Y¹ and X¹ have the meanings given above,
in the presence of a catalyst, where appropriate in the presence of an acid binder and, where appropriate, in the presence of a diluent,
or
C) reacting Δ¹ -pyrrolines of the formula (II) 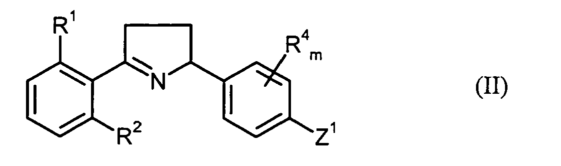 in which
R¹, R², R⁴ and m have the meanings given in Claim 1,
Z¹ has the meanings given above,
with boronic acid derivatives of the formula (V) 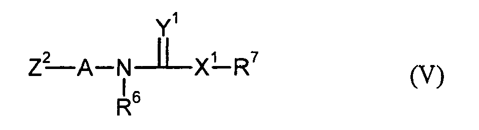 in which
A, R⁶ and R⁷ have the meanings given in Claim 1,
Z², Y¹ and X¹ have the meanings given above,
in the presence of a catalyst, where appropriate in the presence of an acid binder and, where appropriate, in the presence of a diluent,
or
D) reacting Δ¹-pyrrolines of the formula (II-a) 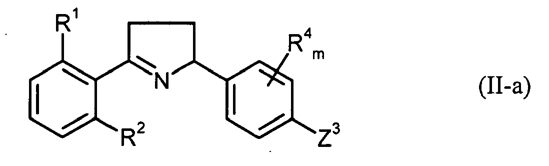 in which
R¹, R², R⁴ and m have the meanings given in Claim 1,
Z³ represents bromine or iodine
with organometallic compounds of the formula (VI) 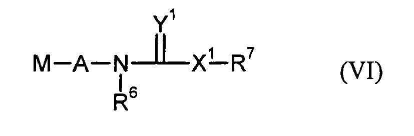 in which
A, R⁶ and R⁷ have the meanings given in Claim 1,
Y¹ and X¹ have the meanings given above,
M represents ZnCl, Sn(Me)₃ or Sn(n-Bu)₃,
in the presence of a catalyst, where appropriate in the presence of an acid binder and, where appropriate, in the presence of a diluent,
or
E) reacting Δ¹-pyrrolines of the formula (VII) 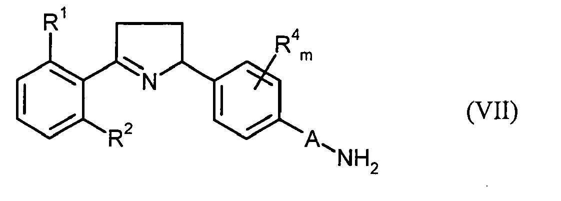
in which
R¹, R², A, R⁴ and m have the meanings given in Claim 1
either with an iso(thio)cyanate of the formula (VIII)
R⁷-N=C=Y (VIII)
in which
Y and R⁷ have the meanings given in Claim 1
or with a (thio)carbonate of the formula (IX) 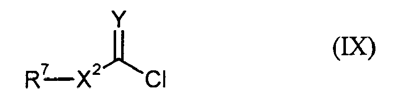 in which
Y and R⁷ have the meanings given in Claim 1,
X² represents O (oxygen) or S (sulphur),
in each case where appropriate in the presence of a diluent and, where appropriate, in the presence of an acid binder.

21. Pesticides, **characterized in that** they comprise at least one compound according to any of Claims 1 to 19 in addition to extenders and/or surface-active substances.

22. Use of compounds according to any of Claims 1 to 19 for controlling pests, except for the therapeutic treatment of the human or animal body.

23. Method of controlling pests, **characterized in that** compounds according to any of Claims 1 to 19 are caused to act on pests and/or their habitat, except for the therapeutic treatment of the human or animal body.

24. Use of compounds according to any of Claims 1 to 19 for producing a veterinary medicament for animal parasites, especially ectoparasites.

25. Process for producing pesticides, **characterized in that** compounds of the formula (I) according to any of Claims 1 to 19 are mixed with extenders and/or surface-active substances.

## Revendications

1. Δ¹-pyrrolines de formule (I) 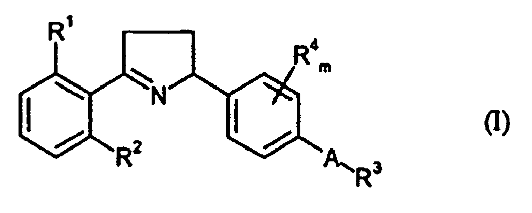 dans laquelle
R¹ représente un halogène ou un groupe méthyle,
R² représente l'hydrogène ou un halogène,
R³ représente un groupe -N(R⁶)-C(=Y)-X-R⁷,
et
a)
A représente un reste arylène ou un reste hétéroarylène pentagonal ayant 1 à 3 hétéroatomes, qui contient 0 à 3 atomes d'azote, 0 ou 1 atome d'oxygène et/ou 0 ou 1 atome de soufre, ou un reste hétéroarylène hexagonal ayant 3 atomes d'oxygène ou un reste hétéroarylène hexagonal ayant 1 atome d'azote et 1 ou deux autres hétéroatomes dont 0 à 2 peuvent être des atomes d'oxygène et/ou 0 à 2 peuvent des atomes de soufre, chaque reste éventuellement substitué une à quatre fois identiques ou différentes par R⁵, et
Y représente O (oxygène) ou S(soufre), et
X représente O (oxygène), S (soufre) ou NR⁸,
ou bien
b)
A représente un reste pyridinylène, pyrimidinylène, parazinylène ou pyridazinylène, chacun éventuellement substitué une ou deux fois identiques ou différentes par R⁵, et
Y représente O (oxygène) ou S (soufre), et
X représente S (soufre) ou NR⁸,
ou bien
c)
A représente un reste pyridinylène, pyrimidinylène, pyrazinylène ou pyridazinylène, chacun éventuellement substitué une ou deux fois identiques ou différentes par R⁵, et
Y représente S (soufre) et
X représente O (oxygène),
ou bien
d)
A représente un reste pyridinylène, pyrimidinylène, pyrazinylène ou pyridazinylène, chacun éventuellement substitué une ou deux fois identiques ou différentes par R⁵, et
Y représente O (oxygène), et
X représente O (oxygène),
et
R⁴ et R⁵ représentent, indépendamment l'un de l'autre, un halogène, un reste alkyle, alkoxy, alkylthio, halogénalkyle, halogénalkoxy ou halogénalkylthio,
m a la valeur 0, 1, 2, 3 ou 4,
R⁶ représente l'hydrogène ou un reste alkyle,
R⁷ et R⁸ représentent, indépendamment l'un de l'autre, l'hydrogène, un reste alkyle ou un reste alcényle, chacun éventuellement substitué une ou plusieurs fois identiques ou différentes par un radical halogéno, alkylcarbonyle, alkylcarbonyloxy, alkylamino, dialkylamino, alkoxy, alkylthio, alkoxyalkoxy, halogénalkoxy, halogénalkylthio et/ou halogénalkoxyalkoxy ;
un reste cycloalkyle, cycloalkylalkyle, aryle, arylalkyle, hétérocyclyle ou hétérocyclylalkyle saturé ou non saturé, pentagonal à décagonal, chacun éventuellement substitué une ou plusieurs fois identiques ou différentes par un radical halogéno, alkyle, halogénalkyle, alkoxy, halogénalkoxy, alkylthio, halogénalkylthio, alkylcarbonyle et/ou alkoxycarbonyle,
R⁶ et R⁷ forment en outre, ensemble, un reste alkylène éventuellement substitué une ou plusieurs fois par un radical alkyle, ou
R⁷ et R⁸ forment en outre, conjointement avec l'atome d'azote auquel ils sont liés, un hétérocycle pentagonal à décagonal saturé ou non saturé, qui peut éventuellement contenir un autre groupement hétéroatomique de la série -O-, -S- ou -NR⁹-, et qui peut éventuellement être substitué une ou plusieurs fois, identiques ou différentes, par un radical halogéno, alkyle, alkoxy, alkylthio, halogénalkyle, halogénalkoxy et/ou halogénalkylthio, et
R⁹ représente l'hydrogène, un reste alkyle ou alcényle.

2. Δ¹-pyrrolines de formule (I) suivant la revendication 1, formule dans laquelle
R¹ représente le fluor, le chlore, le brome ou un groupe méthyle,
R² représente l'hydrogène, le fluor, le chlore ou le brome,
R³ est un groupe -N(R⁶)-C(=Y)-X-R⁷,
et
a)
A représente un reste arylène (en particulier phénylène) ou hétéroarylène pentagonal ayant 1 à 3 hétéroatomes, qui contient 0 à 3 atomes d'azote, 0 ou 1 atome d'oxygène et/ou 0 ou 1 atome de soufre (en particulier de la série pyrrolylène, furylène, thiénylène, pyrazylène, imidazylène, triazylène, thiazylène ou oxazylène) ou un reste hétéroarylène hexagonal ayant 3 atomes d'azote (en particulier triazylène) ou un reste hétéroarylène hexagonal ayant 1 atome d'azote et 1 ou 2 autres hétéroatomes, dont 0 à 2 peuvent être des atomes d'oxygène et/ou 0 à 2 peuvent être des atomes de soufre (en particulier de la série oxazinylène ou thiazinylène), chacun éventuellement substitué une à trois fois identiques ou différentes par R⁵, et
Y représente O (oxygène) ou S (soufre), et
X représente O (oxygène), S (soufre) ou NR⁸,
ou bien
b)
A représente un reste pyridinylène, pyrimidinylène, pyrazinylène ou pyridazinylène, chacun éventuellement substitué une ou deux fois identiques ou différentes par R⁵, et
Y représente O (oxygène) ou S (soufre), et
X représente S (soufre) ou NR⁸,
ou bien
c)
A représente un reste pyridinylène, pyrimidinylène, pyrazinylène ou pyridazinylène, chacun éventuellement substitué une ou deux fois identiques ou différentes par R⁵, et
Y représente S (soufre), et
X représente O (oxygène),
ou bien
d)
A représente un reste pyridinylène, pyrimidinylène, pyrazinylène ou pyridazinylène, chacun éventuellement substitué une ou deux fois identiques ou différentes par R⁵, et
Y représente O (oxygène), et
X représente O (oxygène),
et
R⁴ et R⁵ représentent, indépendamment l'un de l'autre, le fluor, le chlore, le brome, un reste alkyle en C₁ à C₆, alkoxy en C₁ à C₆, alkylthio en C₁ à C₆, halogénalkyle en C₁ à C₆, halogénalkoxy en C₁ à C₆ ou halogénalkylthio en C₁ à C₆,
m a la valeur 0, 1, 2 ou 3,
R⁶ représente l'hydrogène ou un reste alkyle en C₁ à C₆,
R⁷ et R⁸ représentent, indépendamment l'un de l'autre, l'hydrogène, un reste alkyle en C₁ à C₂₀ ou un reste alcényle en C₂ à C₂₀, chacun éventuellement substitué une ou plusieurs fois identiques ou différentes par un radical fluoro, chloro, bromo, (alkyle en C₁ à C₆) carbonyle, (alkyle en C₁ à C₆) carbonyloxy, alkylamino en C₁ à C₆, di (alkyle en C₁ à C₆) amino, alkoxy en C₁ à C₁₀, alkylthio en C₁ à C₁₀, (alkoxy en C₁ à C₁₀) -(alkoxy en C₁ à C₁₀), halogénalkoxy en C₁ à C₁₀, halogénalkylthio en C₁ à C₁₀ et/ou (halogénalkoxy en C₁ à C₁₀) - (alkoxy en C₁ à C₁₀) ;
un reste cycloalkyle en C₃ à C₁₂, (cycloalkyle en C₃ à C₇)-(alkyle en C₁ à C₄) , aryle, aryl- (alkyle en C₁ à C₄), hétérocyclyle pentagonal à décagonal saturé ou non saturé ou hétérocyclyl-(alkyle en C₁ à C₄) ayant 1 à 4 hétéroatomes, qui comprennent 0 à 4 atomes d'oxygène, 0 à 2 atomes d'oxygène non contigus et/ou 0 à 2 atomes de soufre non contigus (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle), chacun éventuellement substitué une à quatre fois identiques ou différentes par un radical fluoro, chloro, bromo, alkyle en C₁ à C₆, alkoxy en C₁ à C₆, alkylthio en C₁ à C₆, halogénalkyle en C₁ à C₆, halogénalkoxy en C₁ à C₆, halogénalkylthio en C₁ à C₆, (alkyle en C₁ à C₆)carbonyle et/ou (alkoxy en C₁ à C₆) carbonyle,
R⁶ et R⁷ représentent en outre ensemble un reste alkylène en C₂ à C₄ éventuellement substitué une à quatre fois par un radical alkyle en C₁ à C₄, ou
R⁷ et R⁸ forment en outre, conjointement avec l'atome d'azote auquel ils sont liés, un hétérocycle pentagonal à décagonal saturé ou non saturé qui peut éventuellement contenir un autre groupement hétéroatomique de la série -O-, -S- ou -NR⁹- et qui peut être substitué le cas échéant une à quatre fois identiques ou différentes par du fluor, du chlore, du brome, un radical alkyle en C₁ à C₆, alkoxy en C₁ à C₆, alkylthio en C₁ à C₆, halogénalkyle en C₁ à C₆, halogénalkoxy en C₁ à C₆ et/ou halogénalkylthio en C₁ à C₆, et
R⁹ représente l'hydrogène, un reste alkyle en C₁ à C₆ ou un reste alcényle en C₂ à C₆.

3. Δ¹-pyrrolines de formule (I) suivant la revendication 1, formule dans laquelle
R¹ représente le fluor, le chlore ou un reste méthyle,
R² représente l'hydrogène, le fluor ou le chlore,
R³ représente un groupe -N(R⁶)-C(=Y)-X-R⁷,
et
a)
A représente un reste phénylène, pyrrolylène, furylène, thiénylène, pyrazylène, imidazylène, triazylène, thiazylène ou oxazylène, chacun éventuellement substitué une ou deux fois identiques ou différentes par R⁵, et
Y représente O (oxygène) ou S (soufre), et
X représente O (oxygène), S (soufre) ou NR⁸,
ou bien
b)
A représente un reste pyridinylène, pyrimidinylène, pyrazinylène ou pyridazinylène, chacun éventuellement substitué une ou deux fois identiques ou différentes par R⁵, et
Y représente O (oxygène) ou S (soufre), et
X représente S (soufre) ou NR⁸,
ou bien
c)
A représente un reste pyridinylène, pyrimidinylène, pyrazinylène ou pyridazinylène, chacun éventuellement substitué une ou deux fois identiques ou différentes par R⁵, et
Y représente S (soufre), et
X représente O (oxygène),
ou bien
d)
A représente un reste pyridinylène, pyrimidinylène, pyrazinylène ou pyridazinylène, chacun éventuellement substitué une ou deux fois identiques ou différentes par R⁵, et
Y représente O (oxygène), et
X représente O (oxygène),
et
R⁴ et R⁵ représentent, indépendamment l'un de l'autre, le fluor, le chlore, un reste alkyle en C₁ à C₄, alkoxy en C₁ à C₄, alkylthio en C₁ à C₄, halogénalkyle en C₁ à C₄, halogénalkoxy en C₁ à C₄ ou halogénalkylthio en C₁ à C₄, avec chacun 1 à 9 atomes de fluor, de chlore et/ou de brome,
m a la valeur 0, 1 ou 2,
R⁶ représente l'hydrogène ou un reste alkyle en C₁ à C₄,
R⁷ et R⁸ représentent, indépendamment l'un de l'autre, l'hydrogène, un reste alkyle en C₁ à C₁₆ ou un reste alcényle en C₂ à C₁₆, chacun éventuellement substitué une ou plusieurs fois identiques ou différentes par un radical fluoro, chloro, bromo, (alkyle en C₁ à C₄)carbonyle, (alkyle en C₁ à C₉) carbonyloxy, alkylamino en C₁ à C₄, di(alkyle en C₁ à C₄)amino, alkoxy en C₁ à C₁₀, alkylthio en C₁ à C₁₀, (alkoxy en C₁ à C₁₀) - (alkoxy en C₁ à C₆) , halogénalkoxy en C₁ à C₁₀, halogénalkylthio en C₁ à C₁₀ et/ou (halogénalkoxy en C₁ à C₁₀) - (alkoxy en C₁ à C₆), ayant chacun 1 à 21 atomes de fluor, de chlore et/ou de brome ;
un reste cycloalkyle en C₃ à C₁₀, (cycloalkyle en C₃ à C₆)-(alkyle en C₁ à C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, alkyle en C₁ à C₄, alkoxy en C₁ à C₄, alkylthio en C₁ à C₄, halogénalkyle en C₁ à C₉, halogénalkoxy en C₁ à C₄, halogénalkylthio en C₁ à C₄ ayant chacun 1 à 9 atomes de fluor, de chlore et/ou de brome, (alkyle en C₁ à C₄)carbonyle et/ou (alkoxy en C₁ à C₄) carbonyle,
R⁶ et R⁷ forment en outre ensemble un reste alkylène en C₂ ou C₃ éventuellement substitué une à trois fois par un radical alkyle en C₁ à C₄, ou bien
R⁷ et R⁸ forment en outre, conjointement avec l'atome d'azote auquel ils sont liés, un hétérocycle pentagonal à heptagonal saturé ou non saturé, qui peut contenir le cas échéant un autre groupement hétéroatomique de la série -O-, -S- ou -NR⁹- (en particulier de la série pipéridino, morpholino, thiomorpholino, pipérazino, pyrrolidino, oxazolidino, thiazolidino, 4H-1-oxazinyle, 4H-1-thiazinyle) et qui peut éventuellement être substitué une à quatre fois identiques ou différentes par un radical fluoro, chloro, alkyle en C₁ à C₄, alkoxy en C₁ à C₄, alkylthio en C₁ à C₄, halogénalkyle en C₁ à C₄, halogénalkoxy en C₁ à C₄ et/ou halogénalkylthio en C₁ à C₄, ayant chacun 1 à 9 atomes de fluor, de chlore et/ou de brome, et
R⁹ représente l'hydrogène, un reste alkyle en C₁ à C₄ ou alcényle en C₂ à C₄.

4. Δ¹-pyrrolines de formule (I) suivant la revendication 1, formule dans laquelle
R¹ représente le fluor ou le chlore,
R² représente l'hydrogène ou le fluor,
R³ représente un groupe -N-(R⁶)-C(=Y)-X-R⁷,
et
a)
A représente un reste 1,2-phénylène, 1,4-phénylène, 2,5-pyrrolylène, 2,5-furylène, 2,4-furylène, 2,5-thiénylène, 2,4-thiénylène, 2,5-thiazylène, 2,4-thiazylène, 2,5-oxazylène ou 2,4-oxazylène, chacun éventuellement substitué une fois par R⁵, et
Y représente O (oxygène) ou S (soufre), et
X représente O (oxygène), S (soufre) ou NR⁸,
ou bien
b)
A représente un reste 2,5-pyridinylène, 2,5-pyrimidinylène, 2,5-pyrazinylène ou 3,6-pyridazinylène, chacun éventuellement substitué une fois par R⁵, et
Y représente O (oxygène) ou S (soufre), et
X représente S (soufre) ou NR⁸,
ou bien
c)
A représente un reste 2,5-pyridinylène, 2,5-pyrimidinylène, 2,5-pyrazinylène ou 3,6-pyridazinylène, chacun éventuellement substitué une fois par R⁵, et
Y représente S (soufre), et
X représente O (oxygène),
ou bien
d)
A représente un reste 2,5-pyridinylène, 2,5-pyrimidinylène, 2,5-pyrazinylène ou 3,6-pyridazinylène, chacun éventuellement substitué une fois par R⁵, et
Y représente O (oxygène), et
X représente O (oxygène),
et
R⁴ et R⁵ représentent, indépendamment l'un de l'autre, le fluor, le chlore, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, trifluorométhyle, trifluoréthyle, trifluorométhoxy, trifluoréthoxy, trifluorométhylthio ou trifluoréthylthio,
m a la valeur 0 ou 1,
R⁶ représente l'hydrogène, un reste méthyle, éthyle, n-propyle, isopropyle, n-butyle, isobutyle, sec.-butyle ou tertiobutyle,
R⁷ et R⁸ représentent, indépendamment l'un de l'autre, l'hydrogène, un reste alkyle en C₁ à C₁₀ ou un reste alcényle en C₂ à C₁₀ (en particulier méthyle, éthyle, n-propyle, isopropyle, n-butyle, isobutyle, sec.-butyle, tertiobutyle, les restes pentyle isomères, les restes hexyle isomères), chacun éventuellement substitué une ou plusieurs fois identiques ou différentes par du fluor, du chlore, du brome, un radical (alkyle en C₁ à C₄) carbonyle, (alkyle en C₁ à C₄) carbonyloxy, alkylamino en C₁ à C₄, di(alkyle en C₁ à C₄)amino, alkoxy en C₁ à C₁₀, (alkoxy en C₁ à C₈)-(alkoxy en C₁ à C₆), alkylthio en C₁ à C₁₀, halogénalkoxy en C₁ à C₁₀, halogénalkylthio en C₁ à C₁₀ ayant dans chaque cas 1 à 21 atomes de fluor, de chlore et/ou de brome, (halogénalkoxy en C₁ à C₈)-(alkoxy en C₁ à C₆) ayant 1 à 17 atomes de fluor, de chlore et/ou de brome ;
un reste cycloalkyle en C₃ à C₈, cyclopropylméthyle, cyclopentylméthyle, cyclohexylméthyle, cyclopropyléthyle, cyclopentyléthyle, cyclohexyléthyle, phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, chacun éventuellement substitué une à trois fois identiques ou différentes par un radical fluoro, chloro, bromo, alkyle en C₁ à C₉, alkoxy en C₁ à C₉, alkylthio en C₁ à C₉, halogénalkyle en C₁ à C₄, halogénalkoxy en C₁ à C₄, halogénalkylthio en C₁ à C₄, ayant dans chaque cas 1 à 9 atomes de fluor, de chlore et/ou de brome, (alkyle en C₁ à C₄)carbonyle, et/ou (alkoxy en C₁ à C₄)carbonyle,
R⁶ et R⁷ forment en outre, ensemble, un reste méthylène ou un reste éthylène, chacun éventuellement substitué une ou deux fois identiques ou différentes par un radical méthyle, éthyle n-propyle ou isopropyle, ou bien
R⁷ et R⁸ forment en outre, conjointement avec l'atome d'azote auquel ils sont liés, un hétérocycle pentagonal ou hexagonal de la série pipéridino, morpholino, thiomorpholino, pipérazino, pyrrolidino, oxazolidino, thiazolidino, 4H-1-oxazinyle, 4H-1-thiazinyle, qui peut éventuellement être substitué une à quatre fois identiques ou différentes par un radical fluoro, chloro, bromo, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, halogénalkyle en C₁ à C₄, halogénalkoxy en C₁ à C₄, halogénalkylthio en C₁ à C₄ ayant dans chaque cas 1 à 9 atomes de fluor, de chlore et/ou de brome, le reste pipérazino étant substitué sur le second atome d'azote par R⁹, et
R⁹ représente l'hydrogène, un reste méthyle, éthyle, n-propyle, isopropyle, n-butyle, isobutyle, sec.-butyle, tertiobutyle, vinyle ou allyle.

5. Δ¹-pyrrolines de formules (I-1) et (I-2) 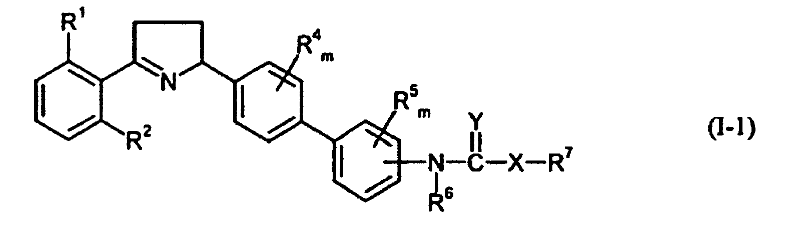 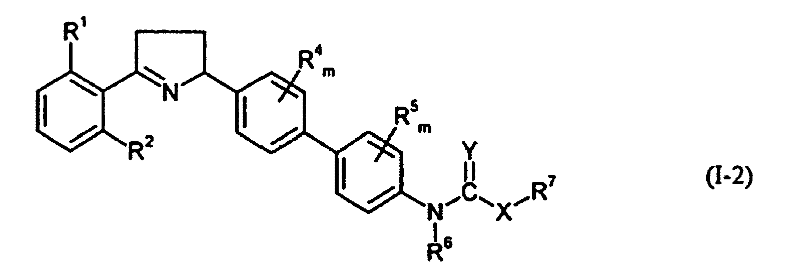 suivant la revendication 1, formules dans chacune desquelles
a)
Y représente O (oxygène) ou S (soufre), et
X représente O (oxygène), S (soufre) ou NR⁸,
et R¹, R², R⁴, R⁵, m, R⁶, R⁷, R⁸ ont les définitions indiquées dans la revendication 1.

6. Δ¹-pyrrolines de formules (I-3) à (I-8) 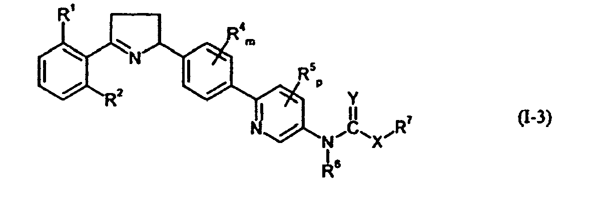 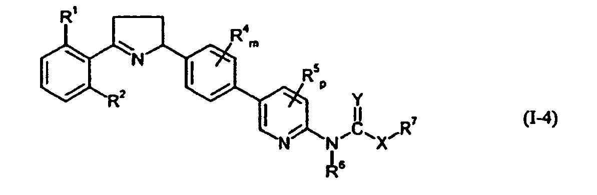 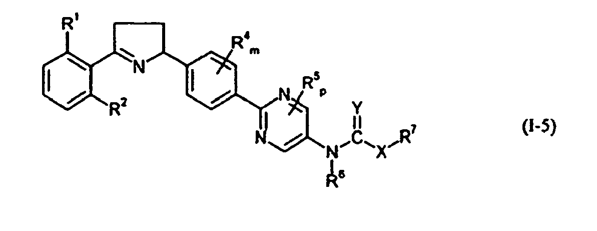 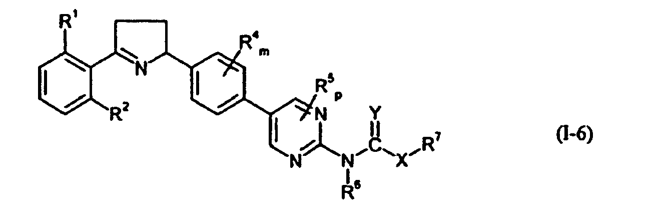 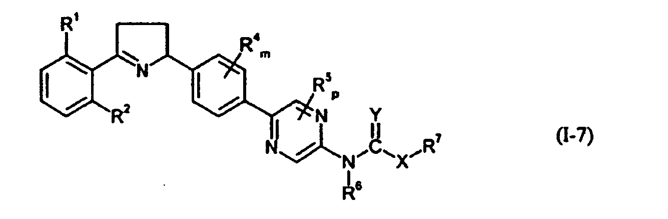 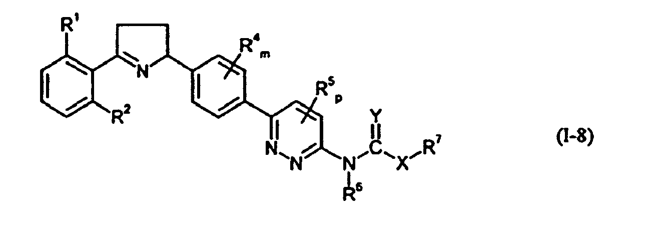 suivant la revendication 1, formules dans chacune desquelles
p a la valeur 0, 1 ou 2,
b)
Y représente O (oxygène) ou S (soufre), et
X représente S (soufre) ou NR⁸,
ou bien
C)
Y représente S (soufre), et
X représente O (oxygène),
ou bien
d)
Y représente O (oxygène), et
X représente O (oxygène),
et R¹, R², R⁴, R⁵, m, R⁶, R⁷ et R⁸ ont les définitions indiquées dans la revendication 1.

7. Δ¹-pyrrolines de formule (I) suivant la revendication 1, formule dans laquelle A est un reste phénylène, avantageusement le reste 1,4-phénylène.

8. Δ¹-pyrrolines de formule (I) suivant la revendication 1, formule dans laquelle A est un reste pyridinylène, pyrimidinylène, pyrazinylène ou pyridazinylène, avantageusement un reste 2,5-pyridinylène, 2,5-pyrimidinylène, 2,5-pyrazinylène ou 3,6-pyridazinylène.

9. Δ¹-pyrrolines de formule (I) suivant la revendication 1, formule dans laquelle Y représente O (oxygène).

10. Δ¹-pyrrolines de formule (I) suivant la revendication 1, formule dans laquelle X représente O (oxygène) ou NR⁸, avantageusement O (oxygène).

11. Δ¹-pyrrolines de formule (I) suivant la revendication 1, formule dans laquelle Y et X représentent chacun O (oxygène).

12. Δ¹-pyrrolines de formule (I) suivant la revendication 1, formule dans laquelle Y représente O (oxygène) et X représente NR⁸.

13. Δ¹-pyrrolines de formule (I) suivant la revendication 1, formule dans laquelle A représente un reste phénylène, avantageusement le reste 1,4-phénylène, Y représente O (oxygène) ou S (soufre), avantageusement O (oxygène) et X représente O (oxygène), S (soufre) ou NR⁸, avantageusement O (oxygène) ou NR⁸, notamment O (oxygène).

14. Δ¹-pyrrolines de formule (I) suivant la revendication 1, formule dans laquelle R¹ et R² représentent chacun le fluor.

15. Δ¹-pyrrolines de formule (I) suivant la revendication 1, formule dans laquelle R¹ est un reste méthyle et R² représente l'hydrogène.

16. Δ¹-pyrrolines de formule (I) suivant la revendication 1, formule dans laquelle R¹ représente le chlore et R² représente l'hydrogène.

17. Δ¹-pyrrolines de formule (I) suivant la revendication 1, formule dans laquelle R¹ représente le chlore et R² représente le fluor.

18. Δ¹-pyrrolines de formule (I) suivant la revendication 1, formule dans laquelle R⁶ représente l'hydrogène.

19. Δ¹-pyrrolines de formule (I-a) ayant la configuration (R) dans la position 5 du noyau de pyrroline 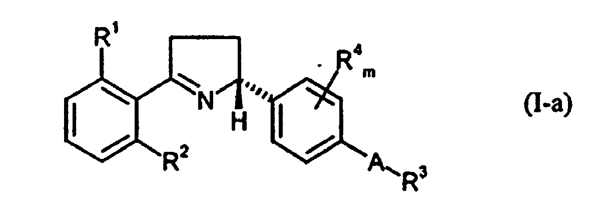 où
R¹, R², A, R³, R⁴ et m ont les définitions indiquées dans la revendication 1.

20. Procédé de production de composés de formule (I) suivant la revendication 1, **caractérisé en ce que** :
A) on fait réagir des Δ¹-pyrrolines de formule (II) 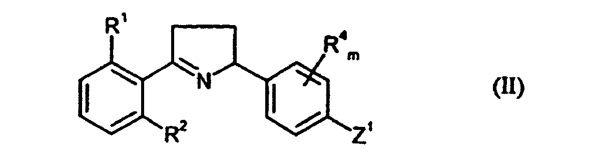 dans laquelle
R¹, R², R⁴ et m ont les définitions indiquées dans la revendication 1, et
Z¹ représente le chlore, le brome, l'iode, un groupe -OSO₂CF₃ ou -OSO₂ (CF₂)₃CF₃,
avec des (hétéro)cycles de formule (III) 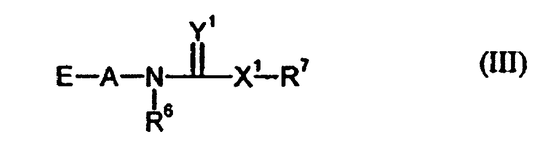 dans laquelle
A, R⁶ et R⁷ ont les définitions indiquées dans la revendication 1,
Y¹ représente O (oxygène),
X¹ représente O (oxygène) ou NR⁸,
E représente le chlore, le brome, l'iode, un groupe -OSO₂CF₃ ou -OSO₂(CF₂)₃CF₃,
en présence d'un catalyseur, en présence d'un ester d'acide diboronique et, le cas échéant, en présence d'un accepteur d'acide et en présence éventuelle d'un diluant dans une réaction en tandem,
ou bien
B) on fait réagir des Δ¹-pyrrolines de formule (IV) 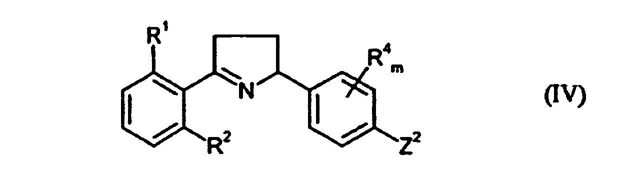 dans laquelle
R¹, R², R⁴ et m ont les définitions indiquées dans la revendication 1,
et
Z² est un groupe -B(OH)₂, (4,4,5,5-tétraméthyl-1,3,2-dioxaborolane)-2-yle, (5,5-diméthyl-1,3,2-dioxaborinane)-2-yle, (4,4,6-triméthyl-1,3,2-dioxaborinane)-2-yle ou 1,3,2-benzodioxaborol-2-yle,
avec des (hétéro)cycles de formulation (III) 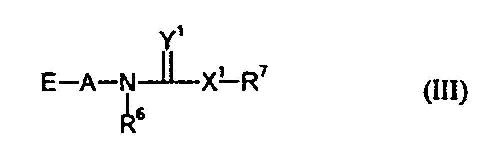 dans laquelle
A, R⁶ et R⁷ ont les définitions indiquées dans la revendication 1,
E, Y¹ et X¹ ont les définitions indiquées ci-dessus,
en présence d'un catalyseur, éventuellement en présence d'un accepteur d'acide et en présence éventuelle d'un diluant,
ou bien
C) on fait réagir des Δ¹-pyrrolines de formule (II) 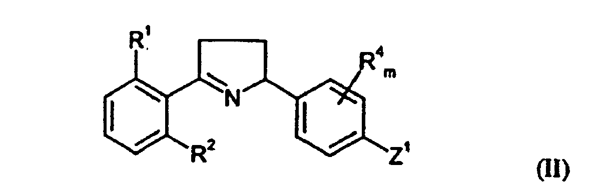 dans laquelle
R¹, R², R⁹ et m ont les définitions indiquées ci-dessus,
Z¹ a les définitions indiquées ci-dessus,
avec des dérivés d'acide boronique de formule (V) 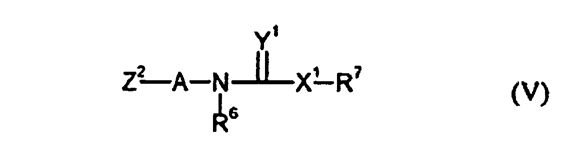 dans laquelle
A, R⁶ et R⁷ ont les définitions indiquées dans la revendication 1,
Z², Y¹ et X¹ ont les définitions indiquées ci-dessus,
en présence d'un catalyseur, éventuellement en présence d'un accepteur d'acide et en présence éventuelle d'un diluant,
ou bien
D) on fait réagir des Δ¹-pyrrolines de formule (II-a) 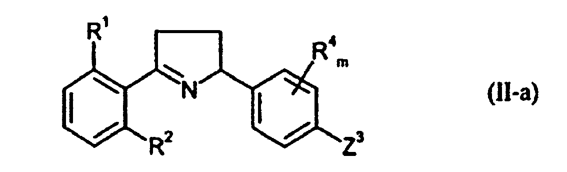 dans laquelle
R¹, ², R⁴ R et m ont les définitions indiquées dans la revendication 1,
Z³ représente le brome ou l'iode,
avec des composés organométalliques de formule (VI) 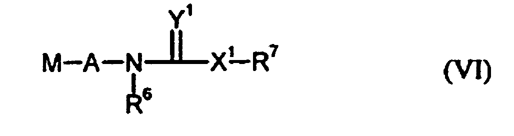 dans laquelle
A, R⁶ et R⁷ ont les définitions indiquées dans la revendication 1,
Y¹ et X¹ ont les définitions indiquées ci-dessus,
M représente ZnCl, Sn(Me)₃ ou Sn(n-Bu)₃,
en présence d'un catalyseur, éventuellement en présence d'un accepteur d'acide et en présence éventuelle d'un diluant,
ou bien
E) on fait réagir des Δ¹-pyrrolines de formule (VII) 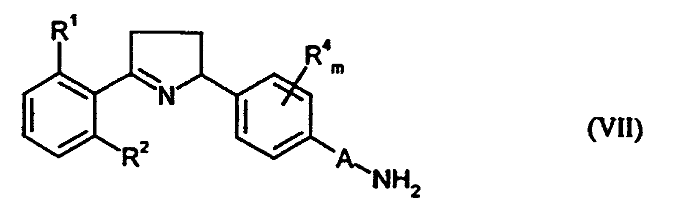 dans laquelle
R¹, R², A, R⁴ et m ont les définitions indiquées dans la revendication 1,
avec un iso(thio)cyanate de formule (VIII)
R⁷-N=C=Y (VIII)
dans laquelle
Y et R⁷ ont les définitions indiquées dans la revendication 1,
ou bien avec un (thio)carbonate de formule (IX) 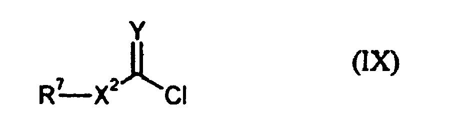
dans laquelle
Y et R⁷ ont les définitions indiquées dans la revendication 1
X² représente O (oxygène) ou S (soufre),
dans chaque cas, en présence éventuelle d'un diluant et, le cas échéant, en présence d'un accepteur d'acide.

21. Compositions pesticides, **caractérisées par** une teneur en au moins un composé selon l'une quelconque des revendications 1 à 19, à côté de diluants et/ou d'agents tensioactifs.

22. Utilisation de composés suivant l'une quelconque des revendications 1 à 19, pour combattre des parasites, excepté pour le traitement thérapeutique du corps humain ou animal.

23. Procédé pour combattre des parasites, **caractérisé en ce qu'**on fait agir des composés selon l'une quelconque des revendications 1 à 19 sur les parasites et/ou sur leur milieu, excepté pour le traitement thérapeutique du corps humain ou animal.

24. Utilisation de composés suivant l'une quelconque des revendications 1 à 19, pour la préparation d'un médicament à usage vétérinaire contre des parasites animaux, en particulier des ectoparasites.

25. Procédé de préparation de compositions pesticides, **caractérisé en ce qu'**on mélange des composés de formule (I) suivant l'une quelconque des revendications 1 à 19 avec des diluants et/ou des agents tensioactifs.
